(19) Europäisches Patentamt
European Patent Office
Office européen des brevets

(11) EP 2 341 051 A1

(12) **EUROPEAN PATENT APPLICATION**
published in accordance with Art. 153(4) EPC

(43) Date of publication:
**06.07.2011 Bulletin 2011/27**

(21) Application number: **09811625.4**

(22) Date of filing: **07.09.2009**

(51) Int Cl.:
***C07D 495/22*** (2006.01) ***C07D 495/04*** (2006.01)
***H01L 29/786*** (2006.01) ***H01L 51/05*** (2006.01)
***H01L 51/30*** (2006.01) ***H01L 51/50*** (2006.01)

(86) International application number:
**PCT/JP2009/065969**

(87) International publication number:
**WO 2010/027106 (11.03.2010 Gazette 2010/10)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **08.09.2008 JP 2008229384**

(71) Applicant: **Sumitomo Chemical Company, Limited**
**Tokyo 104-8260 (JP)**

(72) Inventors:
- **MIYATA, Yasuo**
**Toyonaka-shi**
**Osaka (JP)**
- **YOSHIKAWA, Eiji**
**Tsukuba-shi,**
**Ibaraki (JP)**

(74) Representative: **Vossius & Partner**
**Siebertstrasse 4**
**81675 München (DE)**

# (54) NOVEL COMPOUND AND ORGANIC SEMICONDUCTOR MATERIAL

(57) Discloses is a compound represented by the formula (1):

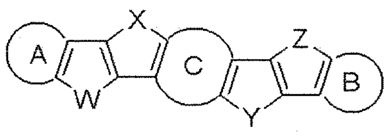

(1)

wherein a ring structure A and a ring structure B independently represent an aromatic ring which may be substituted, or a heterocyclic ring which may be substituted;
a ring structure C represents a benzene ring which may be substituted, a hetero[3,2-b]heterole ring, or a benzo[1,2-b:4,5-b']diheterole ring which may be substituted;
W, X, Y and Z independently represent a sulfur atom, an oxygen atom, a selenium atom, a tellurium atom, $SO_2$ $(R^1)$-C-$(R^2)$, $(R^3)$-Si-$(R^4)$, or N-$(R^5)$, and at least one of W, X, Y and Z is N-$(R^5)$;$R^1$, $R^2$, $R^3$, $R^4$ and $R^5$ independently represent a hydrogen atom, a substituent i, a substituent ii, an aryl group which may be substituted with halogen, or a heteroaryl group which may be substituted with halogen, the substituent i is a group selected from Group P shown below, the substituent ii is a group selected from Group P shown below which group is substituted, and Group P consists of an alkyl group, an alkoxy group, an alkyl-substituted aryl group, an alkoxy-substituted aryl group, and an alkyl-substituted heteroaryl group.

EP 2 341 051 A1

Fig.1

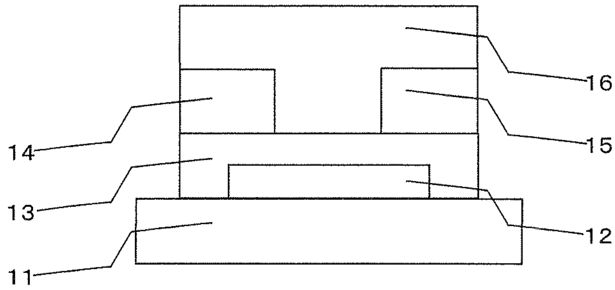

## Description

Technical Field

The present invention relates to a novel compound and an organic semiconductor material.

Background Art

Organic semiconductor devices such as organic transistors are expected as elements applicable to the next-generation technology such as electronic papers and large screen flat panel displays. The organic transistor is constituted of several kinds of members including an organic semiconductor active layer, a substrate, an insulating layer and an electrode, and particularly the organic semiconductor active layer taking on the carrier transport has an important function. Characteristics of a transistor depend largely on the carrier transport ability of an organic material constituting this organic semiconductor active layer.

As an organic semiconductor material for an organic transistor, various organic compounds, for example, polycyclic condensed ring compounds such as pentacene, dinaphthothienothiophene and indro[3,2-b]carbazole, oligomers, polymer materials, and the like, are disclosed (J. Appl. Phys. 2002, 92, 5259; J. Am. Chem. Soc. 2004, 126, 13859; Science 1998, 280, 1741.; J. Am. Chem. Soc. 2007, 129, 2224; J. Am. Chem. Soc. 2005, 127, 614; J. Am. Chem. Soc. 2007, 129, 9125)

DISCLOSURE OF THE INVENTION

An object of the present invention is to provide a novel compound which is useful as an organic semiconductor material for an organic transistor, a method for producing the compound, an organic semiconductor material containing the compound, an organic thin film, and an organic transistor.
The present invention relates to the following inventions.

[1] A compound represented by the formula (1):

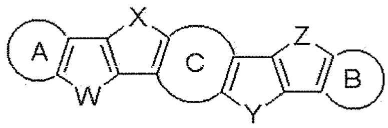

(1)

wherein a ring structure A and a ring structure B independently represent an aromatic ring which may be substituted or a heterocyclic ring which may be substituted;
a ring structure C represents a benzene ring which may be substituted, a hetero[3,2-b]heterole ring, or a benzo[1,2-b:4,5-b']diheterole ring which may be substituted;
W, X, Y and Z independently represent a sulfur atom, an oxygen atom, a selenium atom, a tellurium atom, $SO_2$, ($R^1$)-C-($R^2$), ($R^3$)-Si-($R^4$), or N-($R^5$), and at least one of W, X, Y and Z is N-($R^5$); and
$R^1$, $R^2$, $R^3$, $R^4$ and $R^5$ independently represent a hydrogen atom, a substituent i, a substituent ii, an aryl group which may be substituted with halogen, or a heteroaryl group which may be substituted with halogen, the substituent i is a group selected from Group P shown below, the substituent ii is a group selected from Group P shown below which group is substituted, and Group P consists of an alkyl group, an alkoxy group, an alkyl-substituted aryl group, an alkoxy-substituted aryl group, and an alkyl-substituted heteroaryl group.
[2] The compound according to [1], wherein the ring structure A and the ring structure B independently represent an aromatic ring, a heterocyclic ring, an aromatic ring substituted with a substituent iii, an aromatic ring substituted with a substituent iv, a heterocyclic ring substituted with a substituent iii, a heterocyclic ring substituted with a substituent iv, a halogen-substituted aromatic ring, or a halogen-substituted heterocyclic ring,
the substituent iii is a substituent selected from Group Q shown below,
the substituent iv is a substituent selected from Group Q shown below, which is substituted with a fluorine atom, and Group Q consists of an alkyl group, an alkoxy group, an alkyl-substituted aryl group, an alkoxy-substituted aryl group, an alkoxy-substituted heteroaryl group, an alkyl-substituted heteroaryl group, an alkenyl group, an alkynyl group, an alkylthio group, an alkylcarbonyl group, an alkoxycarbonyl group, a (trialkyl)silyl group, and a (dialkyl) amino group.
[3] The compound according to [2], wherein the ring structure A and the ring structure B independently represent a benzene ring, a benzene ring substituted with the substituent iii, a benzene ring substituted with the substituent iv,

a halogen-substituted benzene ring, a naphthalene ring, a naphthalene ring substituted with the substituent iii, a naphthalene ring substituted with the substituent iv, a halogen-substituted naphthalene ring, a benzo[b]thiophene ring, a benzo[b]thiophene ring substituted with the substituent iii, a benzo[b]thiophene ring substituted with the substituent iv, a halogen-substituted benzo [b] thiophene ring, a benzo [b] furan ring, a benzo[b]furan ring substituted with the substituent iii, a benzo[b]furan ring substituted with the substituent iv, or a halogen-substituted benzo[b] furan ring.

[4] The compound according to [2], wherein the ring structure A and the ring structure B independently represent a benzene ring, a benzene ring substituted with the substituent iii, a benzene ring substituted with the substituent iv, or a halogen-substituted benzene ring.

[5] The compound according to [1], wherein the ring structure C is a benzene ring, a benzene ring substituted with a substituent iii, a benzene ring substituted with a substituent iv, a halogen-substituted benzene ring, a thieno[3,2-b]thiophene ring, a benzo[1,2-b:4,5-b']dithiophene ring, a benzo[1,2-b:4,5-b']dithiophene ring substituted with a substituent iii, a benzo[1,2-b:4,5-b']dithiophene ring substituted with a substituent iv, a halogen-substituted benzo [1,2-b:4,5-b']dithiophene ring, a benzo[1,2-b:4,5-b']difuran ring, a benzo[1,2-b:4,5-b']difuran ring substituted with a substituent iii, a benzo[1,2-b:4,5-b']difuran ring substituted with the substituent iv, or a halogen-substituted benzo [1,2-b:4,5-b']difuran ring,

the substituent iii is a substituent selected from Group Q shown below,

the substituent iv is a substituent selected from Group Q shown below, which is substituted with a fluorine atom, and Group Q consists of an alkyl group, an alkoxy group, an alkyl-substituted aryl group, an alkoxy-substituted aryl group, an alkoxy-substituted heteroaryl group, an alkyl-substituted heteroaryl group, an alkenyl group, an alkynyl group, an alkylthio group, an alkylcarbonyl group, an alkoxycarbonyl group, a (trialkyl)silyl group, and a (dialkyl) amino group.

[6] The compound according to [5], wherein the ring structure C is a benzene ring, a benzene ring substituted with the substituent iii, a benzene ring substituted with the substituent iv, a halogen-substituted benzene ring, or a thieno [3,2-b]thiophene ring.

[7] The compound according to any one of [1] to [6], wherein any one of W and X is N-($R^5$), the other one is sulfur, oxygen, ($R^1$) -C- ($R^2$) or ($R^3$) -Si-($R^4$) Z and W are the same, and Y and X are the same.

[8] The compound according to any one of [1] to [7], wherein any one of W and X is N-($R^5$), the other one is a sulfur atom or an oxygen atom, Z and W are the same, and Y and X are the same.

[9] The compound according to any one of [1] to [8], wherein $R^1$, $R^2$, $R^3$, $R^4$ and $R^5$ independently represent a hydrogen atom, an alkyl group having 1 to 30 carbon atoms, a fluoroalkyl group having 1 to 30 carbon atoms, a phenyl group, a phenyl group substituted with an alkyl group having 1 to 30 carbon atoms, a phenyl group substituted with a fluoroalkyl group having 1 to 30 carbon atoms, a phenyl group substituted with an alkoxy group having 1 to 30 carbon atoms, a phenyl group substituted with a fluoroalkoxy group having 1 to 30 carbon atoms, a thienyl group, a thienyl group substituted with an alkyl group having 1 to 30 carbon atoms, or a thienyl group substituted with a fluoroalkyl group having 1 to 30 carbon atoms.

[10] The compound according to any one of [1] to [9], wherein $R^5$ is an alkyl group having 1 to 20 carbon atoms, a fluoroalkyl group having 1 to 20 carbon atoms, a phenyl group, a phenyl group substituted with an alkyl group having 1 to 20 carbon atoms, a phenyl group substituted with a fluoroalkyl group having 1 to 20 carbon atoms, or a phenyl group substituted with an alkoxy group having 1 to 20 carbon atoms.

[11] The compound according to any one of [1] to [10], wherein the ring structure A, the ring structure B and the ring structure C independently represent a benzene ring, a benzene ring substituted with a substituent iii, or a halogen-substituted benzene ring, X and Y are the same, and N-($R^5$), W and Z are the same and sulfur atoms or oxygen atoms, the substituent iii is a substituent selected from Group Q shown below, and

Group Q consists of an alkyl group, an alkoxy group, an alkyl-substituted aryl group, an alkoxy-substituted aryl group, an alkoxy-substituted heteroaryl group, an alkyl-substituted heteroaryl group, an alkenyl group, an alkynyl group, an alkylthio group, an alkylcarbonyl group, an alkoxycarbonyl group, a (trialkyl)silyl group, and a (dialkyl) amino group.

[12] The compound according to any one of [1] to [10], wherein the ring structure A, the ring structure B and the ring structure C independently represent a benzene ring, a benzene ring substituted with a substituent iii, or a halogen-substituted benzene ring, X and Y are the same and are sulfur atoms or oxygen atoms, W and Z are the same and are N-($R^5$),

the substituent iii is a substituent selected from Group Q shown below, and

Group Q consists of an alkyl group, an alkoxy group, an alkyl-substituted aryl group, an alkoxy-substituted aryl group, an alkoxy-substituted heteroaryl group, an alkyl-substituted heteroaryl group, an alkenyl group, an alkynyl group, an alkylthio group, an alkylcarbonyl group, an alkoxycarbonyl group, a (trialkyl)silyl group, and a (dialkyl) amino group.

[13] The compound according to any one of [2] to [10], wherein the ring structure A and the ring structure B independently represent a benzene ring, a benzene ring substituted with the substituent iii, or a halogen-substituted benzene ring, the ring structure C is a thieno[3,2-b]thiophene ring, X and Y are the same and are N-($R^5$), and W and Z are the same and are sulfur atoms or oxygen atoms.

[14] The compound according to any one of [2] to [10], wherein the ring structure A and the ring structure B independently represent a benzene ring, a benzene ring substituted with the substituent iii, or a halogen-substituted benzene ring, the ring structure C is a thieno[3,2-b]thiophene ring, X and Y are the same and are sulfur atoms or oxygen atoms, and W and Z are the same and are N-($R^5$).

[15] The compound according to any one of [1] to [10], wherein the ring structure A and the ring structure B independently represent a benzene ring, a benzene ring substituted with a substituent iii, or a halogen-substituted benzene ring, the ring structure C is a benzo[1,2-b:4,5-b']dithiophene ring, a benzo[1,2-b:4,5-b']dithiophene ring substituted with a substituent iii, a halogen-substituted benzo[1,2-b:4,5-b']dithiophene ring, a benzo[1,2-b:4;5-b'] difuran ring, a benzo[1,2-b:4,5-b']difuran ring substituted with a substituent iii, or a halogen-substituted benzo[1,2-b:4,5-b']difuran ring, X and Y are the same and are N-($R^5$), W and Z are the same and are sulfur atoms or oxygen atoms, the substituent iii is a substituent selected from Group Q shown below,

Group Q consists of an alkyl group, an alkoxy group, an alkyl-substituted aryl group, an alkoxy-substituted aryl group, an alkoxy-substituted heteroaryl group, an alkyl-substituted heteroaryl group, an alkenyl group, an alkynyl group, an alkylthio group, an alkylcarbonyl group, an alkoxycarbonyl group, a (trialkyl)silyl group, and a (dialkyl) amino group.

[16] The compound according to any one of [1] to [10], wherein the ring structure A and the ring structure B independently represent a benzene ring, a benzene ring substituted with a substituent iii, or a halogen-substituted benzene ring, the ring structure C is a benzo[1,2-b:4,5-b']dithiophene ring, a benzo[1,2-b:4,5-b']dithiophene ring substituted with a substituent iii, a halogen-substituted benzo[1,2-b:4,5-b']dithiophene ring, a benzo[1,2-b:4,5-b'] difuran ring, a benzo[1,2-b:4,5-b']difuran ring substituted with a substituent iii, or a halogen-substituted benzo[1,2-b:4,5-b']difuran ring, X and Y are the same and are sulfur atoms or oxygen atoms, W and Z are the same and are N-($R^5$),

the substituent iii is a substituent selected from Group Q shown below,

Group Q consists of an alkyl group, an alkoxy group, an alkyl-substituted aryl group, an alkoxy-substituted aryl group, an alkoxy-substituted heteroaryl group, an alkyl-substituted heteroaryl group, an alkenyl group, an alkynyl group, an alkylthio group, an alkylcarbonyl group, an alkoxycarbonyl group, a (trialkyl)silyl group, and a (dialkyl) amino group.

[17] A method for producing the compound according to [1], comprising a step of reacting a tetrahalogen compound represented by the formula (2) or (3):

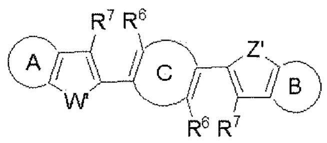

(2)

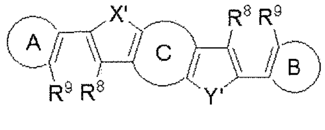

(3)

wherein a ring structure A, a ring structure B and a ring structure C are each as defined in [1], W' , X' , Y' and Z' independently represent a sulfur atom, an oxygen atom, a selenium atom, a tellurium atom, $SO_2$, ($R^1$) -C- ($R^2$), ($R^3$) -Si-($R^4$), or N-($R^5$), $R^1$, $R^2$, $R^3$, $R^4$ and $R^5$ are as defined in [1], and $R^6$, $R^7$, $R^8$ and $R^9$ independently represent a halogen atom, with $R^5$-$NH_2$, wherein $R^5$ is as defined in [1].

[18] The method according to [17], wherein $R^6$, $R^7$, $R^8$ and $R^9$ independently represent bromine or iodine.

[19] The method according to [17] or [18], wherein X' ,Y' ,W' and Z' independently represent a sulfur atom or an oxygen atom.

[20] The method according to any one of [17] to [19], wherein $R^5$ is an alkyl group having 1 to 20 carbon atoms, a fluoroalkyl group having 1 to 20 carbon atoms, a phenyl group, a phenyl group substituted with an alkyl group having 1 to 20 carbon atoms, a phenyl group substituted with a fluoroalkyl group having 1 to 20 carbon atoms, or a phenyl group substituted with an alkoxy group having 1 to 20 carbon atoms.

[21] A tetrahalogen compound represented by the formula (2) :

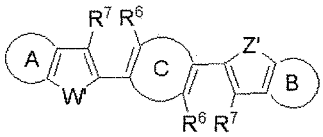

(2)

wherein a ring structure A and a ring structure B are each as defined in [1], a ring structure C is a thieno[3,2-b] thiophene ring, W' and Z' independently represent a sulfur atom or an oxygen atom, and $R^6$, $R^7$, $R^8$ and $R^9$ independently represent a halogen atom.

[22] The tetrahalogen compound according to [21], wherein the ring structure A and the ring structure B independently represent a benzene ring, a naphthalene ring, a thiophene ring, a benzothiophene ring, a furan ring, or a benzofuran ring.

[23] A tetrahalogen compound represented by the formula (3) :

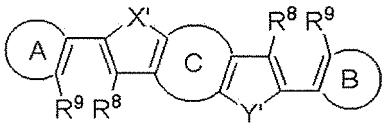

(3)

wherein a ring structure A and a ring structure B are each as defined in [1], a ring structure C is a thieno[3,2-b] thiophene ring, a furo[3,2-b]furan ring, a benzo[1,2-b:4,5-b']dithiophene ring which may be substituted, or a benzo [1,2-b:4,5-b']difuran ring which may be substituted, and X' and Y' independently represent a sulfur atom or an oxygen atom.

[24] The tetrahalogen compound according to [23], wherein the ring structure A and the ring structure B independently represent a benzene ring, a naphthalene ring, a thiophene ring, a benzothiophene ring, a furan ring, or a benzofuran ring.

[25] A method for producing a tetrahalogen compound represented by the formula (2):

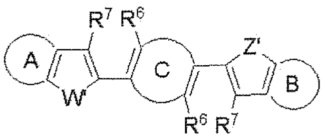

(2)

wherein W' , Z' , $R^6$ and $R^7$ are each as defined in [17], the method comprising a step of reacting a compound represented by the formula (4):

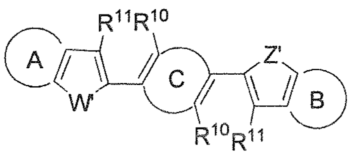

(4)

wherein a ring structure A, a ring structure B, a ring structure C, W' and Z' are as defined in [17], $R^{10}$ and $R^{11}$ independently represent a hydrogen atom or a halogen atom, at least one of $R^{10}$ and $R^{11}$ is a hydrogen atom, with a halogenating agent.

[26] The method according to [25], wherein $R^{10}$ and $R^{11}$ independently represent a hydrogen, bromine or iodine atom, and at least one of $R^{10}$ and $R^{11}$ is a hydrogen atom.

[27] The method according to [25], wherein W' and Z' independently represent a sulfur atom or an oxygen atom.

[28] A method for producing a tetrahalogen compound represented by the formula (3):

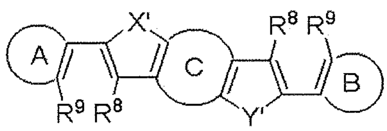

(3)

wherein X' , Y' , $R^8$ and $R^9$ are each as defined in [17], the method comprising a step of reacting a compound represented by the formula (5):

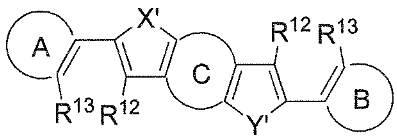

(5)

wherein a ring structure A, a ring structure B, a ring structure C, X' and Y' are as defined in [17], $R^{12}$ and $R^{13}$ independently represent a hydrogen atom or a halogen atom, and at least one of $R^{12}$ and $R^{13}$ is a hydrogen atom, with a halogenating agent.

[29] The method according to [28], wherein $R^{12}$ and $R^{13}$ independently represent a hydrogen atom, a bromine atom or an iodine atom, and at least one of $R^{12}$ and $R^{13}$ is a hydrogen atom.

[30] The method according to [28] or [29], wherein X' and Y' independently represent a sulfur atom or an oxygen atom.

[31] An organic semiconductor device comprising the compound according to any one of [1] to [16].

[32] A conductive thin film comprising the compound according to any one of [1] to [16].

[33] A light emitting thin film comprising the compound according to any one of [1] to [16].

[34] An organic semiconductor thin film comprising the compound according to any one of [1] to [16].

[35] The organic semiconductor thin film according to [34], wherein a carrier mobility is $10^{-6}$ $cm^2/Vs$ or more.

[36] An organic transistor comprising the organic semiconductor thin film according to [34] to [35].

[37] A light emitting element comprising the light emitting thin film according to [33].

Brief Description of the Drawings

Fig. 1 is a schematic view of the respective organic transistors obtained in Examples 17, 19, 21, 23 and 25.

Fig. 2 is a schematic view of an organic transistor obtained in Example 15.

Explanation of Symbols

11, 21: SUBSTRATE
12, 22: GATE ELECTRODE
13, 23: GATE INSULATING FILM
14, 24: SOURCE ELECTRODE
15, 25: DRAIN ELECTRODE
16, 26: ORGANIC SEMICONDUCTOR LAYER

Best Mode for Carrying Out the Invention

The present invention will be described in detail below.

The compound I of the present invention is represented by the above-described formula (1).

The above compound I includes pyrrole or pyrrole having a substituent on a nitrogen atom, as shown in the formula (1), and also has a heteroacene skeleton having 7 or more rings as a basic structure.

In the formula (1), a ring structure A and a ring structure B independently represent an aromatic ring which may be substituted, or a heterocyclic ring which may be substituted.

Examples of the aromatic ring in the present invention include benzene, naphthalene, anthracene, phenanthrene, fluorene, and the like, and preferably benzene and naphthalene.

The heterocyclic ring in the present invention generally has 5 to 12 constituent atoms, and has a ring structure containing carbon and hetero atoms. Examples of the hetero atom include O, Se, S, N, and the like. The heterocyclic ring preferably has 1 or 2 hetero atom(s).

Examples of the heterocyclic ring include monocyclic heterocyclic rings such as a thiophene ring, a furan ring, a selenophene ring, a pyrrole ring, an oxazole ring, a thiazole ring, a pyridine ring, a pyrazine ring, a pyrimidine ring, and a pyridazine ring; and bicyclic heterocyclic rings such as a thieno[3,2-b]thiophene ring, a furo[3,2-b]furan ring, a thieno[3,2-b]furan ring, a benzo[b]thiophene ring, and a benzo[b]furan ring.

In the ring structure A and the ring structure B, the aromatic ring and the heterocyclic ring are preferably a monocyclic or bicyclic ring, more preferably a benzene ring, a thiophene ring, a furan ring, a selenophene ring, a pyrrole ring, a thiazole ring, a thieno[3,2-b]thiophene ring, a furo[3,2-b]furan ring, a thieno[3,2-b]furan ring, a benzo[b]thiophene ring, a benzo[b]furan ring, or a naphthalene ring, still more preferably a benzene ring, a thiophene ring subjected to annulation at the 2,3-positions, a furan ring subjected to annulation at the 2,3-positions, a benzo[b]thiophene ring subjected to annulation at the 2,3-positions, or a benzo[b]furan ring subjected to annulation at the 2,3-positions, and most preferably a benzene ring.

In the present invention, examples of the substituent in the aromatic ring and the heterocyclic ring include halogen, a cyano group, a nitro group, an aryl group which may be substituted with halogen, a heteroaryl group which may be substituted with halogen, a substituent iii, a substituent iv, and the like, and preferably halogen, a substituent iii, and a substituent iv.

Examples of the halogen in the present invention include fluorine, chlorine, bromine, and iodine, and preferably fluorine, bromine, and iodine.

In the case where the substituent is an aryl group which may be substituted with halogen, the aryl group is preferably a phenyl group or a naphthyl group.

In the case where the substituent is a heteroaryl group which may be substituted with halogen, the heteroaryl group is preferably monocyclic or bicyclic, more preferably a thiophenyl group, a furyl group, a thiazolyl group, a thieno[3,2-b]thiophenyl group, a furo[3,2-b]furanyl group, a thieno[3,2-b]furanyl group, a benzo[b]thiophenyl group, or a benzo[b]furanyl group, and more preferably a thiophenyl group, a furyl group, a thieno[3,2-b]thiophenyl group, a benzo[b]thiophenyl group, or a benzo[b]furyl group.

In the present description, the substituent iii is a group selected from Group Q shown below and the substituent iv is a group selected from Group Q shown below, which is substituted with a fluorine atom.

Group Q consists of an alkyl group, an alkoxy group, an alkyl-substituted aryl group, an alkoxy-substituted aryl group, an alkyl-substituted heteroaryl group, an alkoxy-substituted heteroaryl group, an alkenyl group, an alkynyl group, an alkylthio group, an alkylcarbonyl group, an alkoxycarbonyl group, a (trialkyl)silyl group, and a (dialkyl)amino group. Each group of Group Q is preferably has 1 to 30 carbon atoms, and more preferably 1 to 20 carbon atoms.

The substituent iii is preferably a group selected from Group Q1 consisting of an alkyl group, an alkoxy group, an alkyl-substituted aryl group, an alkoxy-substituted aryl group, and an alkyl-substituted heteroaryl group, and more preferably a group selected from Group Q2 consisting of an alkyl group having 1 to 20 carbon atoms, an alkoxy group having 1 to 20 carbon atoms, an aryl group substituted with an alkyl having 1 to 20 carbon atoms, an aryl group substituted with an alkoxy having 1 to 20 carbon atoms, and a heteroaryl group substituted with an alkyl having 1 to 20 carbon atoms. The substituent iv is preferably a group in which a group selected from Group Q1 is substituted with a fluorine atom, and more preferably a group in which a group selected from Group Q2 is substituted with a fluorine atom.

The ring structure A and the ring structure B are preferably aromatic rings, heterocyclic rings, aromatic rings substituted with a substituent iii, aromatic rings substituted with a substituent iv, heterocyclic rings substituted with a substituent iii, heterocyclic rings substituted with a substituent iv, halogen-substituted aromatic rings, or halogen-substituted heterocyclic rings, more preferably benzene rings, benzene rings substituted with a substituent iii, benzene rings substituted with a substituent iv, halogen-substituted benzene rings, naphthalene rings, naphthalene rings substituted with a substituent iii, naphthalene rings substituted with a substituent iv, halogen-substituted naphthalene rings, benzo[b]thiophene rings substituted with a substituent iii, benzo[b]thiophene ring substituted with a substituent iv, benzo[b]thiophene ring, halogen-substituted benzo[b]thiophene rings, benzo[b]furan rings, benzo[b]furan rings substituted with a substituent iii, benzo[b]furan rings substituted with a substituent iv, or halogen-substituted benzo[b]furan rings, and most preferably benzene rings, benzene rings substituted with a substituent iii, benzene rings substituted with a substituent iv, or halogen-substituted benzene rings, respectively.

The ring structure C represents a benzene ring which may be substituted, a hetero[3,2-b]heterole ring, or a benzo[1,2-b:4,5-b']diheterole ring which may be substituted.

Examples of the benzene ring which may be substituted include a benzene ring, or a benzene ring substituted with halogen, a cyano group, a nitro group, halogen, a substituent iii or a substituent iv, and preferably a benzene ring, or a benzene ring substituted with halogen, a substituent iii or a substituent iv.

Examples of the hetero atom in the hetero[3,2-b]heterole ring and benzo[1,2-b:4,5-b']diheterole ring include S, Se, N, and O.

Examples of the hetero[3,2-b]heterole ring include a thieno[3,2-b]thiophene ring, a furo[3,2-b]furan ring, a seleno[3,2-b]selenophene ring, a thieno[3,2-b]furan ring, a thieno[3,2-b]selenophene ring, a seleno[3,2-b]furan ring, and a thieno[2,3-f][1]benzofuran.

Examples of the benzo[1,2-b:4,5-b']diheterole ring include a benzo[1,2-b:4,5-b']dithiophene ring, a benzo[1,2-b:4,5-b']difuran ring, or benzo[1,2-b:4,5-b']diselenophene ring, and those in which these groups are substituted with the substituent in the above-described aromatic ring and the heterocyclic ring.

Examples of the substituent in the ring structure C include halogen, a cyano group, a nitro group, halogen, a substituent iii, and a substituent iv.

The ring structure C is preferably a benzene ring which may be substituted, a hetero[3,2-b]heterole ring in which a hetero atom is O, S and/ or N, or a benzo [1,2-b:4,5-b']diheterole ring which may be substituted, in which a hetero atom is O, S and/or N, more preferably a benzene ring which may be substituted, a thieno[3,2-b]thiophene ring, a furo[3,2-b]furan ring, a benzo[1,2-b:4,5-b']dithiophene ring which may be substituted, or a benzo[1,2-b:4,5-b']difuran ring which may be substituted, still more preferably a benzene ring, a benzene ring substituted with a substituent iii, a benzene ring substituted with a substituent iv, a halogen-substituted benzene ring, a thieno[3,2-b]thiophene ring, a benzo [1,2-b:4,5-b']dithiophene ring, a benzo[1,2-b:4,5-b']dithiophene ring substituted with a substituent iii, a benzo[1,2-b:4,5-b']dithiophene ring substituted with a substituent iv, a halogen-substituted benzo[1,2-b:4,5-b']dithiophene ring, a benzo [1,2-b:4,5-b']difuran ring, a benzo[1,2-b:4,5-b']difuran ring substituted with a substituent iii, a benzo[1,2-b:4,5-b']difuran ring substituted with a substituent iv, or a halogen-substituted benzo[1,2-b:4,5-b']difuran ring, particularly preferably a benzene ring, a benzene ring substituted with a substituent iii, a benzene ring substituted with a substituent iv, a halogen-substituted benzene ring, or a thieno[3,2-b]thiophene ring, and most preferably a benzene ring, a benzene ring substituted with an alkyl group having 1 to 16 carbon atoms, a fluorobenzene ring, or a thieno[3,2-b]thiophene ring.

In the present invention, W, X, Y and Z independently represent a sulfur atom, an oxygen atom, a selenium atom, a tellurium atom, $SO_2$, ($R^1$) -C- ($R^2$) ($R^3$) -Si-($R^4$) , or N- ($R^5$) . At least one of W, X, Y and Z is N- ($R^5$).

In the compound I of the present invention, it is preferred that W and Z have the same structure, and X and Y have the same structure since it becomes easy to synthesize the compound.

In the compound I of the present invention, it is more preferred that one of W and X is N-($R^5$), the other one is a sulfur atom, an oxygen atom, ($R^1$)-C-($R^2$), or ($R^3$)-Si-($R^4$), Z and W are the same, and Y and X are the same. It is still more preferred that one of W and X is N-($R^5$), the other one is a sulfur atom or an oxygen atom, Z and W are the same, and Y and X are the same.

$R^1$, $R^2$, $R^3$, $R^4$ and $R^5$ independently represent a hydrogen atom, a substituent i, a substituent ii, an aryl group which may be substituted with halogen, or a heteroaryl group which may be substituted with halogen.

In the present description, the substituent i is a group selected from Group P shown below, and the substituent ii is a group selected from Group P which is substituted shown below. Group P consists of an alkyl group, an alkoxy group, an alkyl-substituted aryl group, an alkoxy-substituted aryl group, an alkyl-substituted heteroaryl group, and an alkoxy-substituted heteroaryl group.

The alkyl group in the present invention may be straight chain or branched and examples thereof include straight chain or branched alkyl groups having 1 to 30 carbon atoms.

Examples of the alkyl group include a methyl group, an ethyl group, an n-propyl group, an isopropyl group, an n-butyl group, an s-butyl group, a t-butyl group, an n-pentyl group, a neopentyl group, an n-hexyl group, an 2-ethylhexyl group, a cyclohexyl group, an n-heptyl group, an n-octyl group, a 2-hexyloctyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, an n-nonadecyl group, an n-icosyl group, an n-henicosyl group, an n-docosyl group, an n-tricosyl group, an n-tetracosyl group, an n-pentacosyl group, an n-hexacosyl group, an n-heptacosyl group, an n-octacosyl group, an n-nonacosyl group, and an n-triacontyl group, preferably alkyl groups having 1 to 20 carbon atoms, such as a methyl group, an ethyl group, an n-propyl group, an isopropyl group, an n-butyl group, an s-butyl group, a t-butyl group, an n-pentyl group, a neopentyl group, an n-hexyl group, a 2-ethylhexyl group, an n-heptyl group, an n-octyl group, a 2-hexyloctyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, an n-nonadecyl group, and an n-icosyl group, and more preferably alkyl groups having 1 to 16 carbon atoms, such as a methyl group, an ethyl group, an n-propyl group, an n-butyl group, an n-pentyl group, an n-hexyl group, a 2-ethylhexyl group, an n-heptyl group, an n-octyl group, an n-nonyl group, an n-decyl group, an n-undecyl group, an n-dodecyl group, an n-tridecyl group, an 2-hexyloctyl group, an n-tetradecyl group, an n-pentadecyl group, and an n-hexadecyl group.

The fluoroalkyl group in the present invention may be straight chain and branched and examples thereof include straight chain or branched fluoroalkyl groups having 1 to 30 carbon atoms. Examples of the fluoroalkyl group include groups in which one or more hydrogen atoms are substituted with a fluorine atom in the above-described alkyl group.

The alkoxy group in the present invention may be straight chain or branched and examples thereof include straight chain or branched alkoxy groups usually having 1 to 30 carbon atoms.

Examples of the alkoxy group include a methoxy group, an ethoxy group, an n-propoxy group, an isopropoxy group, an n-butoxy group, an isobutoxy group, an t-butoxy group, an n-pentyloxy group, a neopentyloxy group, an n-hexyloxy group, an n-heptyloxy group, an n-octyloxy group, a 2-ethylhexyloxy group, a nonyloxy group, a decyloxy group, a 3,7-dimethyloctyloxy group, an n-undecyloxy group, an n-dodecyloxy group, an n-tridecyloxy group, an n-tetradecyloxy group, a 2-n-hexyl-n-octyloxy group, an n-pentadecyloxy group, an n-hexadecyloxy group, an n-heptadecyloxy group, an n-octadecyloxy group, an n-nonadecyloxy group, an n-icosyloxy group, an n-henicosyloxy group, an n-docosyloxy group, an n-tricosyloxy group, an n-tetracosyloxy group, an n-pentacosyloxy group, an n-hexacosyloxy group, an n-heptacosyloxy group, an n-octacosyloxy group, an n-nonacosyloxy group, an n-triacontyloxy group, a methoxymethoxy group, a methoxyethoxy group, a methoxymethoxymethoxy group, a methoxyethoxyethoxy group, and a polyethyleneglycoxy group, preferably alkoxy group having 1 to 20 carbon atoms such as an ethoxy group, an n-propoxy group, an isopropoxy group, an n-butoxy group, an isobutoxy group, a t-butoxy group, an n-pentyloxy group, an n-hexyloxy group, an n-heptyloxy group, an n-octyloxy group, a 2-ethylhexyloxy group, an nonyloxy group, a decyloxy group, a 3,7-dimethyloctyloxy group, an n-undecyloxy group, an n-dodecyloxy group, an n-tridecyloxy group, an n-tetradecyloxy group, an n-pentadecyloxy group, an n-hexadecyloxy group, an n-heptadecyloxy group, an n-octadecyloxy group, an n-nonadecyloxy group, an n-icosyloxy group, a methoxymethoxy group, a methoxyethoxy group, a methoxymethoxymethoxy group, and a methoxyethoxyethoxy group, and more preferably alkoxy groups having 1 to 16 carbon atoms such as an ethoxy group, an n-propoxy group, an n-butoxy group, an n-pentyloxy group, an n-hexyloxy group, an n-heptyloxy group, an n-octyloxy group, a 2-ethylhexyloxy group, an nonyloxy group, a decyloxy group, a 3,7-dimethyloctyloxy group, an n-undecyloxy group, an n-dodecyloxy group, an n-tridecyloxy group, an n-tetradecyloxy group, an n-pentadecyloxy group, an n-hexadecyloxy group, a methoxymethoxy group, a methoxyethoxy group, a methoxymethoxymethoxy group, and a methoxyethoxyethoxy group.

Examples of the aryl group in the present invention include phenyl, naphthyl, phenanthrenyl, furorenyl, and the like, and preferably phenyl and naphthyl.

Examples of the alkyl-substituted aryl group in the present invention include aryl groups having the above-described alkyl group as the substituent. Examples of the aryl group in the alkyl-substituted aryl group include aryl groups described above.

Examples of the alkoxy-substituted aryl group in the present invention include aryl groups having the above alkoxy group as the substituent. Examples of the aryl group in the alkoxy-substituted aryl group include the above aryl group.

The heteroaryl in the present invention is preferably a monocyclic or bicyclic aromatic ring containing at least one kind or two kinds of S, N and O, more preferably a thiophene ring, a furan ring, a thiazole ring, a thieno[3,2-b]thiophene ring, a furo[3,2-b]furan ring, a thieno[3,2-b]furan ring, a benzo[b]thiophene ring, or a benzo[b]furan ring, and still more preferably a thiophene ring, a furan ring, a thieno[3,2-b]thiophene ring, a benzo[b]thiophene ring, or a benzo[b]furan ring.

Examples of the alkyl-substituted heteroaryl group in the present invention include heteroaryl groups having the above alkyl group as the substituent. Examples of the heteroaryl group in the alkyl-substituted heteroaryl group include the above heteroaryl groups.

Examples of the alkoxy-substituted heteroaryl group in the present invention include heteroaryl groups having the above alkoxy group as the substituent. Examples of the heteroaryl group in the alkoxy-substituted heteroaryl group include the above heteroaryl groups.

The alkenyl group in the present invention may be straight chain or branched and examples thereof include a straight chain or branched alkenyl group having 2 to 30 carbon atoms.

Examples of the alkenyl group include an ethenyl group, a 1-propenyl group, a 1-butenyl group, a 1-pentenyl group, a 1-hexenyl group, a 1-cyclohexenyl group, a 1-heptenyl group, a 1-octenyl group, a 1-nonenyl group, a 1-decenyl group, a 1-undecenyl group, a 1-dodecenyl group, a 1-tridecenyl group, a 1-tetradecenyl group, a 1-pentadecenyl group, a 1-hexadecenyl group, a 1-heptadecenyl group, a 1-octadecenyl group, a 1-nonadecenyl group, a 1-icosenyl group, a 1-henicosenyl group, a 1-docosenyl group, a 1-tricosenyl group, a 1-tetracosenyl group, a 1-pentacosenyl group, a 1-hexacosenyl group, a 1-heptacosenyl group, a 1-octacosenyl group, a 1-nonacosenyl group, and a 1-triacontenyl group, preferably an alkenyl group having 2 to 20 carbon atoms such as an ethenyl group, a 1-propenyl group, a 1-butenyl group, a 1-pentenyl group, a 1-hexenyl group, a 1-heptenyl group, a 1-octenyl group, a 1-nonenyl group, a 1-decenyl group, a 1-undecenyl group, a 1-dodecenyl group, a 1-tridecenyl group, a 1-tetradecenyl group, a 1-pentadecenyl group, a 1-hexadecenyl group, a 1-heptadecenyl group, a 1-octadecenyl group, a 1-nonadecenyl group, and a 1-icosenyl group, and more preferably an alkenyl group having 2 to 16 carbon atoms such as an ethenyl group, a 1-propenyl group, a 1-butenyl group, a 1-pentenyl group, a 1-hexenyl group, a 1-heptenyl group, a 1-octenyl group, a 1-nonenyl group, a 1-decenyl group, a 1-undecenyl group, a 1-dodecenyl group, a 1-tridecenyl group, a 1-tetradecenyl group, a 1-pentadecenyl group, and a 1-hexadecenyl group.

The alkynyl group in the present invention may be a straight chain or branched and examples thereof include straight chain or branched alkynyl groups having 2 to 30 carbon atoms.

Specific examples of the alkynyl group include an ethynyl group, a 1-propynyl group, a 1-butynyl group, a 1-pentynyl group, a 1-hexynyl group, a 1-heptynyl group, a 1-octynyl group, a 1-nonynyl group, a 1-decynyl group, a 1-undecynyl group, a 1-dodecynyl group, a 1-tridecynyl group, a 1-tetradecynyl group, a 1-pentadecynyl group, a 1-hexadecynyl group, a 1-heptadecynyl group, a 1-octadecynyl group, a 1-nonadecynyl group, a 1-icosynyl group, a 1-henicosynyl group, a 1-docosynyl group, a 1-tricosynyl group, a 1-tetracosynyl group, a 1-pentacosynyl group, a 1-hexacosynyl group, a 1-heptacosynyl group, a 1-octacosynyl group, a 1-nonacosynyl group, and a 1-triacontynyl group, preferably an alkynyl group having 2 to 20 carbon atoms such as an ethynyl group, a 1-propynyl group, a 1-butynyl group, a 1-pentynyl group, a 1-hexynyl group, a 1-heptynyl group, a 1-octynyl group, a 1-nonynyl group, a 1-decynyl group, a 1-undecynyl group, a 1-dodecynyl group, a 1-tridecynyl group, a 1-tetradecynyl group, a 1-pentadecynyl group, a 1-hexadecynyl group, a 1-heptadecynyl group, a 1-octadecynyl group, a 1-nonadecynyl group, and a 1-icosynyl group, and more preferably an alkynyl group having 2 to 16 carbon atoms such as an ethynyl group, a 1-propynyl group, a 1-butynyl group, a 1-pentynyl group, a 1-hexynyl group, a 1-heptynyl group, a 1-octynyl group, a 1-nonynyl group, a 1-decynyl group, a 1-undecynyl group, a 1-dodecynyl group, a 1-tridecynyl group, a 1-tetradecynyl group, a 1-pentadecynyl group, and a 1-hexadecynyl group.

The alkylthio group in the present invention may be a straight chain or branched and examples thereof include straight chain or branched alkylthio groups usually having 1 to 30 carbon atoms. Examples of the alkylthio group include a methylthio group, an ethylthio group, an n-propylthio group, an isopropylthio group, an n-butylthio group, an isobutylthio group, an s-butylthio group, a t-butylthio group, an n-pentylthio group, a cyclopentylthio group, an n-hexylthio group, a cyclohexylthio group, an n-heptylthio group, a cycloheptylthio group, an n-octylthio group, a cyclooctylthio group, a 2-ethyl-n-hexylthio group, an n-nonylthio group, an n-decylthio group, an n-undecylthio group, an n-dodecylthio group, an n-tridecylthio group, an n-tetradecylthio group, a 2-n-hexyl-n-octylthio group, an n-pentadecylthio group, an n-hexadecylthio group, an n-heptadecylthio group, an n-octadecylthio group, an n-nonadecylthio group, an n-icosylthio group, an n-henicosylthio group, an n-docosylthio group, an n-tricosylthio group, an n-tetracosylthio group, an n-pentacosylthio group, an n-hexacosylthio group, an n-heptacosylthio group, an n-octacosylthio group, an n-nonacosylthio group, and an n-triacontylthio group, preferably an alkylthio group having 2 to 20 carbon atoms such as an ethylthio group, an n-propylthio group, an isopropylthio group, an n-butylthio group, an isobutylthio group, an s-butylthio group, a t-butylthio group, an n-pentylthio group, an n-hexylthio group, a cyclohexylthio group, an n-heptylthio group, a cycloheptylthio group, an n-octylthio group, a cyclooctylthio group, a 2-ethyl-n-hexylthio group, an n-nonylthio group, an n-decylthio group, an n-undecylthio group, an n-dodecylthio group, an n-tridecylthio group, an n-tetradecylthio group, a 2-n-hexyl-n-octylthio group, an n-pentadecylthio group, an n-hexadecylthio group, an n-heptadecylthio group, an n-octadecylthio group, an n-nonadecylthio group, and an n-icosylthio group, and more preferably an alkylthio group having 2 to 16 carbon atoms such as an ethylthio group, an n-propylthio group, an n-butylthio group, an n-pentylthio group, an n-hexylthio group, a cyclohexylthio group, an n-heptylthio group, a cycloheptylthio group, an n-octylthio group, a cyclooctylthio group, a 2-ethyl-n-hexylthio group, an n-nonylthio group, an n-decylthio group, an n-undecylthio group, an n-dodecylthio group, an n-tridecylthio group, an n-tetradecylthio group, a 2-n-hexyl-n-octylthio group, an n-pentadecylthio group, and an n-hexadecylthio group.

The alkylcarbonyl group in the present invention may be straight chain or branched and examples thereof include straight chain or branched alkylcarbonyl groups usually having 2 to 30 carbon atoms, and preferably 2 to 17 carbon atoms.

Examples of the alkylcarbonyl group include a methylcarbonyl group, an ethylcarbonyl group, an n-butylcarbonyl group, an n-hexylcarbonyl group, an n-octylcarbonyl group, an n-dodecylcarbonyl group, an n-pentadecylcarbonyl group, an n-icosylcarbonyl group, and the like, and preferably an n-hexylcarbonyl group, an n-dodecylcarbonyl group, an n-pentadecylcarbonyl group, and the like.

The alkoxycarbonyl group in the present invention may be straight chain or branched and examples thereof include straight chain or branched alkoxycarbonyl groups usually having 2 to 30 carbon atoms.

Examples of the alkoxycarbonyl group include a methoxycarbonyl group, an ethoxycarbonyl group, an n-butoxycarbonyl group, an n-hexyloxycarbonyl group, an n-octyloxycarbonyl group, an n-dodecyloxycarbonyl group, an n-pentadecyloxycarbonyl group, an n-icosyloxycarbonyl group, and the like, and preferably alkoxycarbonyl groups having 2 to 17 carbon atoms such as an n-hexyloxycarbonyl group, an n-dodecyloxycarbonyl group, and an n-pentadecyloxycarbonyl group.

Examples of the (trialkyl)silyl group in the present invention include trisubstituted silyl groups substituted with an alkyl group having 1 to 30 carbon atoms.

Examples of the (trialkyl)silyl group include a trimethylsilyl group, a triethylsilyl group, a tri-n-propylsilyl group, a tri-isopropylsilyl group, a tri-n-butylsilyl group, a tri-s-butylsilyl group, a tri-t-butylsilyl group, a tri-isobutylsilyl group, a t-butyldimethylsilyl group, a tri-n-pentylsilyl group, a tri-n-hexylsilyl group, and the like, and preferably trisubstituted silyl groups substituted with an alkyl group having 1 to 6 carbon atoms, such as a trimethylsilyl group, a tri-n-propylsilyl group, a tri-n-butylsilyl group, a tri-n-pentylsilyl group, and a tri-n-hexylsilyl group.

Examples of the (dialkyl)amino group in the present invention include disubstituted amino groups substituted

with an alkyl group having 1 to 30 carbon atoms, and preferably 1 to 10 carbon atoms.

Examples of the (dialkyl)amino group preferably include a dimethylamino group, a diethylamino group, a din-propylamino group, a diisopropylamino group, a di-n-butylamino group, a di-s-butylamino group, a di-t-butylamino group, a di-isobutylamino group, a t-butylisopropylamino group, a di-n-hexylamino group, a di-n-octylamino group, di-n-decylamino group, and the like.

It is preferred that $R^1$, $R^2$, $R^3$, $R^4$ and $R^5$ independently represent a hydrogen atom, an alkyl group having 1 to 30 carbon atoms, a fluoroalkyl group having 1 to 30 carbon atoms, a phenyl group, a phenyl group substituted with an alkyl group having 1 to 30 carbon atoms, a phenyl group substituted with a fluoroalkyl group having 1 to 30 carbon atoms, a phenyl group substituted with an alkoxy group having 1 to 30 carbon atoms, a phenyl group substituted with a fluoroalkoxy group having 1 to 30 carbon atoms, a thienyl group, a thienyl group substituted with an alkyl group having 1 to 30 carbon atoms, or a thienyl group substituted with a fluoroalkyl group having 1 to 30 carbon atoms.

$R^5$ is more preferably an alkyl group having 1 to 20 carbon atoms, a fluoroalkyl group having 1 to 20 carbon atoms, a phenyl group, a phenyl group substituted with an alkyl group having 1 to 20 carbon atoms, a phenyl group substituted with a fluoroalkyl group having 1 to 20 carbon atoms, or a phenyl group substituted with an alkoxy group having 1 to 20 carbon atoms.

Preferred aspects of the compound I of the present invention are shown below.

- a compound of the formula (1) in which a ring structure A, a ring structure B and a ring structure C independently represent a benzene ring, a benzene ring substituted with a substituent iii, or a halogen-substituted benzene ring, X and Y are the same and are N-($R^5$), W and Z are the same and are sulfur atoms or oxygen atoms;
- a compound of the formula (1) in which a ring structure A, a ring structure B and a ring structure C independently represent a benzene ring, a benzene ring substituted with an alkyl group having 1 to 20 carbon atoms or a fluoroalkyl group, or fluorobenzene, X and Y are the same and are N-($R^5$), W and Z are the same and are sulfur atoms or oxygen atoms, and $R^5$ is an alkyl group having 1 to 20 carbon atoms, a fluoroalkyl group having 1 to 20 carbon atoms, a phenyl group, a phenyl group substituted with an alkyl group having 1 to 20 carbon atoms, a phenyl group substituted with a fluoroalkyl group having 1 to 20 carbon atoms, or a phenyl group substituted with an alkoxy group having 1 to 20 carbon atoms;
- a compound of the formula (1) in which a ring structure A, a ring structure B and a ring structure C independently represent a benzene ring, a benzene ring substituted with a substituent iii, or a halogen-substituted benzene ring, X and Y are the same and are sulfur atoms or oxygen atoms, W and Z are the same and are N-($R^5$);
- a compound of the formula (1) in which a ring structure A, a ring structure B and a ring structure C independently represent a benzene ring, a benzene ring substituted with an alkyl group having 1 to 20 carbon atoms or a fluoroalkyl group, or fluorobenzene, X and Y are the same and are sulfur atoms or oxygen atoms, W and Z are the same and are N- ($R^5$), and $R^5$ is an alkyl group having 1 to 20 carbon atoms, a fluo-roalkyl group having 1 to 20 carbon atoms, a phenyl group, a phenyl group substituted with an alkyl group having 1 to 20 carbon atoms, a phenyl group substituted with a fluoroalkyl group having 1 to 20 carbon atoms, or a phenyl group substituted with an alkoxy group having 1 to 20 carbon atoms;
- a compound of the formula (1) in which a ring structure A and a ring structure B independently represent a benzene ring, a benzene ring substituted with a substituent iii, or a halogen-substituted benzene ring, a ring structure C is a thieno[3,2-b]thiophene ring, X and Y are the same and are N-($R^5$), and W and Z are the same and are sulfur atoms or oxygen atoms;
- a compound of the formula (1) in which a ring structure A and a ring structure B independently represent a benzene ring, a benzene ring substituted with an alkyl group having 1 to 20 carbon atoms or a fluoroalkyl group, or a fluorobenzene ring, a ring structure C is a thieno[3,2-b]thiophene ring, X and Y are the same and are N-($R^5$), W and Z are the same and are sulfur atoms or oxygen atoms, and $R^5$ is an alkyl group having 1 to 20 carbon atoms, a fluoroalkyl group having 1 to 20 carbon atoms, a phenyl group, a phenyl group substituted with an alkyl group having 1 to 20 carbon atoms, a fluoroalkyl group having 1 to 20 carbon atoms, or a phenyl group substituted with an alkoxy group having 1 to 20 carbon atoms;
- a compound of the formula (1) in which a ring structure A and a ring structure B independently represent a benzene ring, a benzene ring substituted with a substituent iii, or a halogen-substituted benzene ring, a ring structure C is a thieno[3,2-b]thiophene ring, X and Y are the same and are sulfur atoms or oxygen atoms, and W and Z are the same and are N- ($R^5$) ;
- a compound of the formula (1) in which a ring structure A and a ring structure B independently represent a benzene ring, a benzene ring substituted with an alkyl group having 1 to 20 carbon atoms or a fluoroalkyl group, or a fluorobenzene ring, a ring structure C is a thieno[3,2-b]thiophene ring, X and Y are the same and are N-($R^5$), W and Z are the same and are sulfur atoms or oxygen atoms, and $R^5$ is an alkyl group having 1 to 20 carbon atoms, a fluoroalkyl group having 1 to 20 carbon atoms, a phenyl group, a phenyl group substituted with an alkyl group having 1 to 20 carbon atoms, a fluoroalkyl group having 1 to 20 carbon atoms, or a phenyl group substituted with an alkoxy

group having 1 to 20 carbon atoms;

- a compound of the formula (1) in which a ring structure A and a ring structure B independently represent a benzene ring, a benzene ring substituted with a substituent iii, or a halogen-substituted benzene ring, a ring structure C is a benzo[1,2-b:4,5-b']dithiophene ring, a benzo[1,2-b:4,5-b']dithiophene ring substituted with a substituent iii, a halogen-substituted benzo[1,2-b:4,5-b']dithiophene ring, a benzo[1,2-b:4,5-b']difuran ring, a benzo[1,2-b:4,5-b']difuran ring substituted with a substituent iii, or a halogen-substituted benzo[1,2-b:4,5-b']difuran ring, X and Y are the same and are N-($R^5$),and W and Z are the same and are sulfur atoms or oxygen atoms;
- a compound of the formula (1) in which a ring structure A and a ring structure B independently represent a benzene ring, a benzene ring substituted with an alkyl group having 1 to 20 carbon atoms or a fluoroalkyl group, or a halogen-substituted benzene ring, a ring structure C is a benzo[1,2-b:4,5-b']dithiophene ring, a benzo[1,2-b:4,5-b']dithiophene ring substituted with an alkyl group having 1 to 20 carbon atoms or a fluoroalkyl group, a halogen-substituted benzo [1,2-b:4,5-b']dithiophene ring, a benzo[1,2-b:4,5-b']difuran ring, a benzo[1,2-b:4,5-b']difuran ring substituted with an alkyl group having 1 to 20 carbon atoms or a fluoroalkyl group, or a fluorine-substituted benzo[1,2-b:4,5-b']difuran ring, X and Y are the same and are N-($R^5$), W and Z are the same and are sulfur atoms or oxygen atoms, and $R^5$ is an alkyl group having 1 to 20 carbon atoms, a fluoroalkyl group having 1 to 20 carbon atoms, a phenyl group, a phenyl group substituted with an alkyl group having 1 to 20 carbon atoms, a phenyl group substituted with a fluoroalkyl group having 1 to 20 carbon atoms, or a phenyl group substituted with an alkoxy group having 1 to 20 carbon atoms;
- a compound of the formula (1) in which a ring structure A and a ring structure B independently represent a benzene ring, a benzene ring substituted with a substituent iii, or a halogen-substituted benzene ring, a ring structure C is a benzo[1,2-b:4,5-b']dithiophene ring, a benzo[1,2-b:4,5-b']dithiophene ring substituted with a substituent iii, a halogen-substituted benzo[1,2-b:4,5-b']dithiophene ring, benzo[1,2-b:4,5-b']difuran ring, a benzo[1,2-b:4,5-b']difuran ring substituted with a substituent iii, or a halogen-substituted benzo[1,2-b:4,5-b']difuran ring, X and Y are the same and are sulfur atoms or oxygen atoms, and W and Z are the same and are N-($R^5$); and
- a compound of the formula (1) in which a ring structure A and a ring structure B independently represent a benzene ring, a benzene ring substituted with an alkyl group having 1 to 20 carbon atoms or a fluoroalkyl group, or a halogen-substituted benzene ring, a ring structure C is a benzo[1,2-b:4,5-b']dithiophene ring, a benzo[1,2-b:4,5-b']dithiophene ring substituted with an alkyl group having 1 to 20 carbon atoms or a fluoroalkyl group, a halogen-substituted benzo [1,2-b:4,5-b']dithiophene ring, a benzo[1,2-b:4,5-b']difuran ring, a benzo[1,2-b:4,5-b']difuran ring substituted with an alkyl group having 1 to 20 carbon atoms or a fluoroalkyl group, or a fluorine-substituted benzo[1,2-b:4,5-b']difuran ring, X and Y are the same and are sulfur atoms or oxygen atoms, W and Z are the same and are N-($R^5$) , and $R^5$ is an alkyl group having 1 to 20 carbon atoms, a fluoroalkyl group having 1 to 20 carbon atoms, a phenyl group, a phenyl group substituted with an alkyl group having 1 to 20 carbon atoms, a phenyl group substituted with a fluoroalkyl group having 1 to 20 carbon atoms, or a phenyl group substituted with an alkoxy group having 1 to 20 carbon atoms.

Examples of the compound I of the present invention include, but are not limited to, compounds shown below. Provided that n represents an integer of 0 to 30 in the respective formulas.

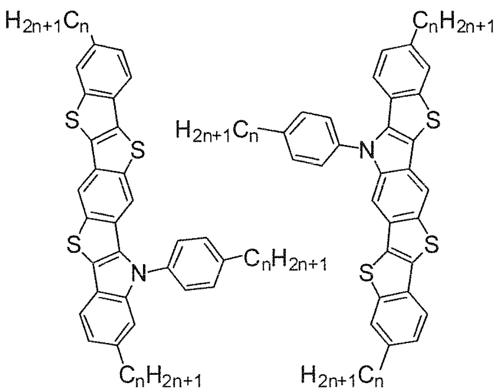

(1-1) (1-2)

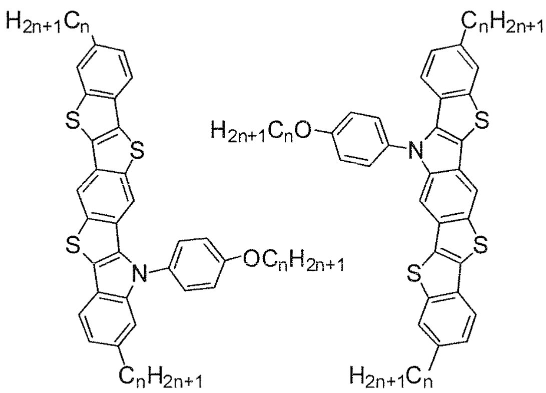

(1-3) (1-4)

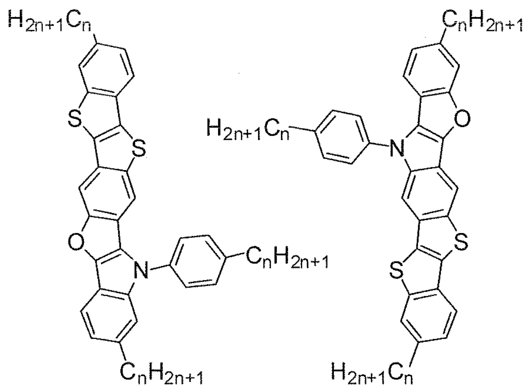

(1-5) (1-6)

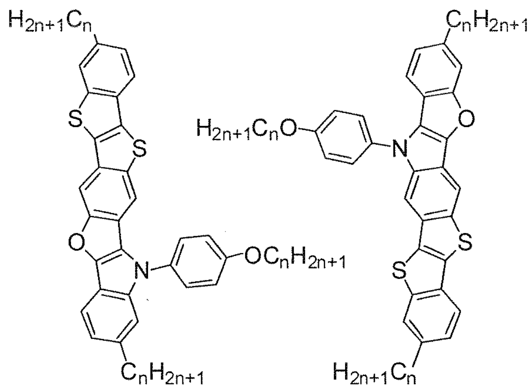

(1-7) (1-8)

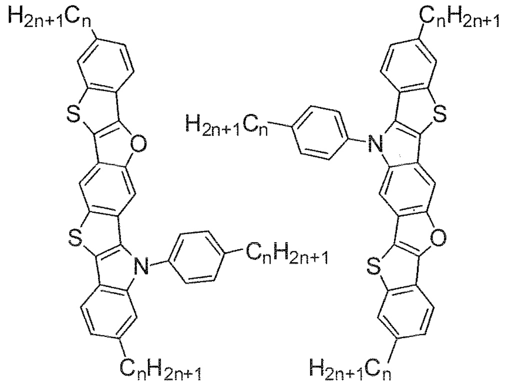

(1-9) (1-10)

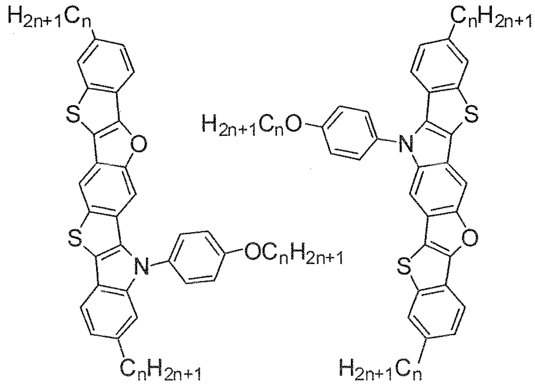

(1-11) (1-12)

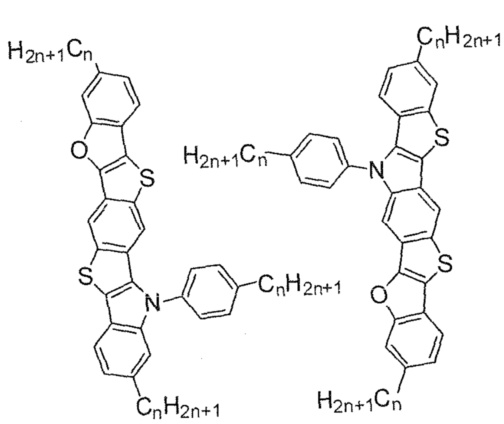

(1-13) (1-14)

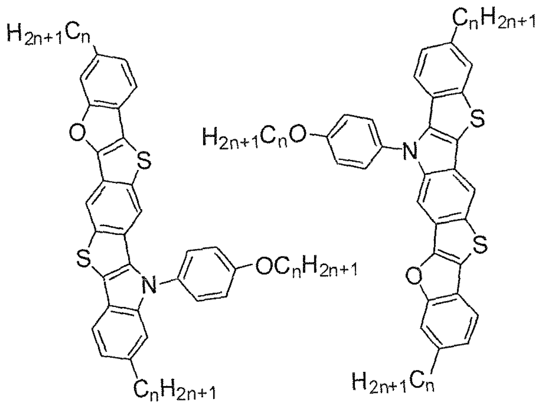

(1-15) (1-16)

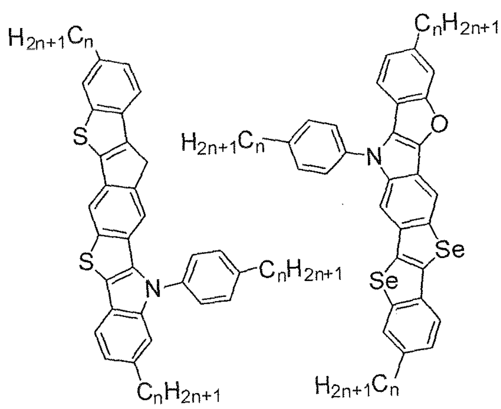

(1-17) (1-18)

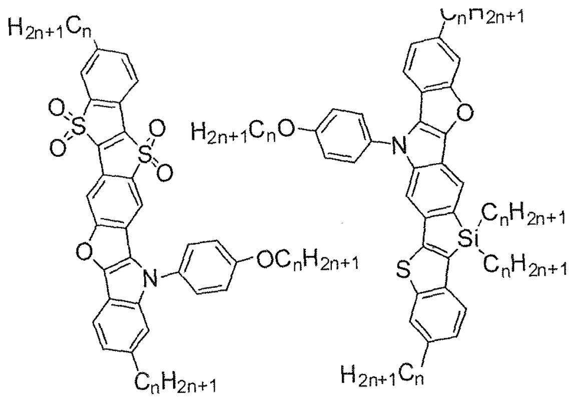

(1-19) (1-20)

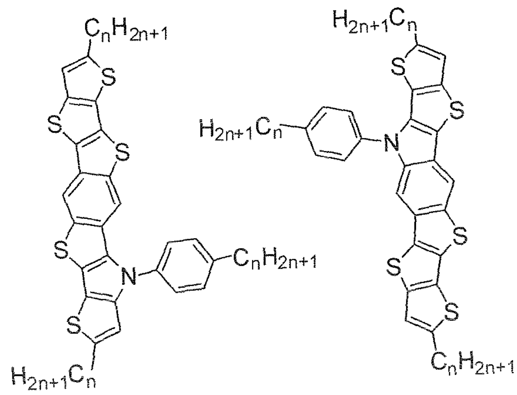

(1-21) (1-22)

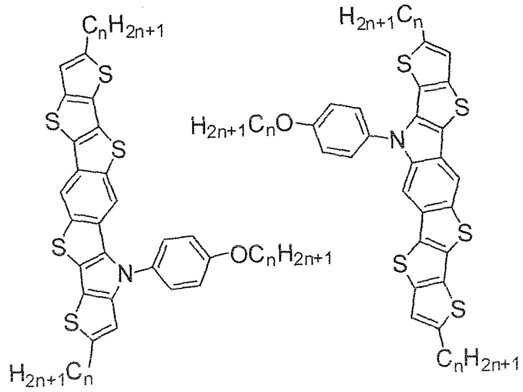

(1-23) (1-24)

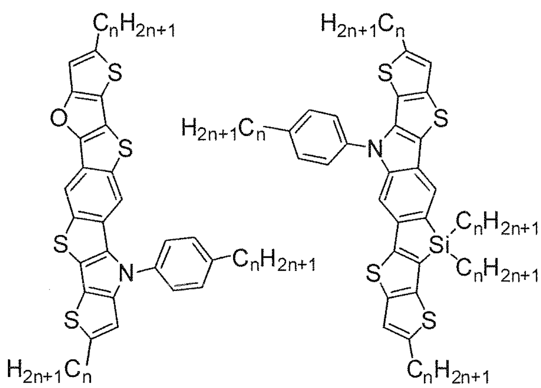

(1-25) (1-26)

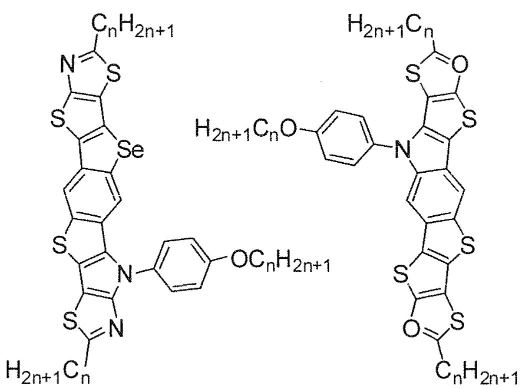

(1-27) (1-28)

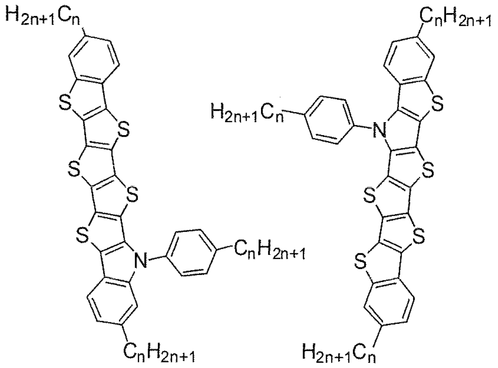

(1-29) (1-30)

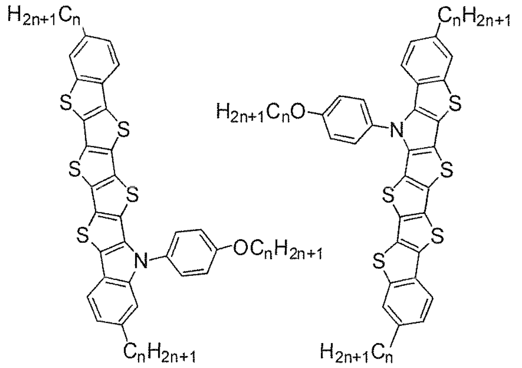

(1-31) (1-32)

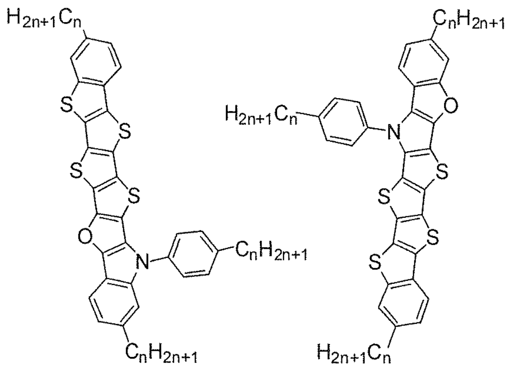

(1-33) (1-34)

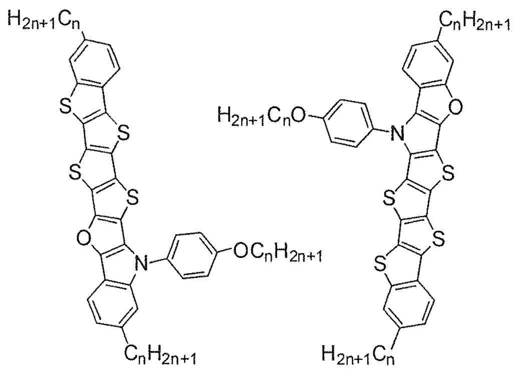

(1-35) (1-36)

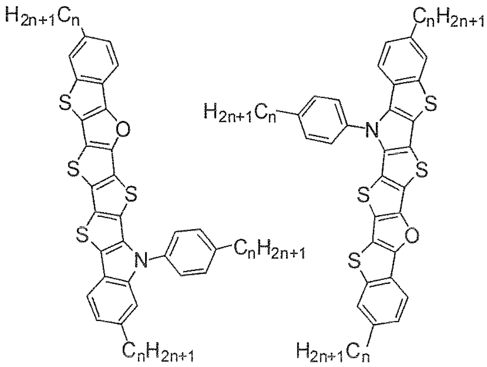

(1-37) (1-38)

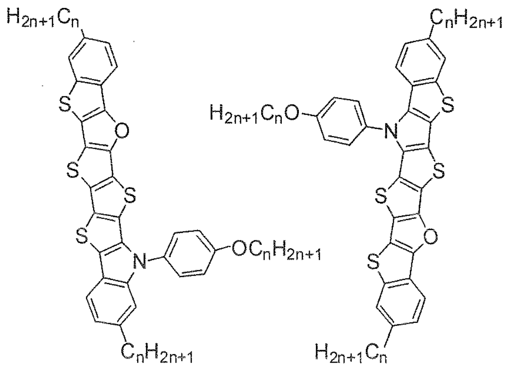

(1-39) (1-40)

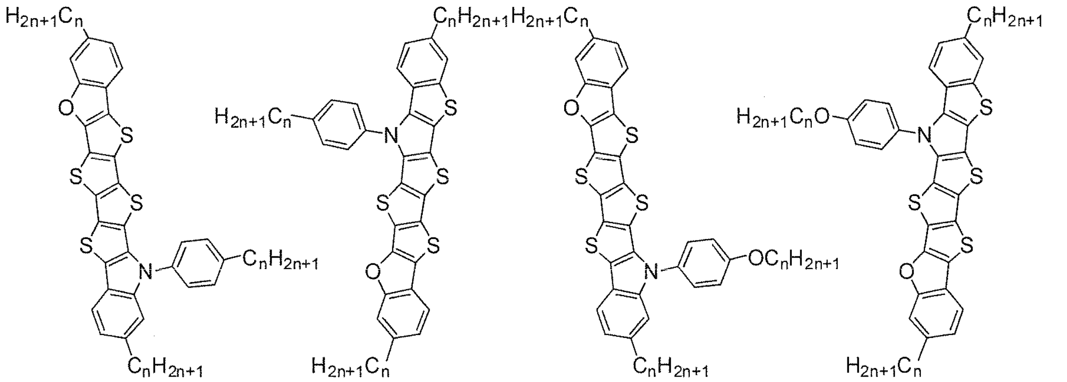

(1-41) (1-42) (1-43) (1-44)

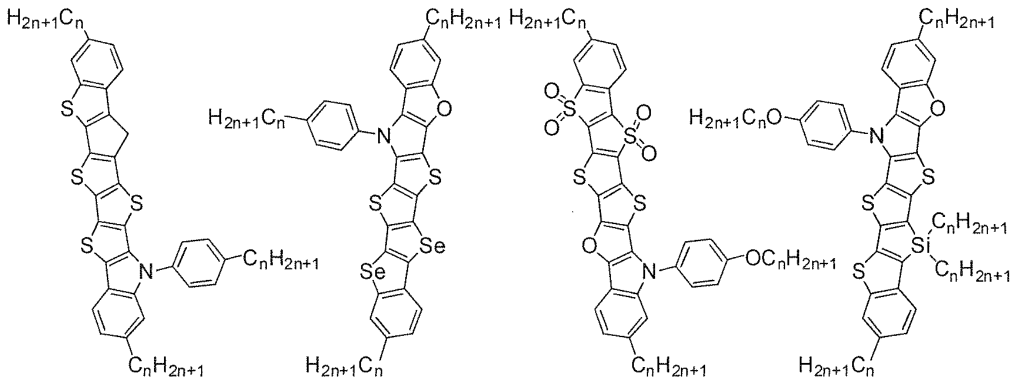

(1-45) (1-46) (1-47) (1-48)

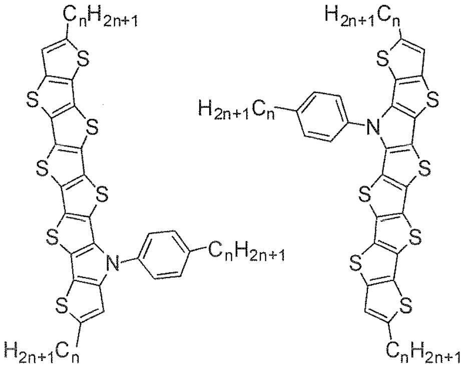

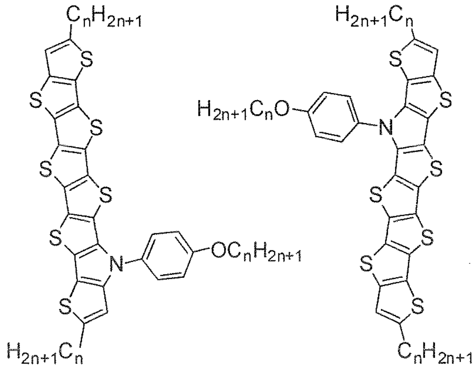

(1-49) (1-50) (1-51) (1-52)

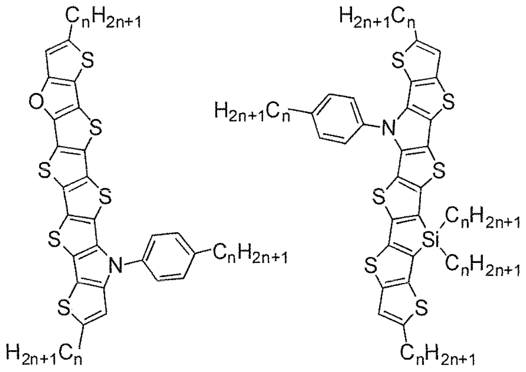
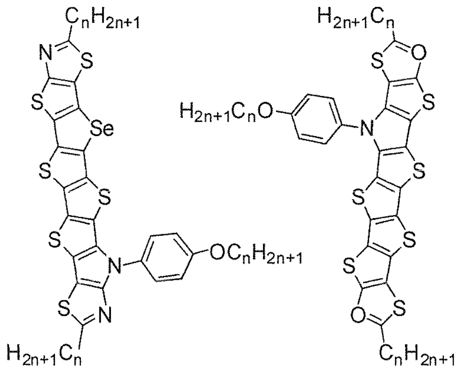

(1-53) (1-54) (1-55) (1-56)

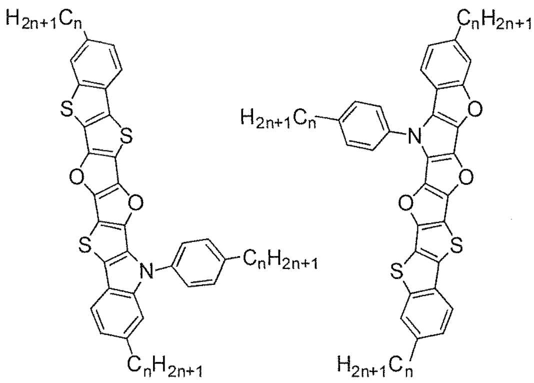
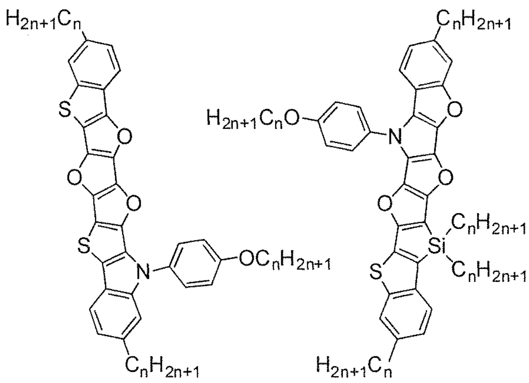

(1-57) (1-58) (1-59) (1-60)

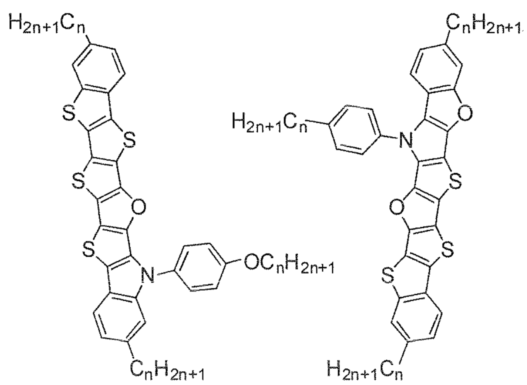
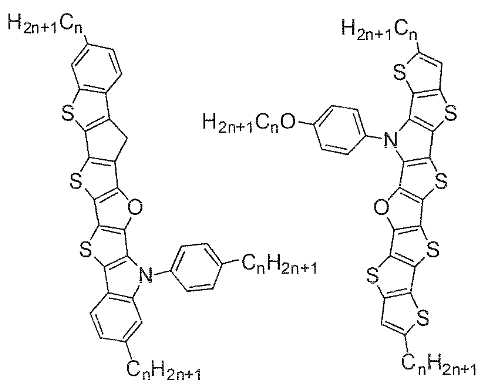

(1-61) (1-62) (1-63) (1-64)

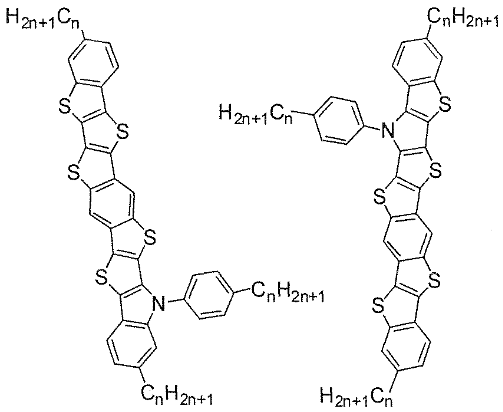

(1-65) (1-66)

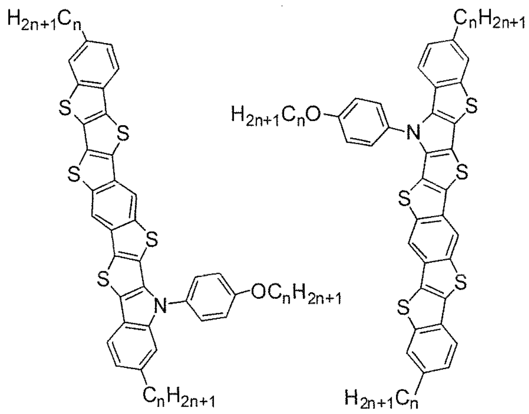

(1-67) (1-68)

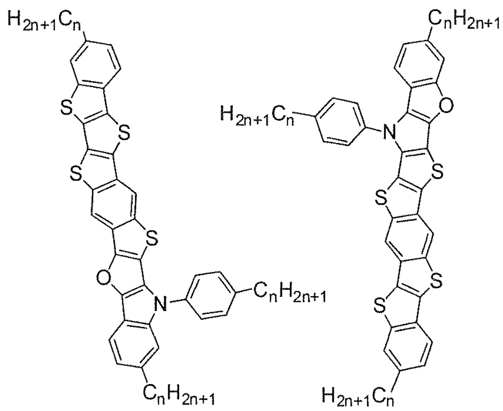

(1-69) (1-70)

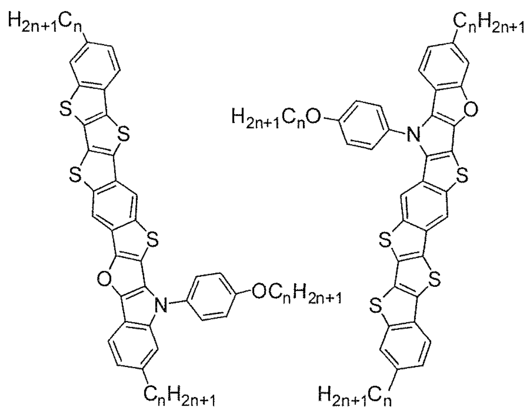

(1-71) (1-72)

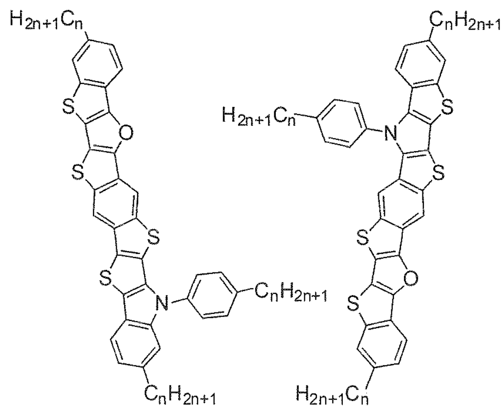

(1-73) (1-74)

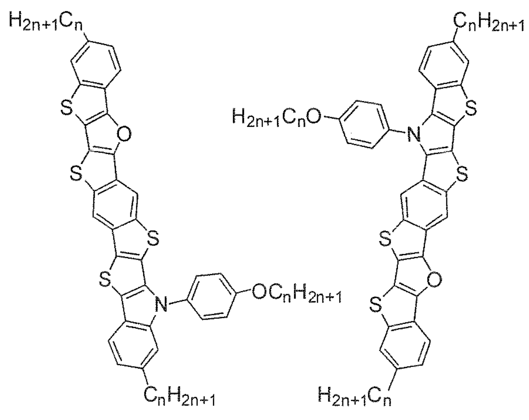

(1-75) (1-76)

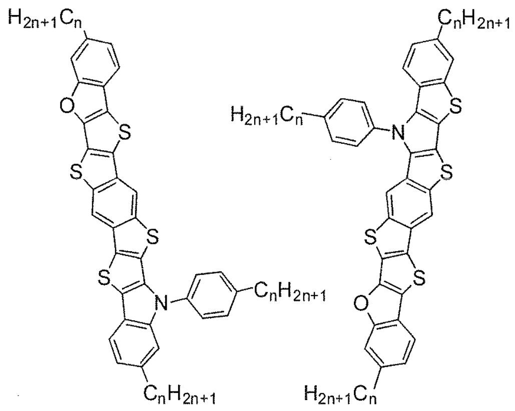

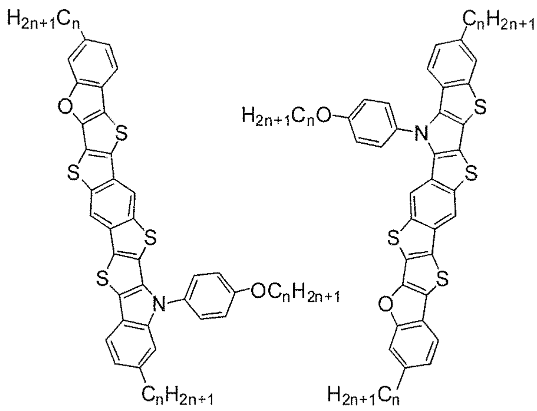

(1-77) (1-78) (1-79) (1-80)

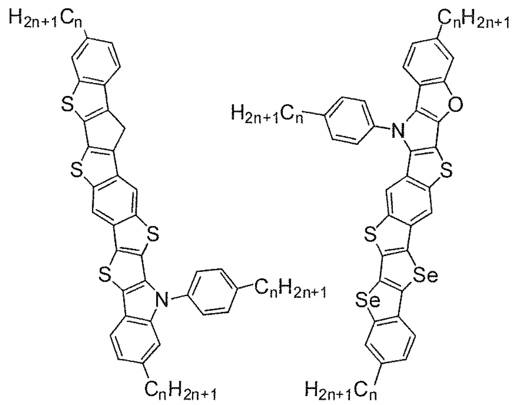

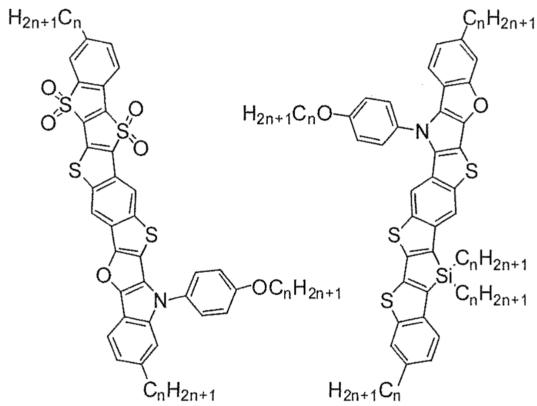

(1-81) (1-82) (1-83) (1-84)

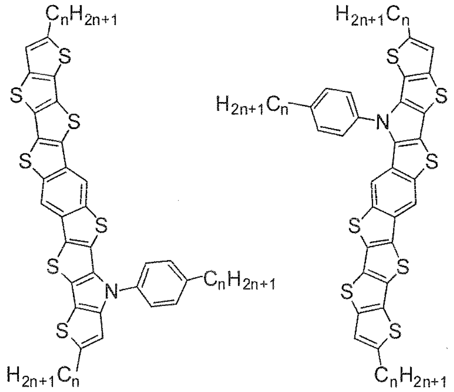

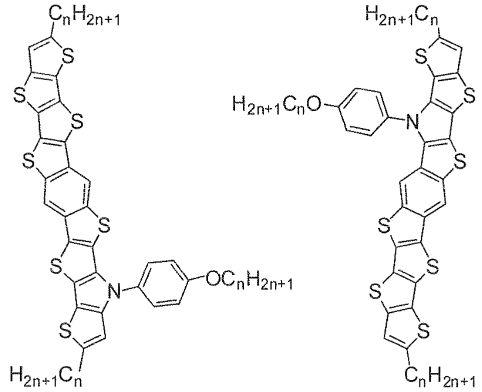

(1-85) (1-86) (1-87) (1-88)

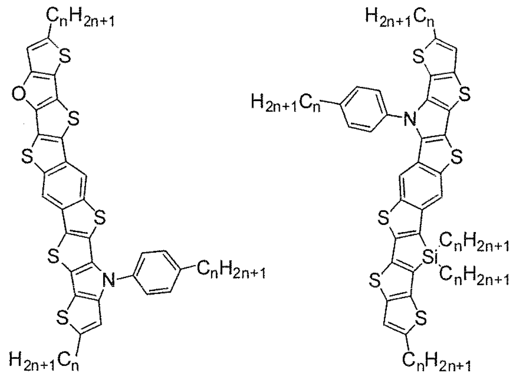

(1-89) (1-90)

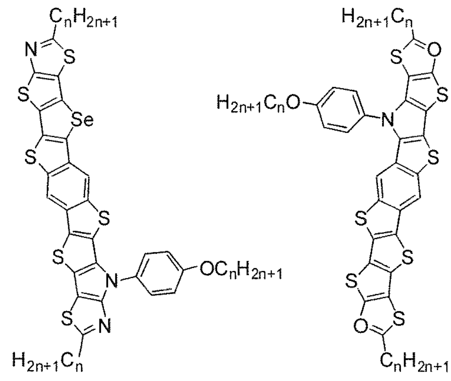

(1-91) (1-92)

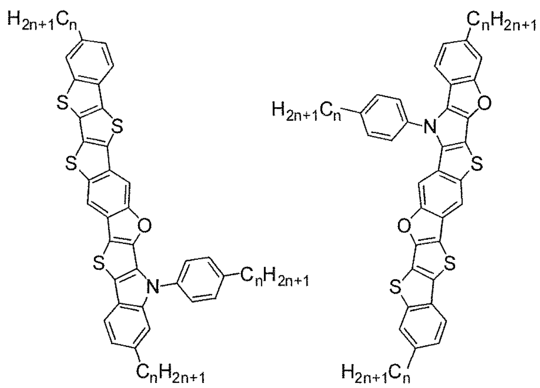

(1-93) (1-94)

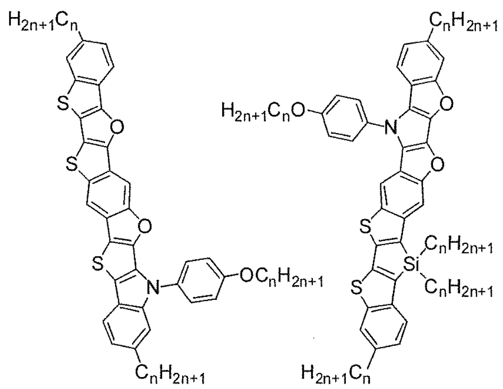

(1-95) (1-96)

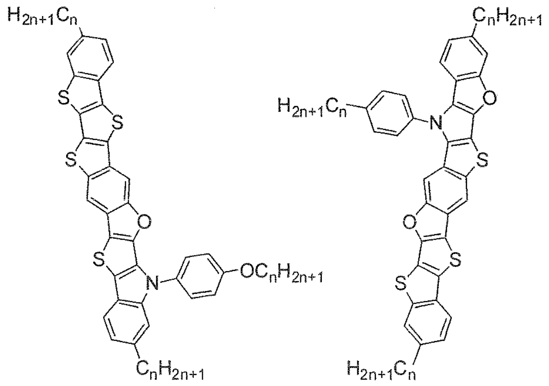

(1-97) (1-98)

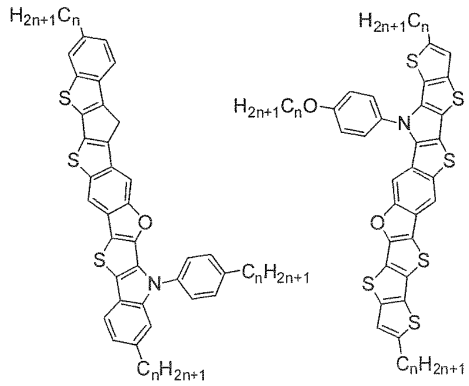

(1-99) (1-100)

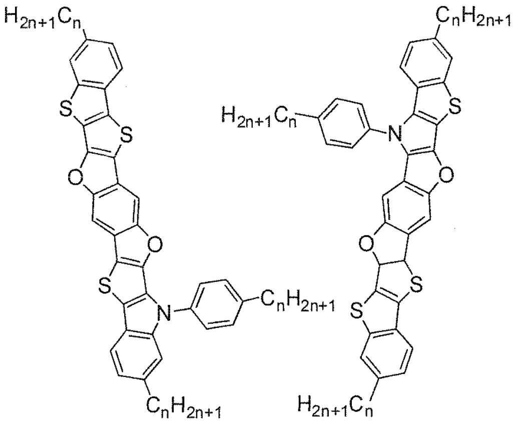

(1-101) (1-102)

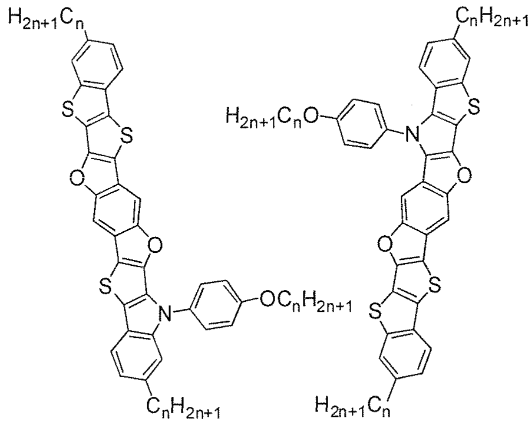

(1-103) (1-104)

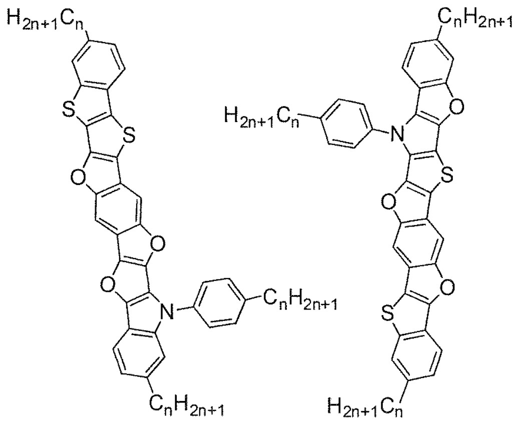

(1-105) (1-106)

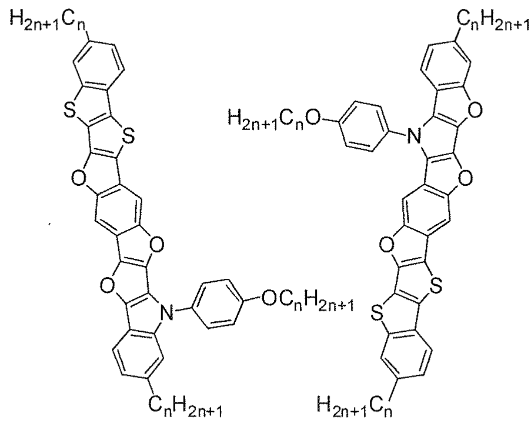

(1-107) (1-108)

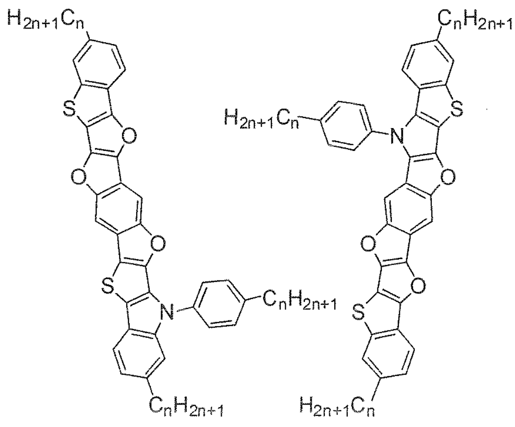

(1-109) (1-110)

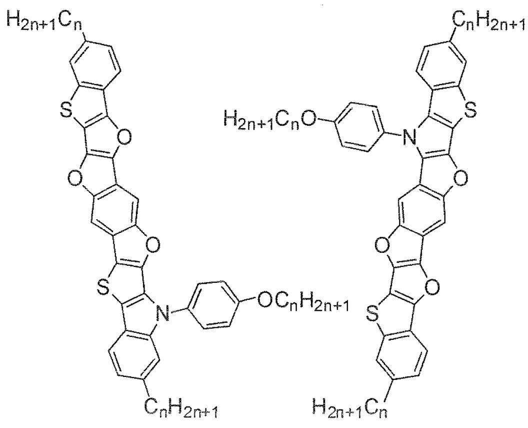

(1-111) (1-112)

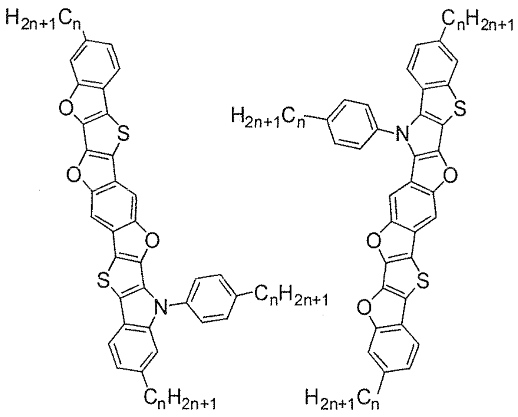

(1-113) (1-114)

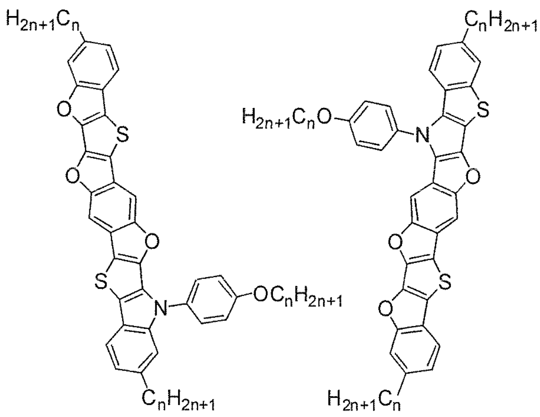

(1-115) (1-116)

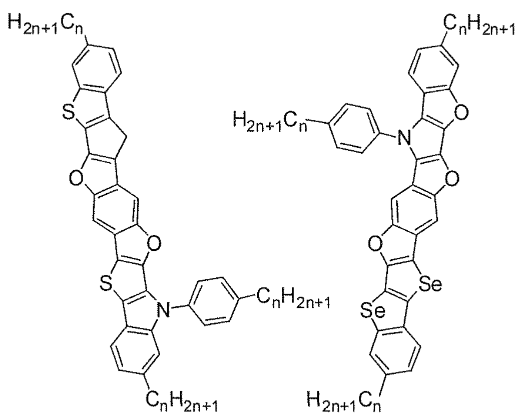

(1-117) (1-118)

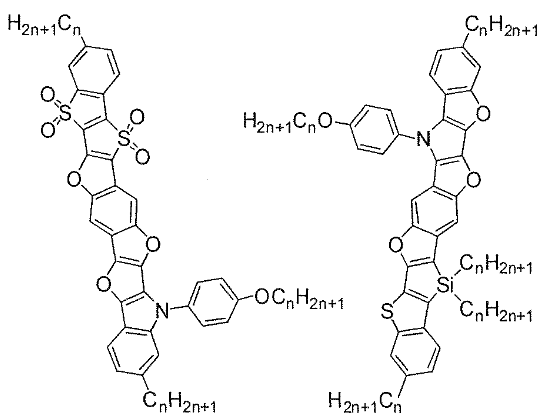

(1-119) (1-120)

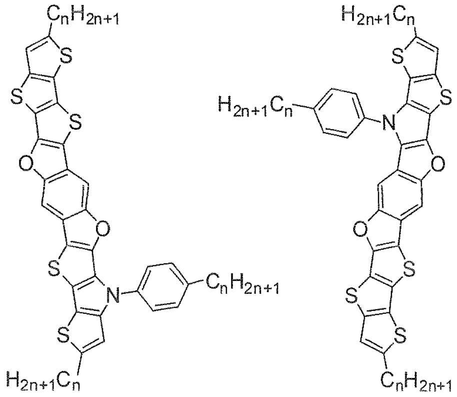

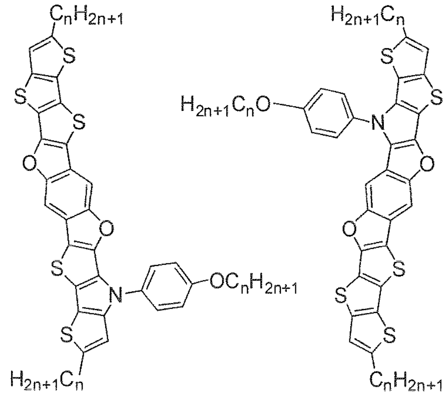

(1-121) (1-122) (1-123) (1-124)

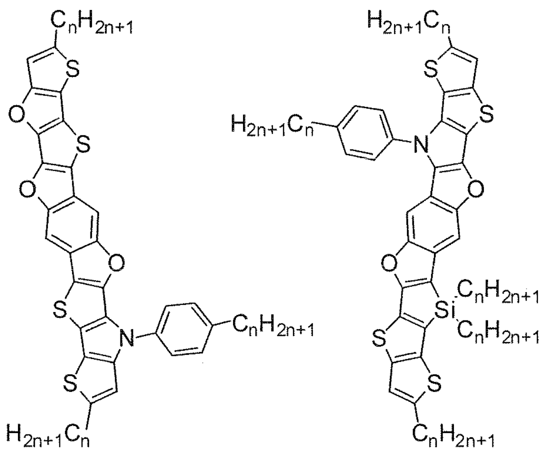

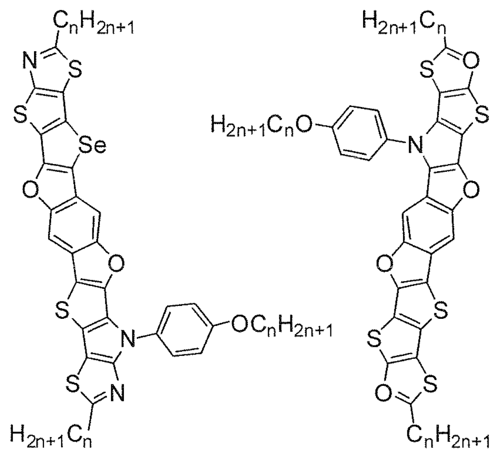

(1-125) (1-126) (1-127) (1-128)

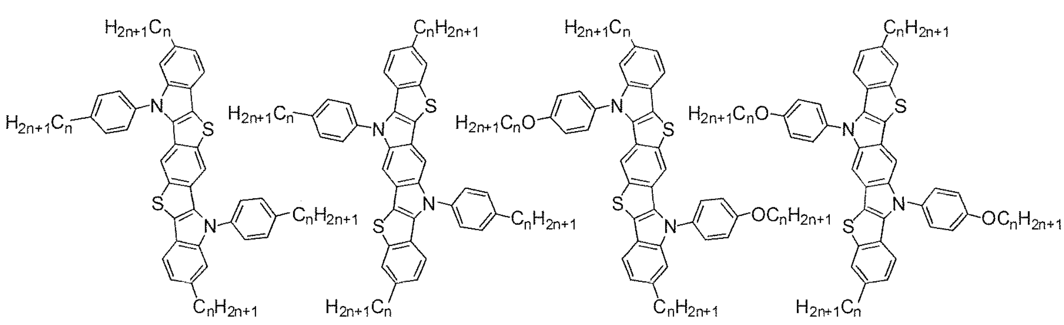

(1-129) (1-130) (1-131) (1-132)

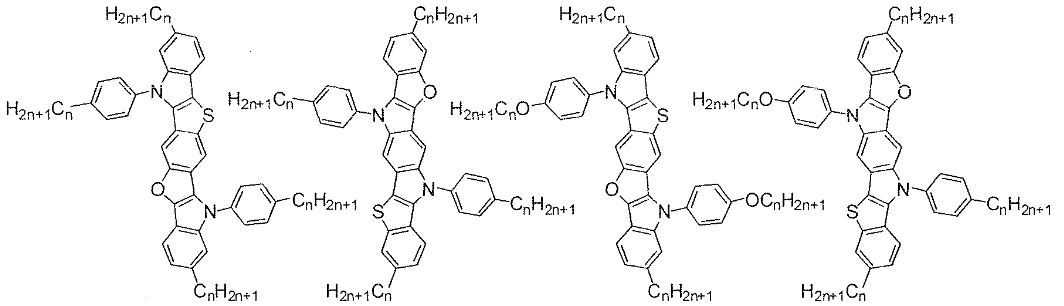

(1-133) (1-134) (1-135) (1-136)

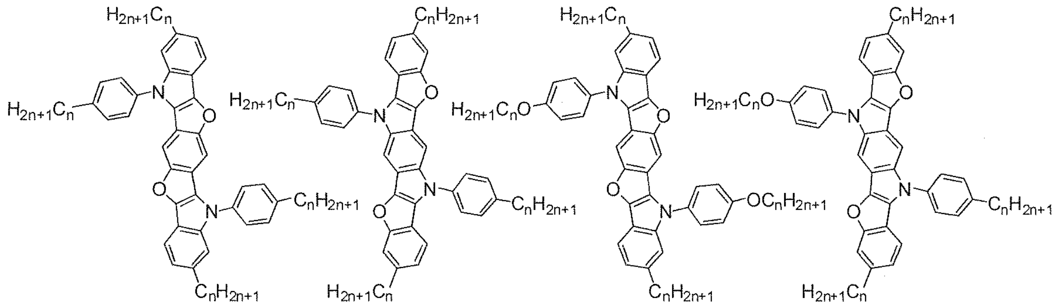

(1-137) (1-138) (1-139) (1-140)

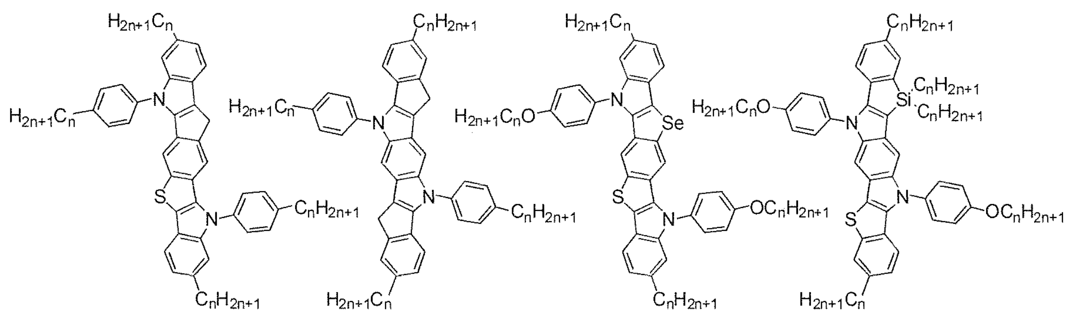

(1-141) (1-142) (1-143) (1-144)

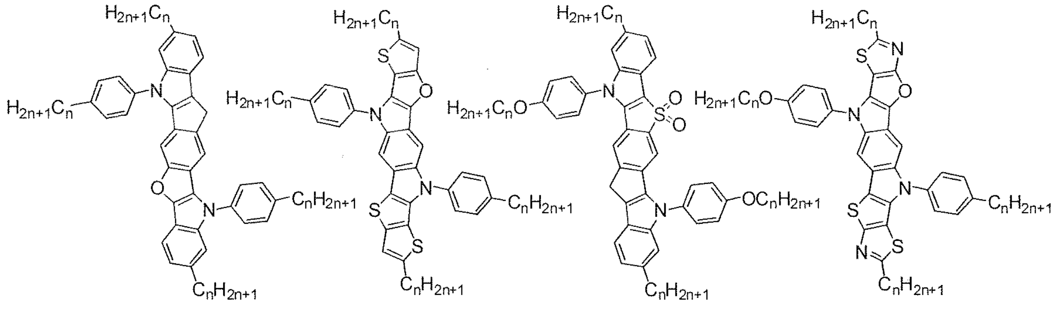

(1-145) (1-146) (1-147) (1-148)

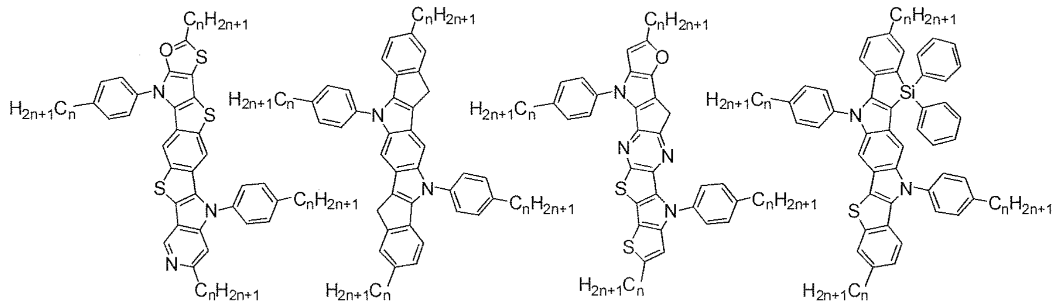

(1-149) (1-150) (1-151) (1-152)

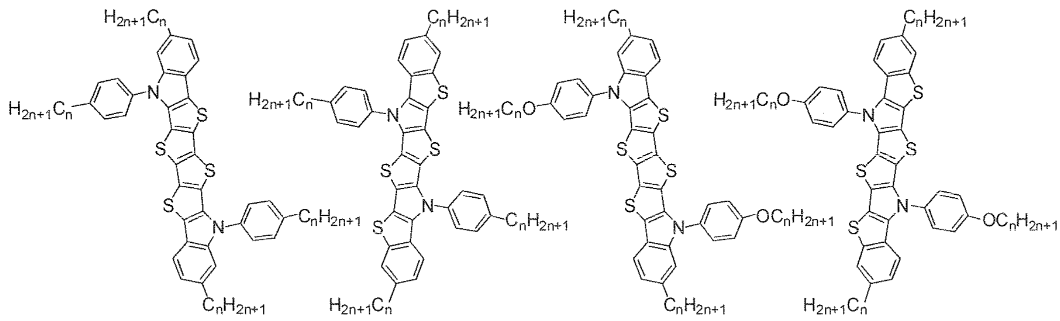

(1-153) (1-154) (1-155) (1-156)

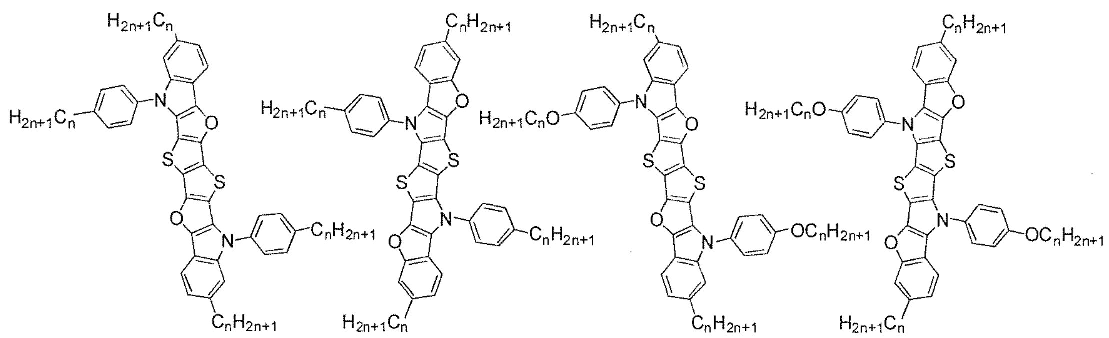

(1-157) (1-158) (1-159) (1-160)

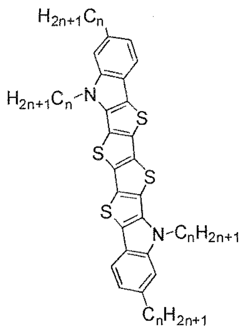

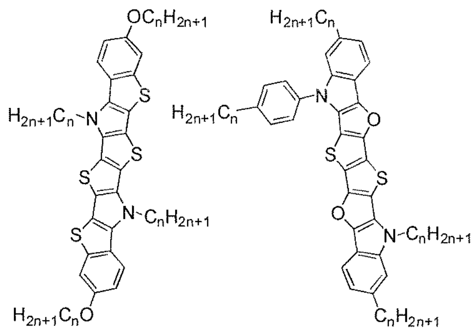

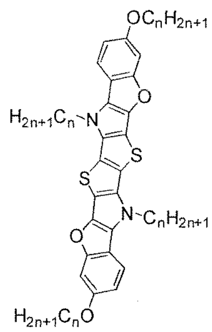

(1-161) (1-162) (1-163) (1-164)

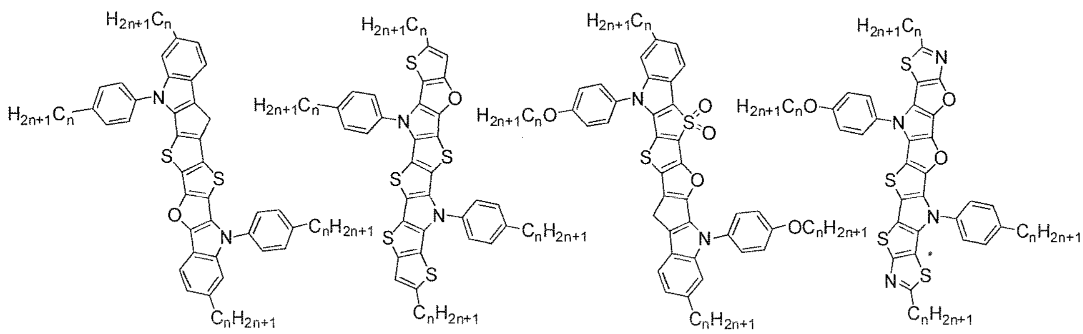

(1-165) (1-166) (1-167) (1-168)

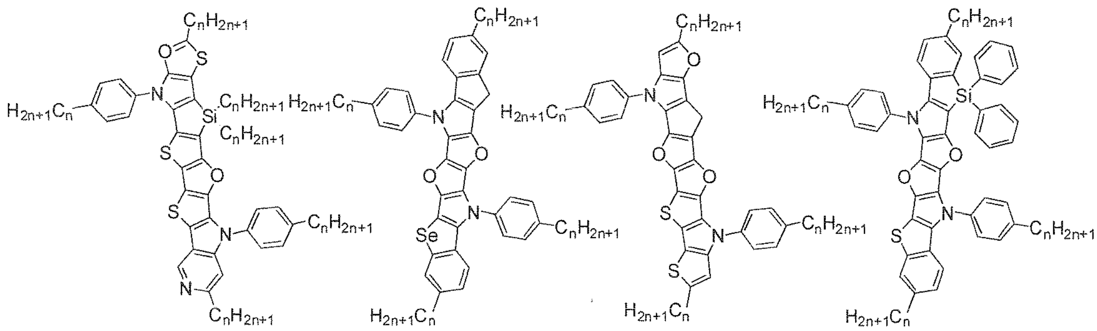

(1-169) (1-170) (1-171) (1-172)

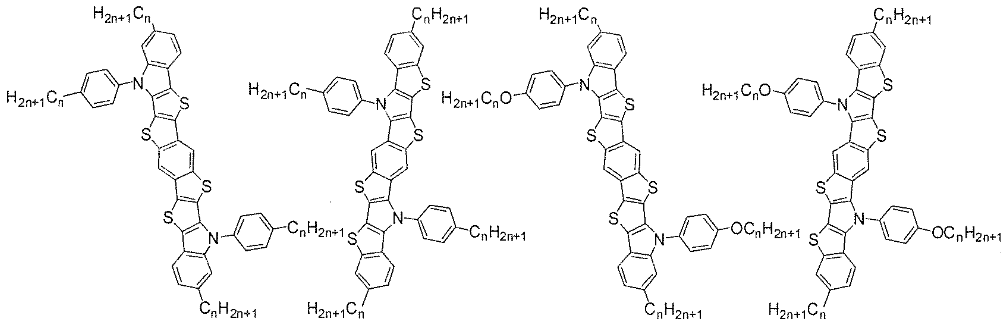

(1-173) (1-174) (1-175) (1-176)

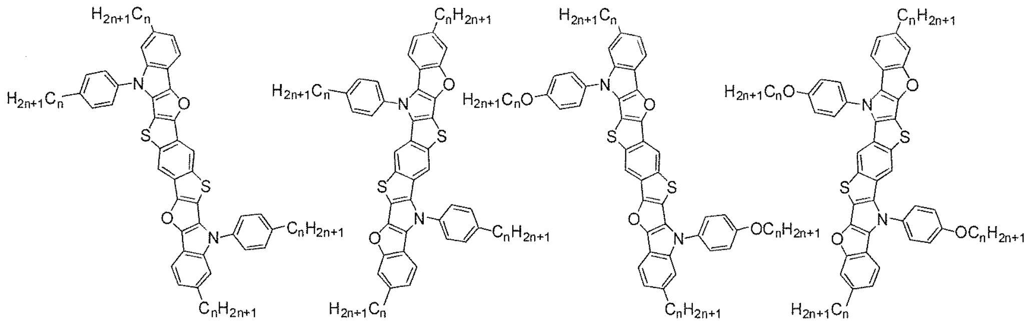

(1-177) (1-178) (1-179) (1-180)

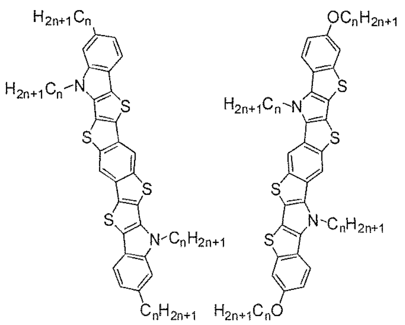

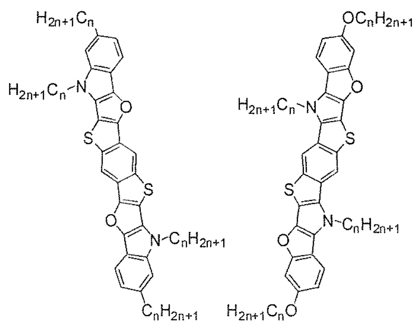

(1-181) (1-182) (1-183) (1-184)

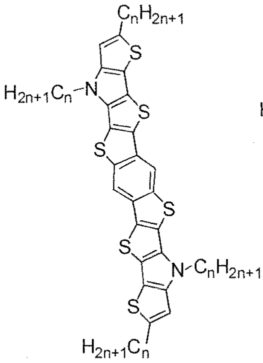
(1-185)

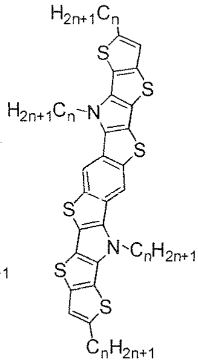
(1-186)

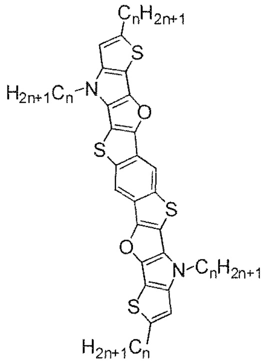
(1-187)

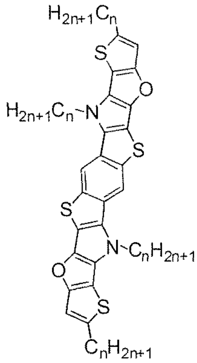
(1-188)

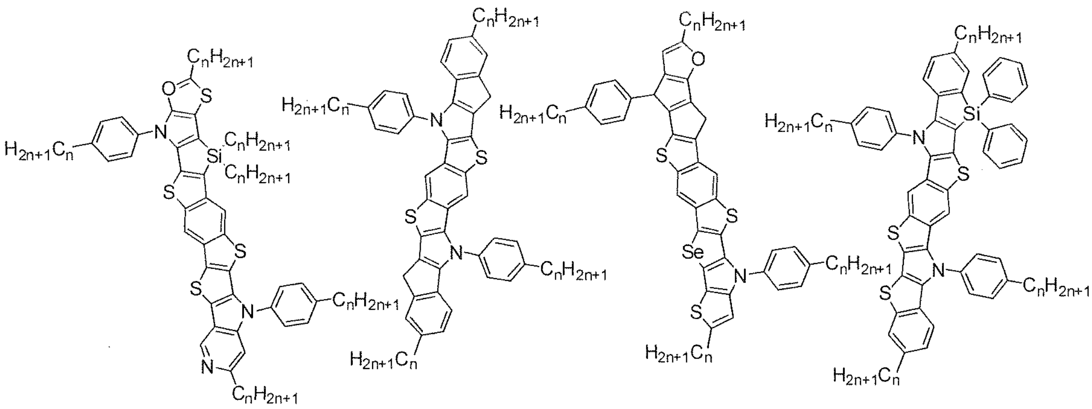
(1-189) (1-190) (1-191) (1-192)

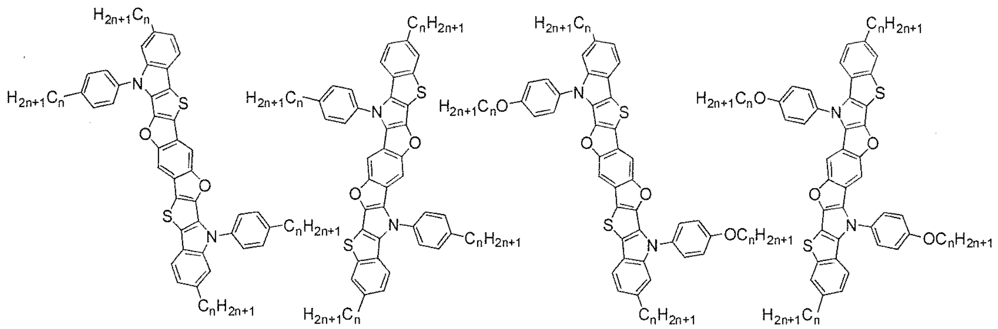
(1-193) (1-194) (1-195) (1-196)

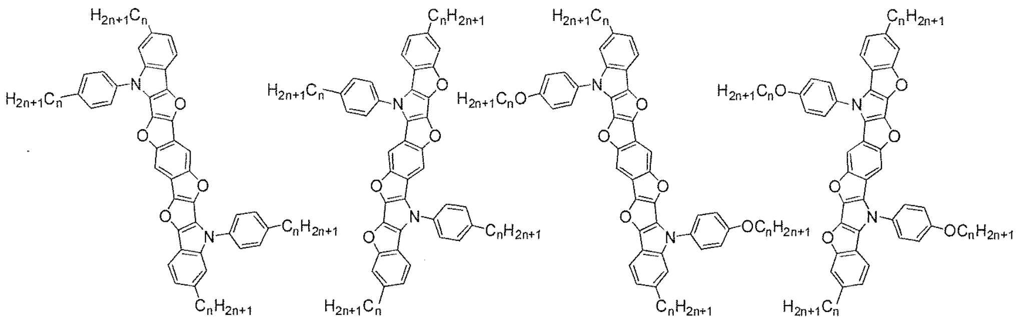

(1-197) (1-198) (1-199) (1-200)

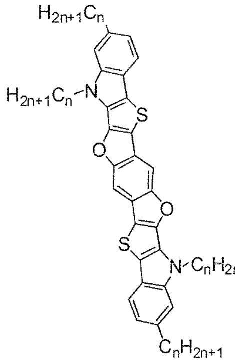

(1-201)

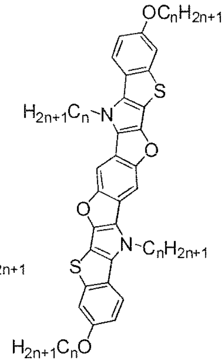

(1-202)

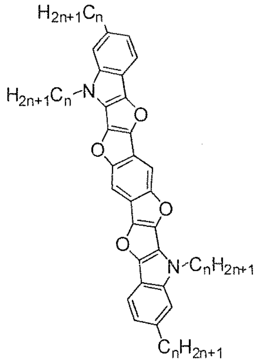

(1-203)

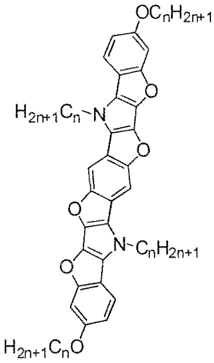

(1-204)

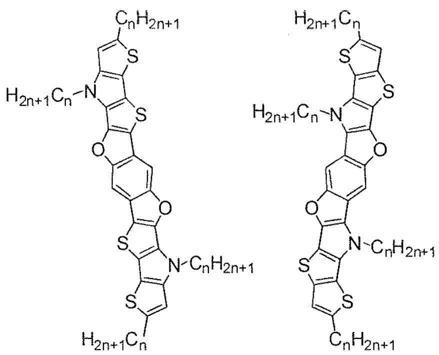

(1-205) (1-206)

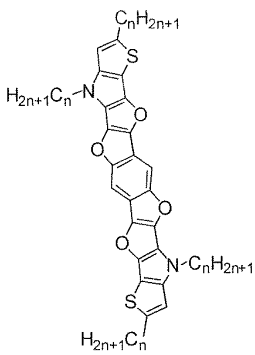

(1-207)

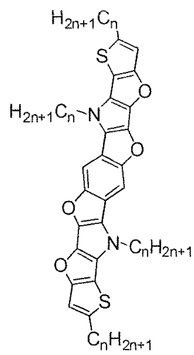

(1-208)

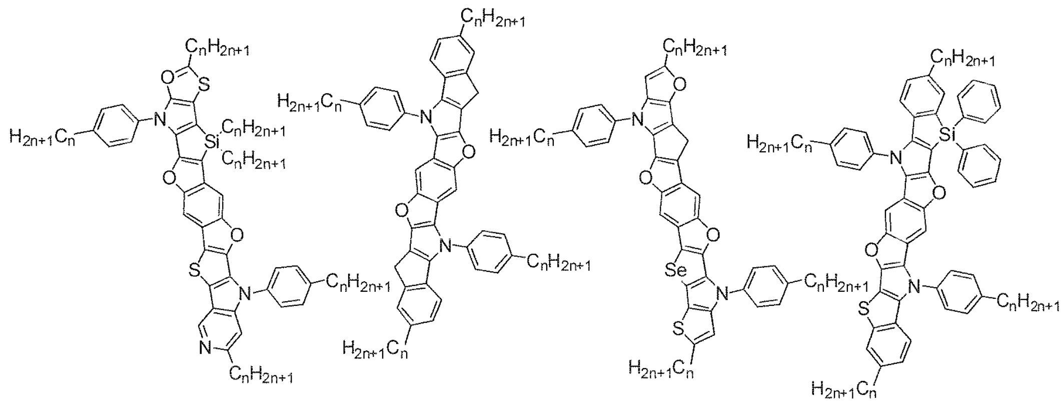

(1-209) (1-210) (1-211) (1-212)

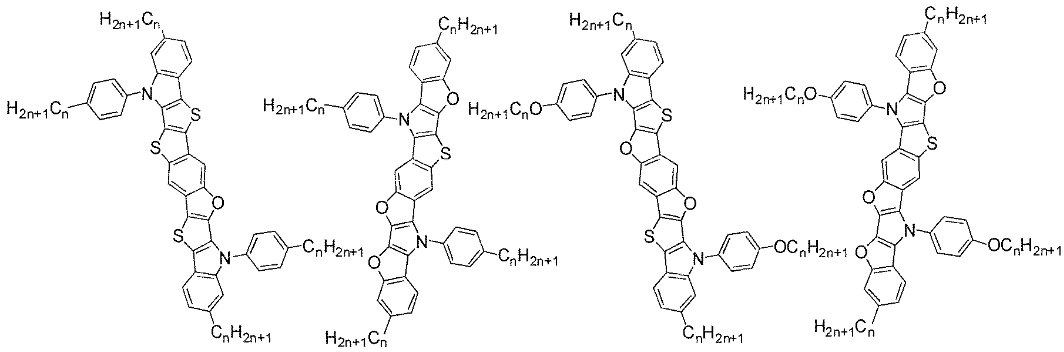

(1-213) (1-214) (1-215) (1-216)

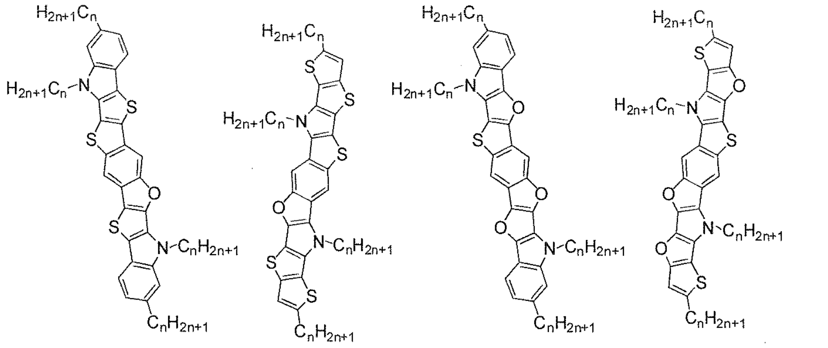

(1-217) (1-218) (1-219) (1-220)

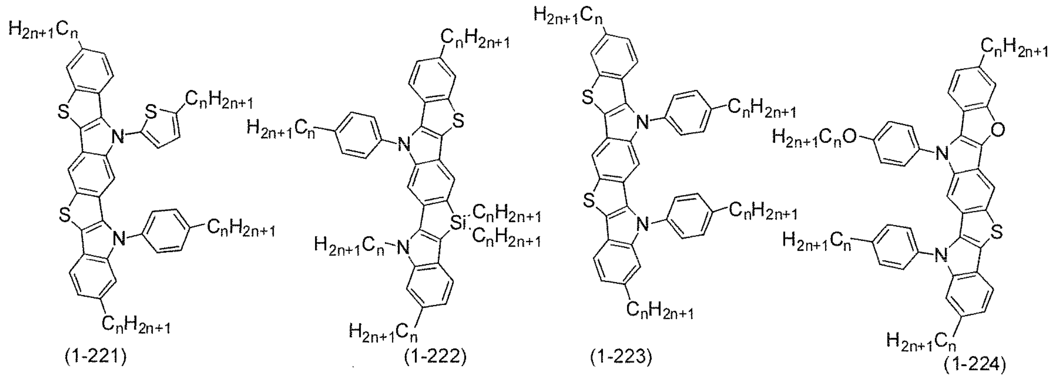

(1-221) (1-222) (1-223) (1-224)

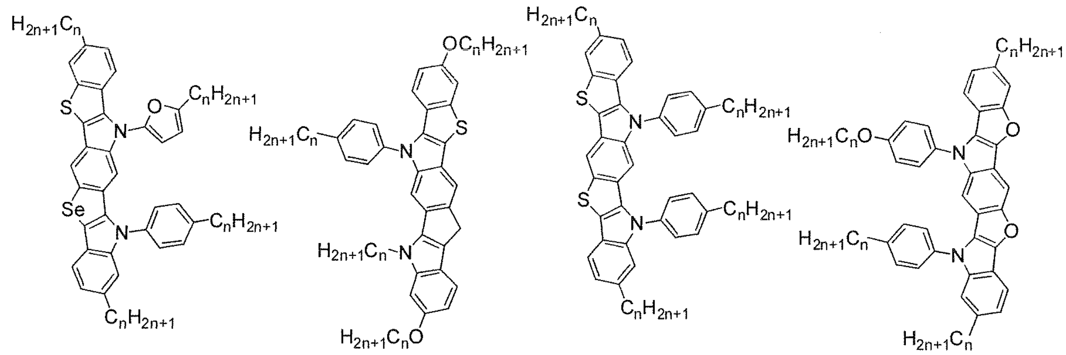

(1-225) (1-226) (1-227) (1-228)

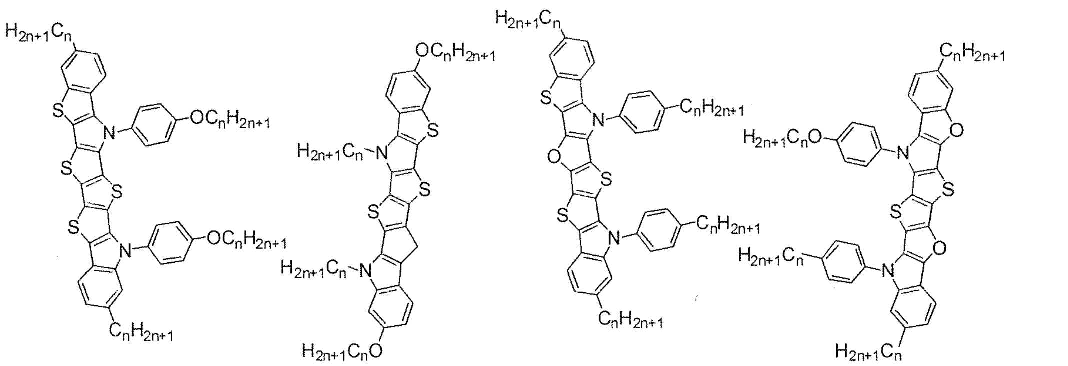

(1-229) (1-230) (1-231) (1-232)

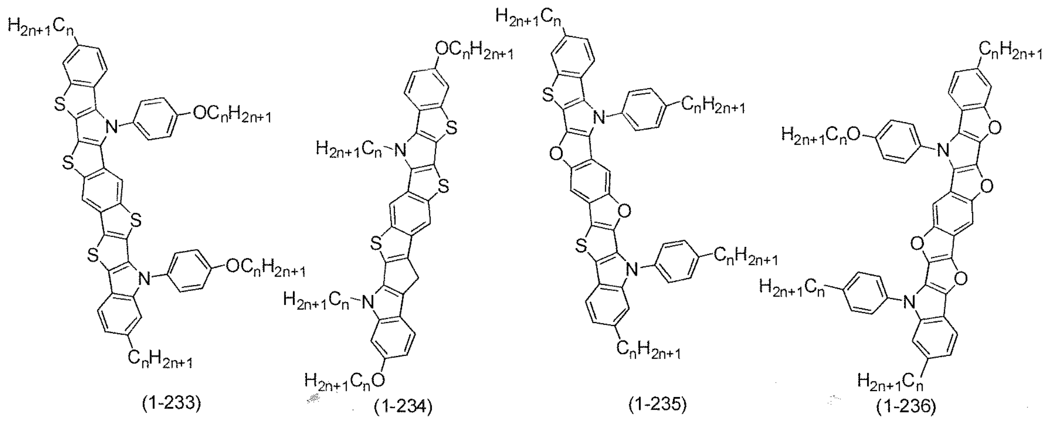

(1-233) (1-234) (1-235) (1-236)

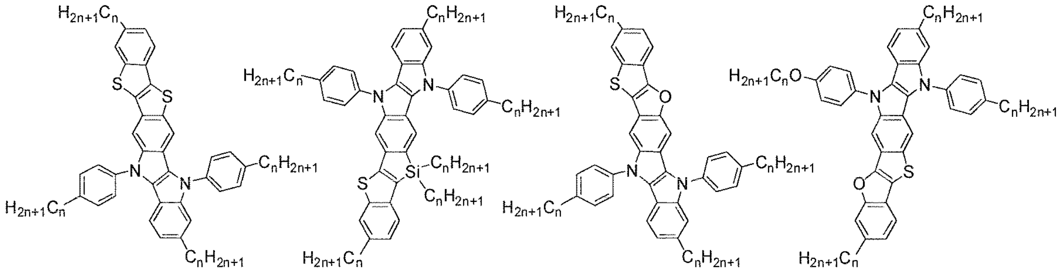

(1-237) (1-238) (1-239) (1-240)

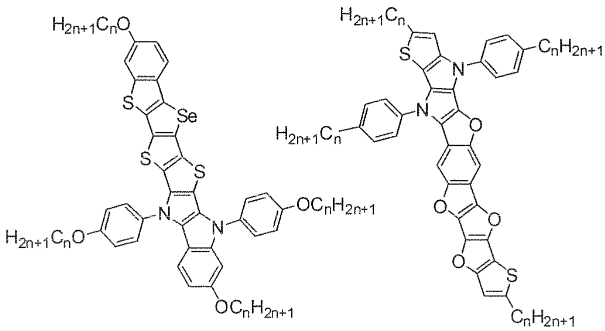

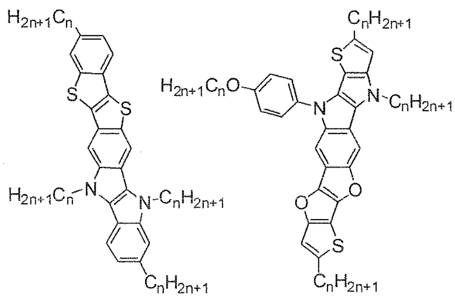

(1-241) (1-242) (1-243) (1-244)

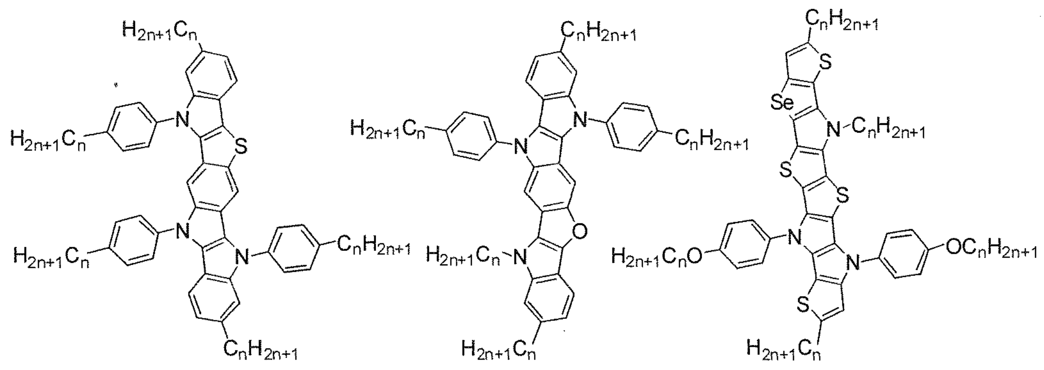

(1-245) (1-246) (1-247)

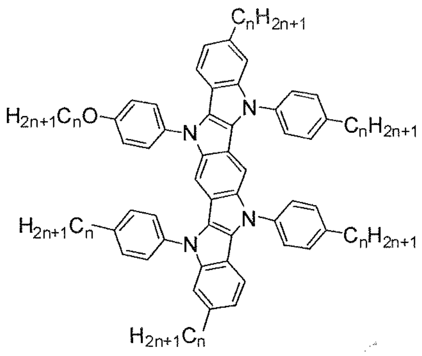

(1-248)

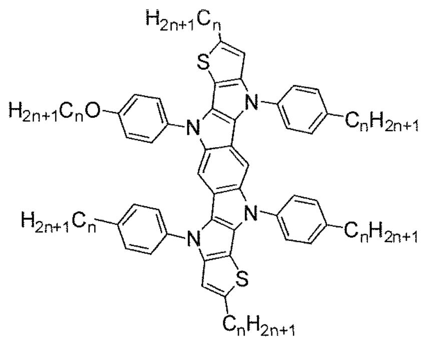

(1-249)

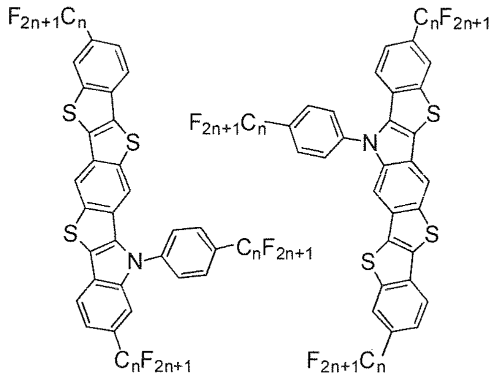

(1-250) (1-251)

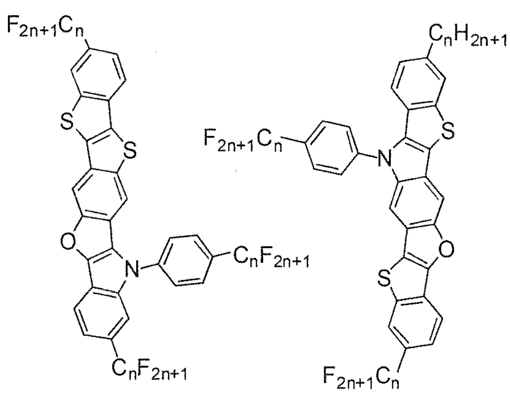

(1-252) (1-253)

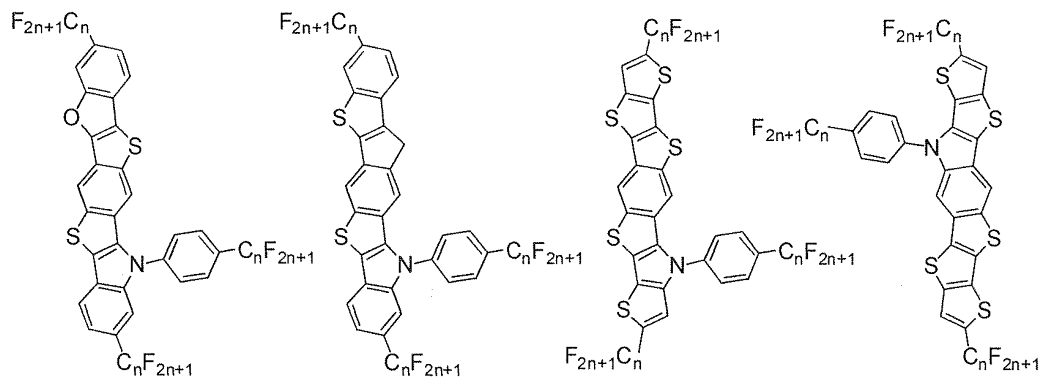

(1-254) (1-255) (1-256) (1-257)

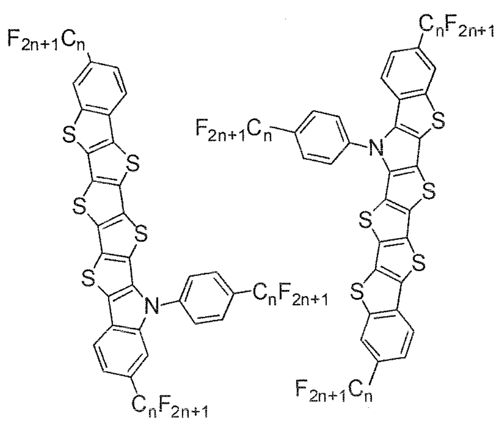

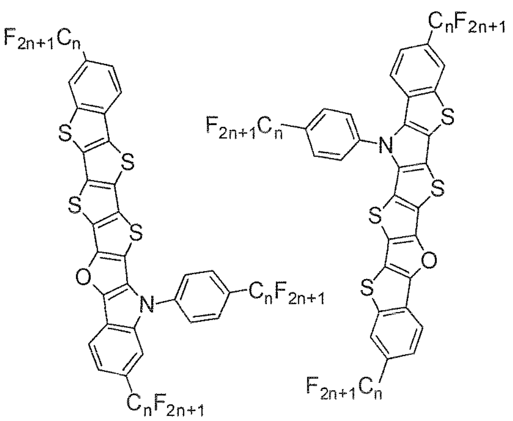

(1-258) (1-259) (1-260) (1-261)

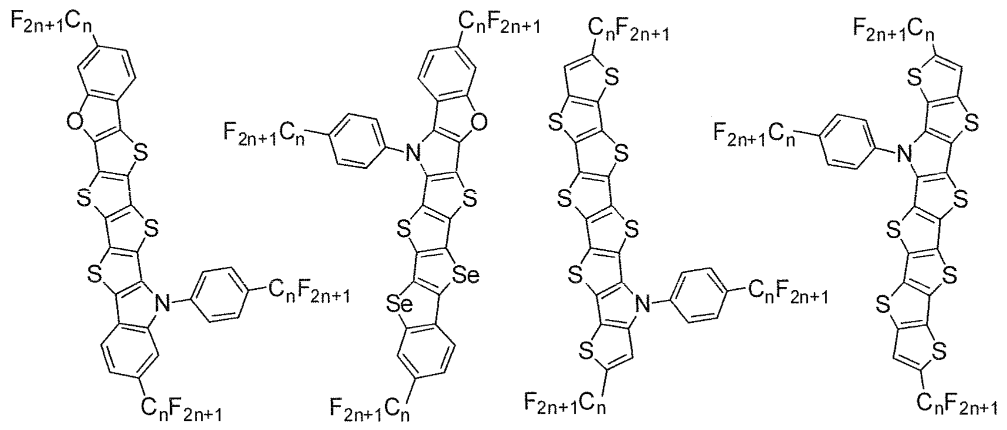

(1-262) (1-263) (1-264) (1-265)

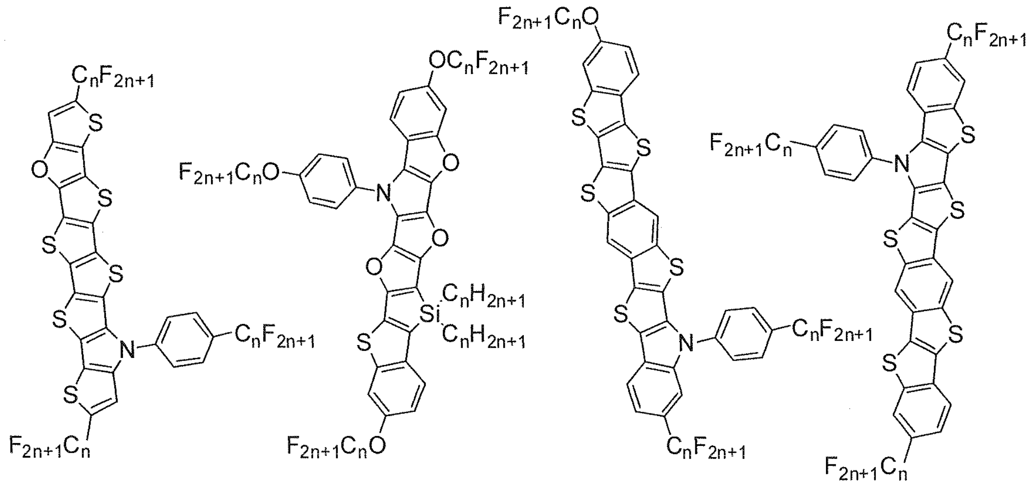

(1-266) (1-267) (1-268) (1-269)

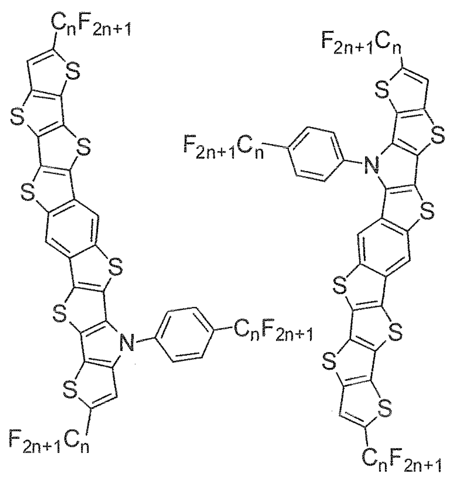

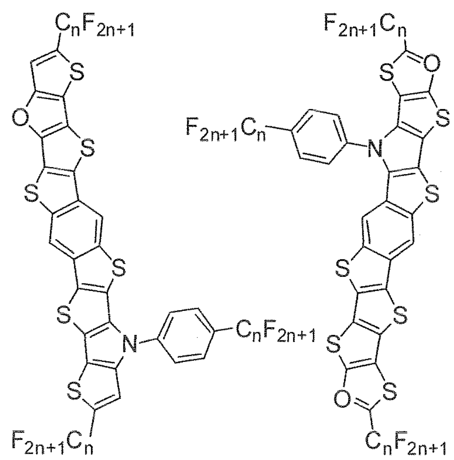

(1-270) (1-271) (1-272) (1-273)

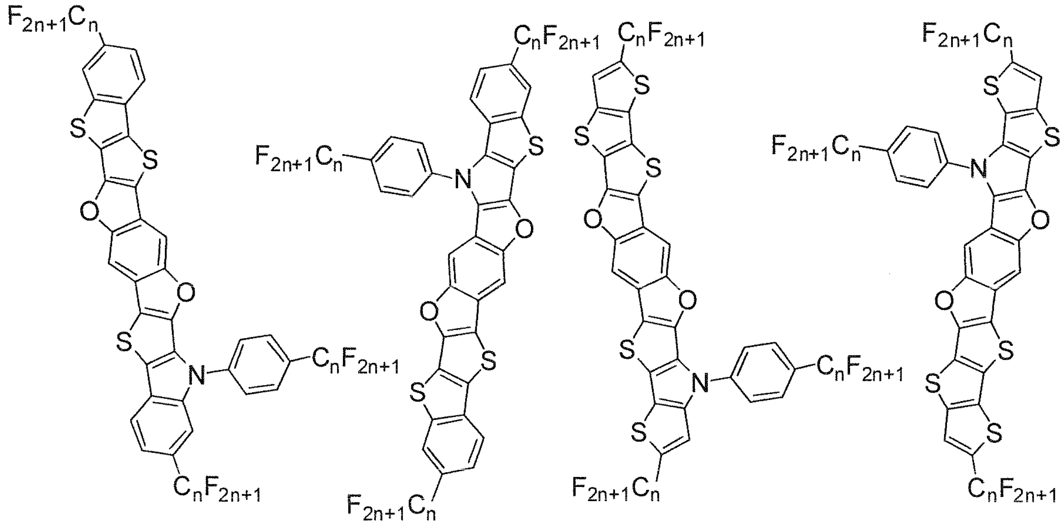

(1-274) (1-275) (1-276) (1-277)

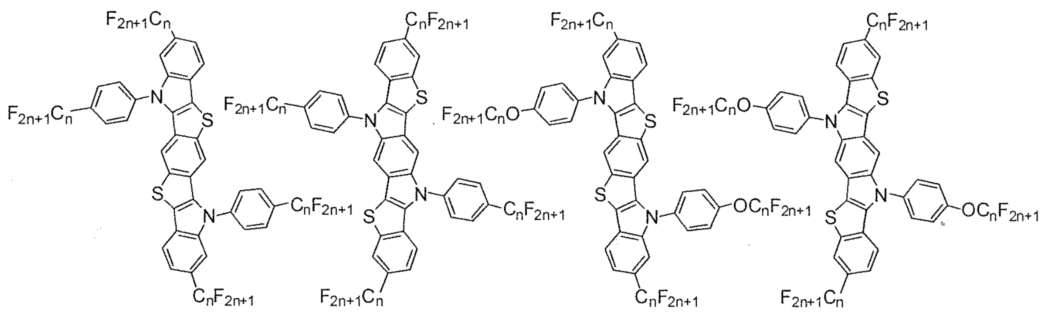

(1-278) (1-279) (1-280) (1-281)

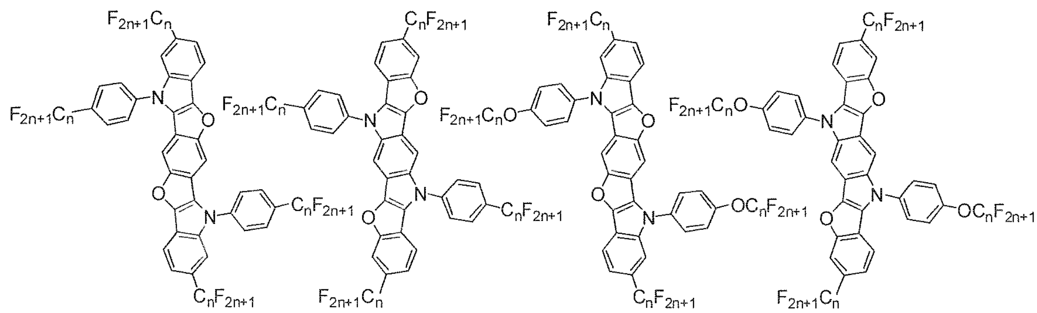

(1-282) (1-283) (1-284) (1-285)

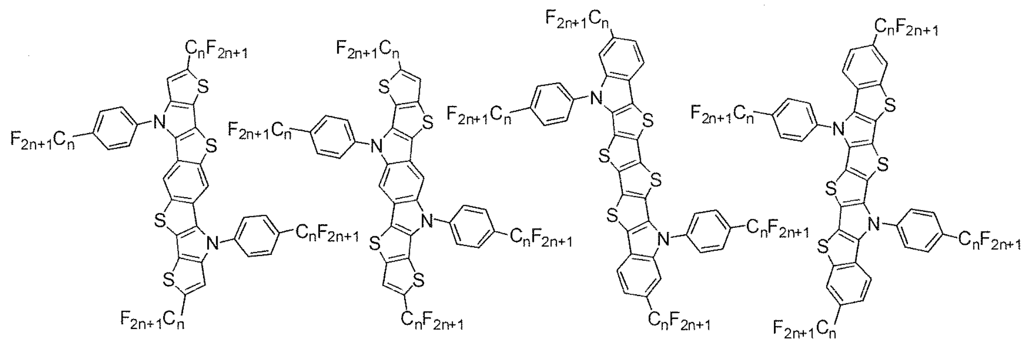

(1-286) (1-287) (1-288) (1-289)

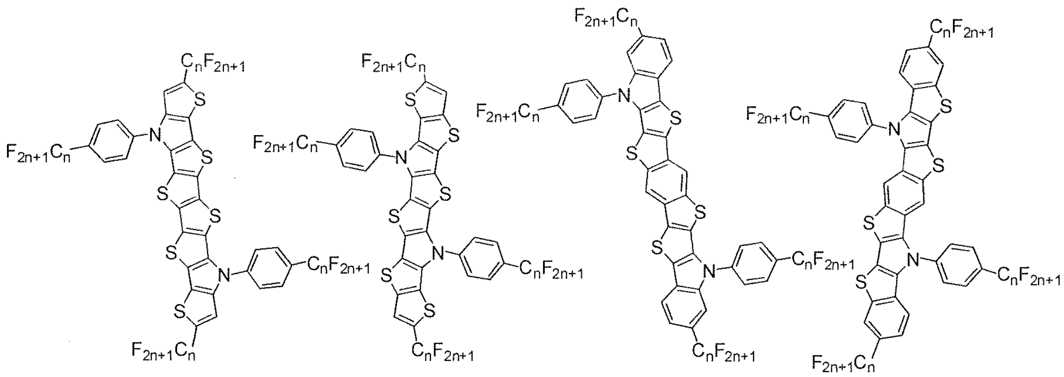

(1-290) (1-291) (1-292) (1-293)

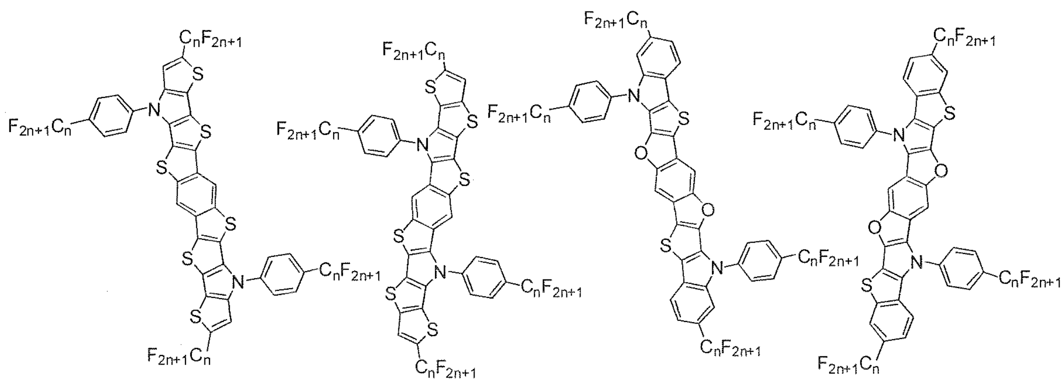

(1-294) (1-295) (1-296) (1-297)

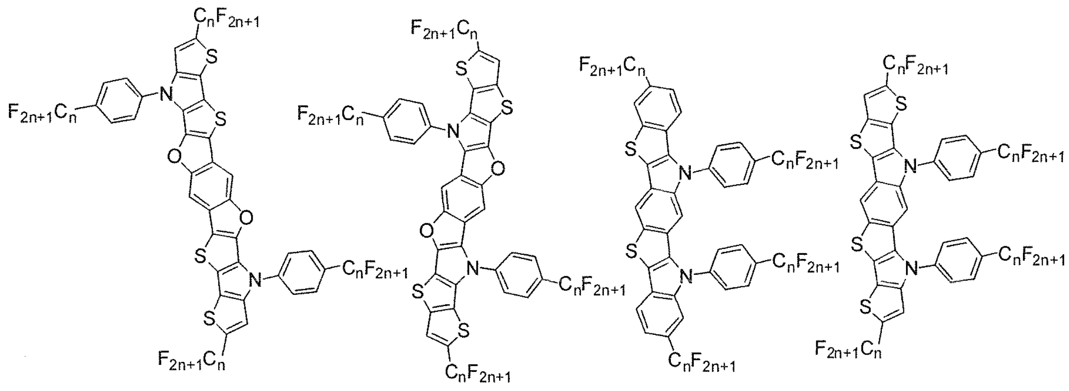

(1-298) (1-299) (1-300) (1-301)

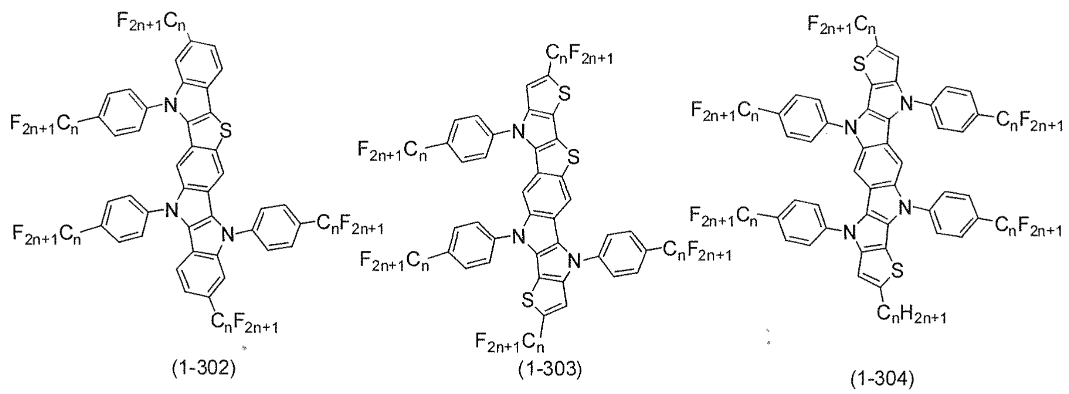

(1-302) (1-303) (1-304)

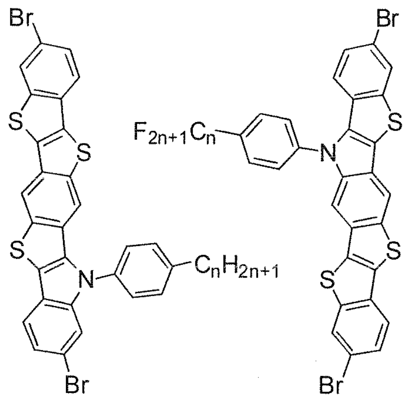

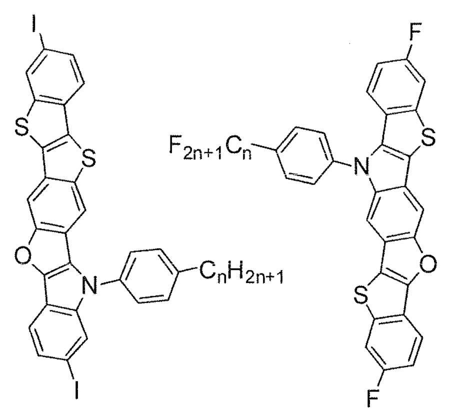

(1-305) (1-306) (1-307) (1-308)

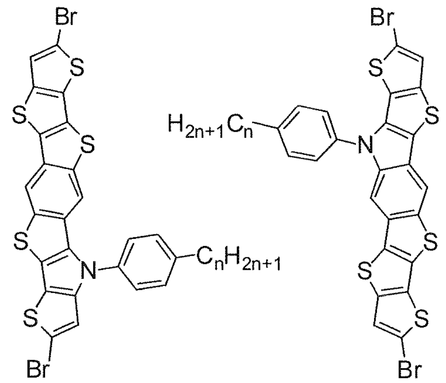 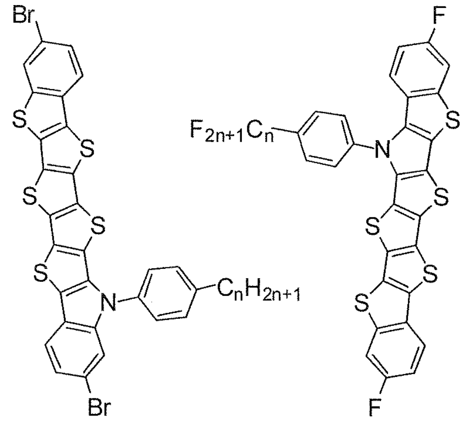

(1-309) (1-310) (1-311) (1-312)

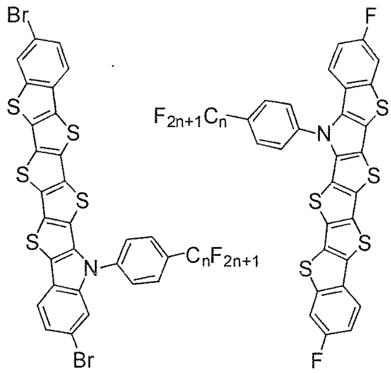 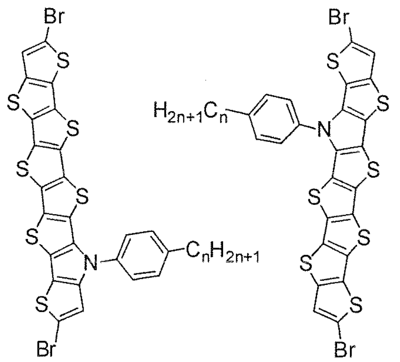

(1-313) (1-314) (1-315) (1-316)

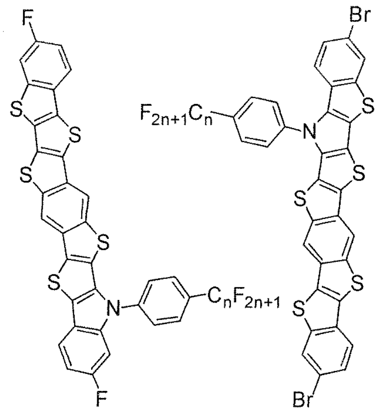 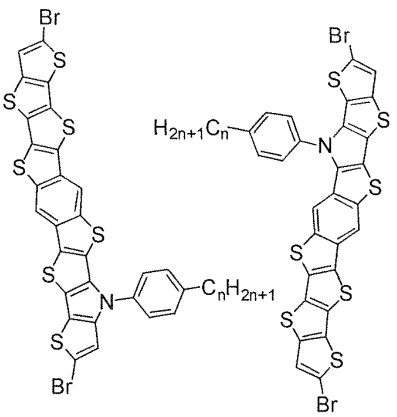

(1-317) (1-318) (1-319) (1-320)

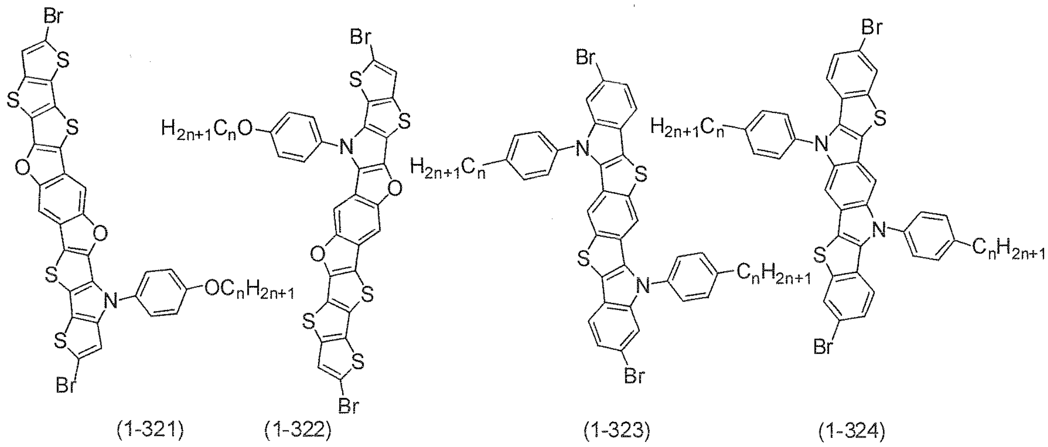

(1-321) (1-322) (1-323) (1-324)

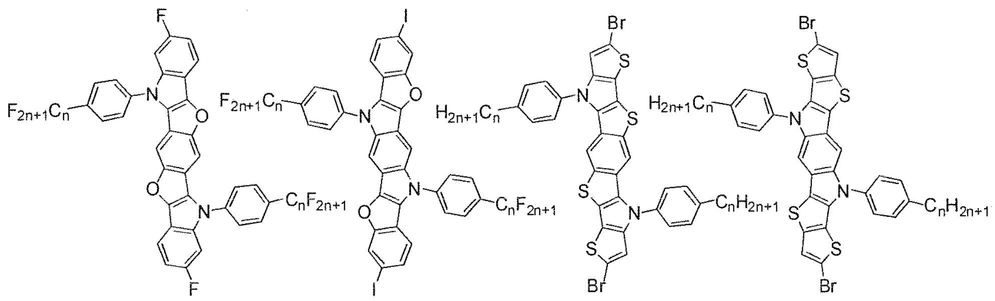

(1-325) (1-326) (1-327) (1-328)

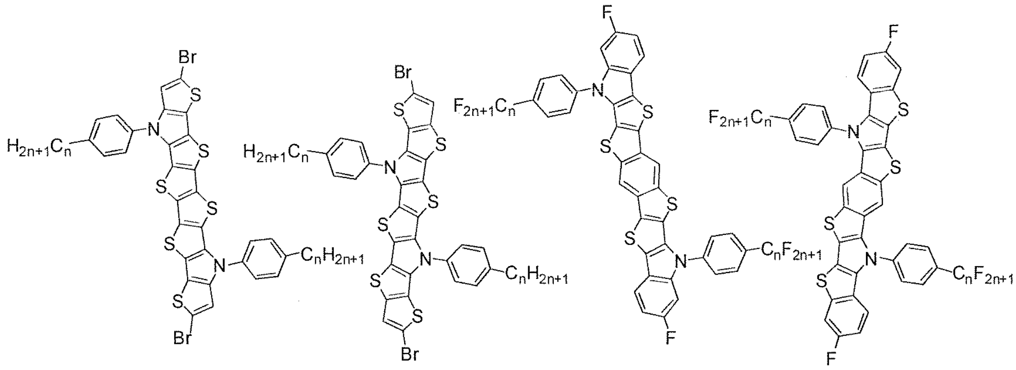

(1-329) (1-330) (1-331) (1-332)

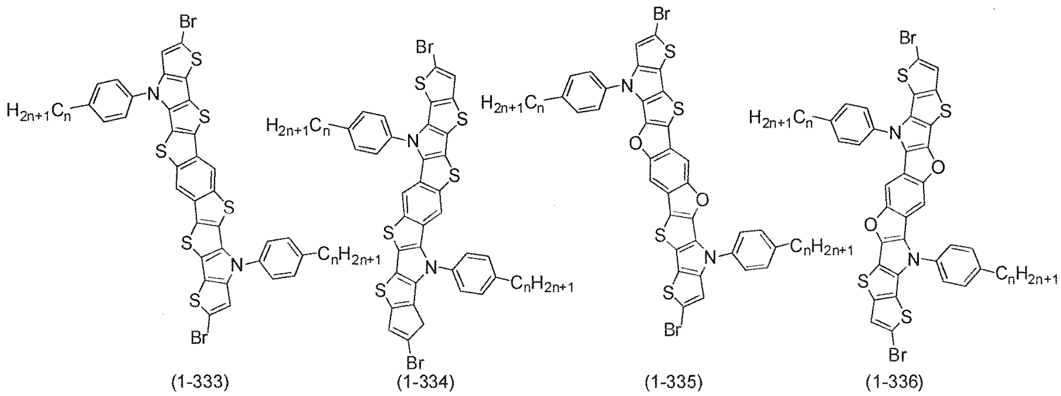

(1-333) (1-334) (1-335) (1-336)

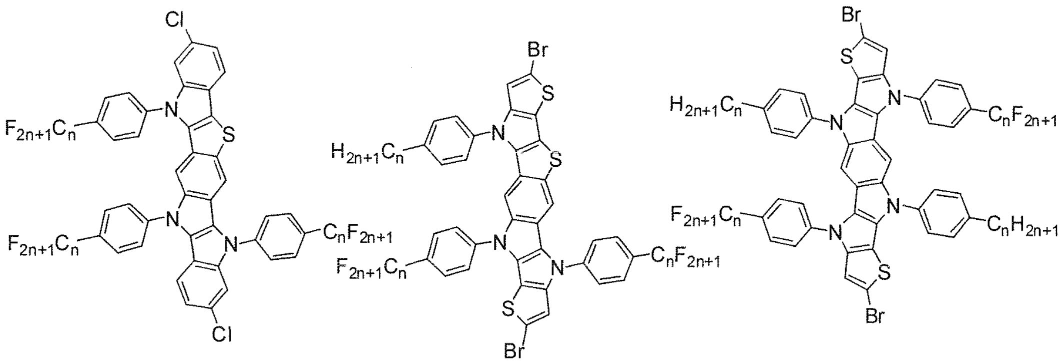

(1-337) (1-338) (1-339)

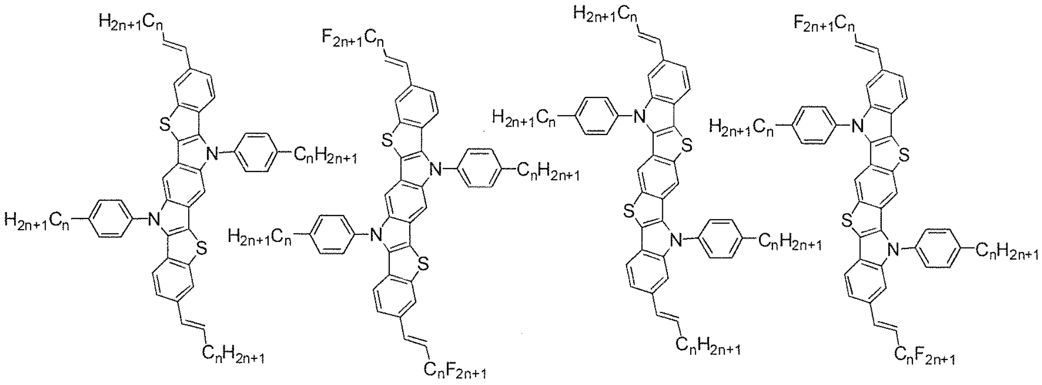

(1-340) (1-341) (1-342) (1-343)

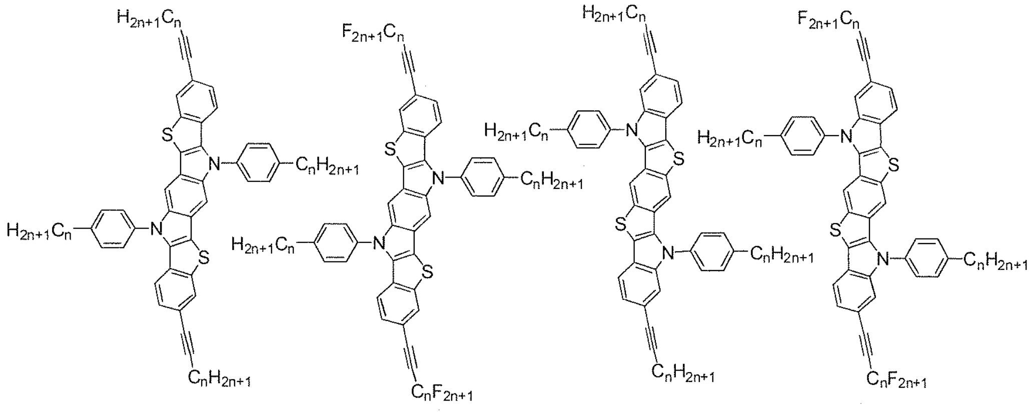

(1-344) (1-345) (1-346) (1-347)

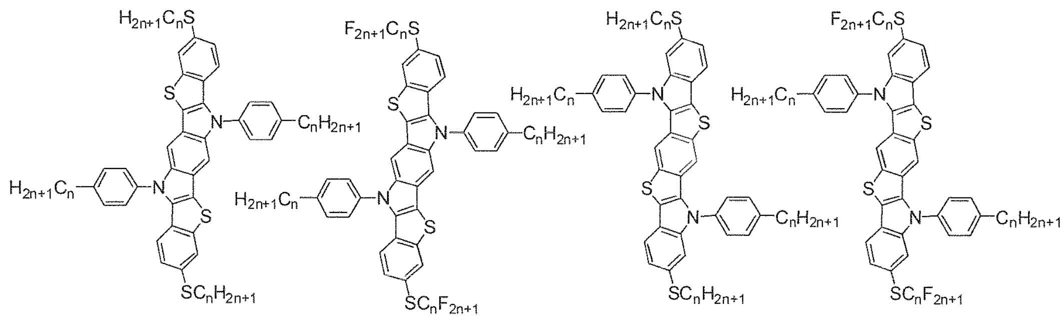

(1-348) (1-349) (1-350) (1-351)

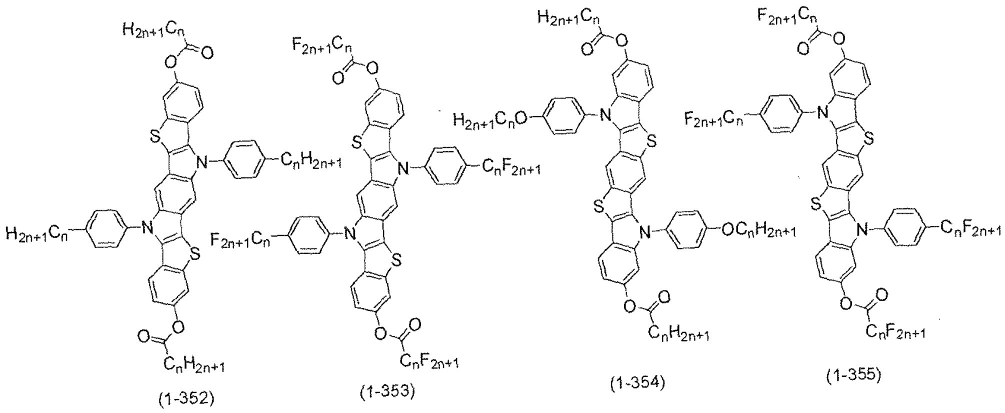

(1-352) (1-353) (1-354) (1-355)

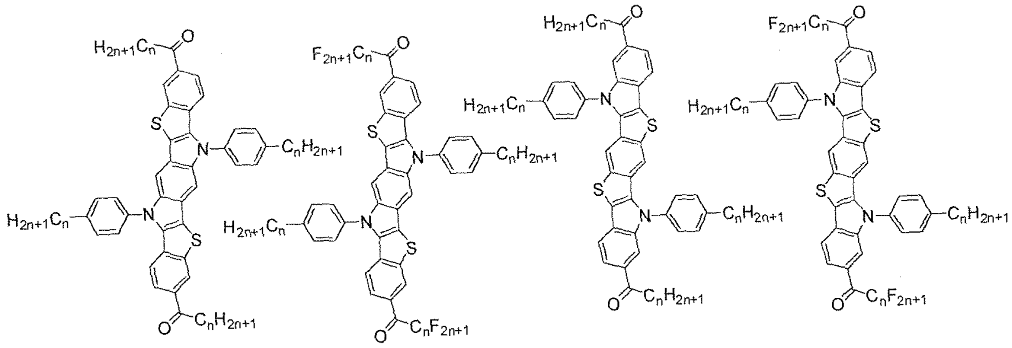

(1-356) (1-357) (1-358) (1-359)

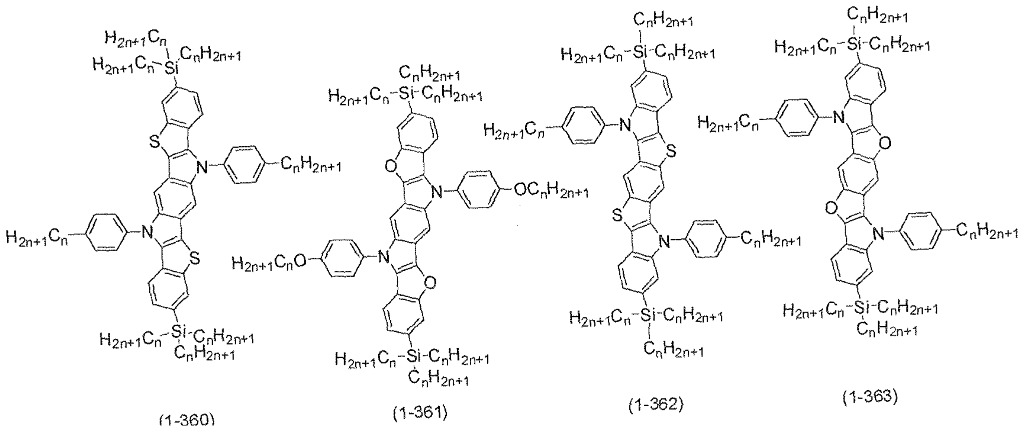

(1-360) (1-361) (1-362) (1-363)

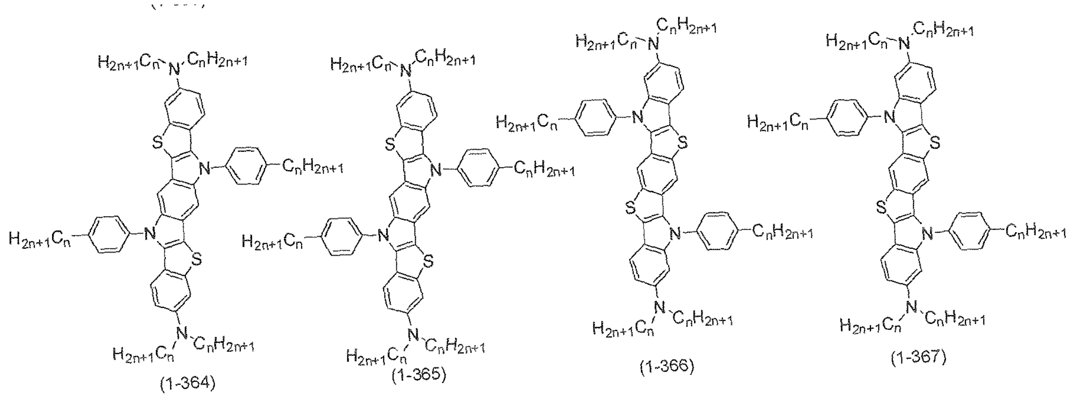

(1-364) (1-365) (1-366) (1-367)

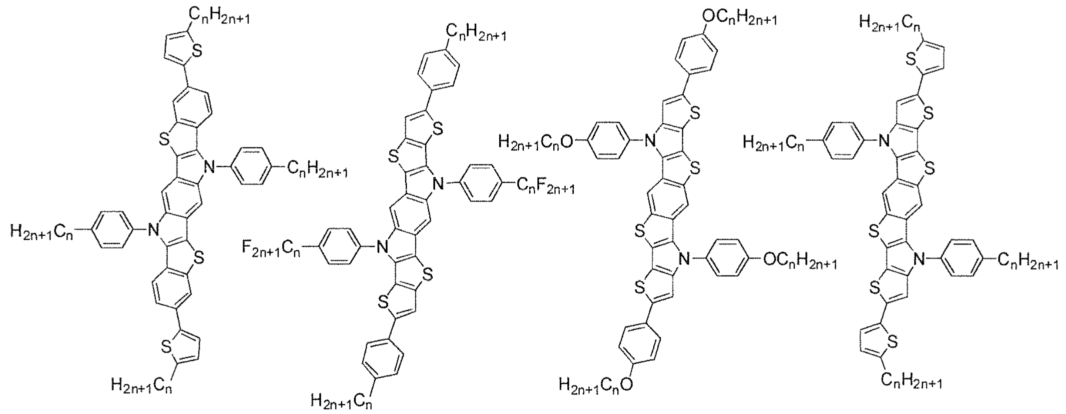

(1-368) (1-369) (1-370) (1-371)

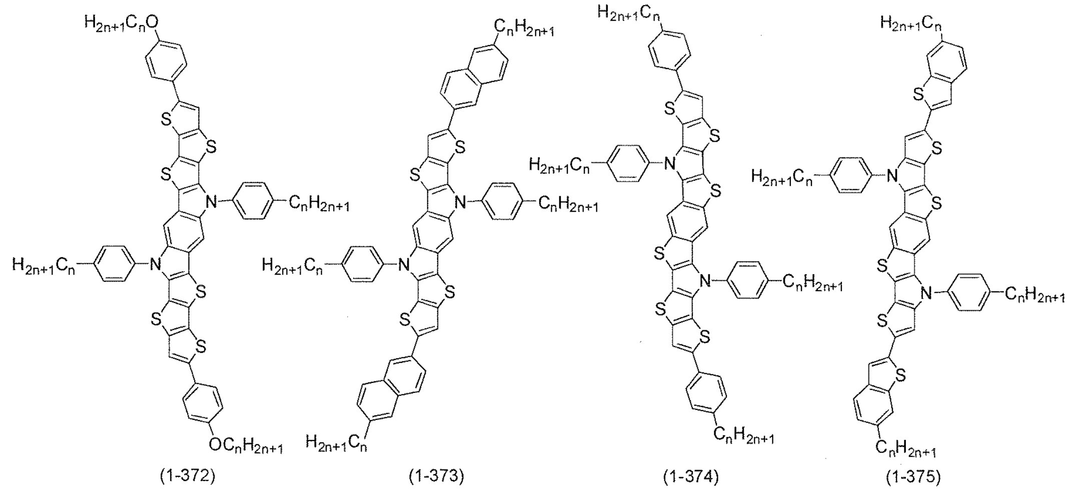

(1-372) (1-373) (1-374) (1-375)

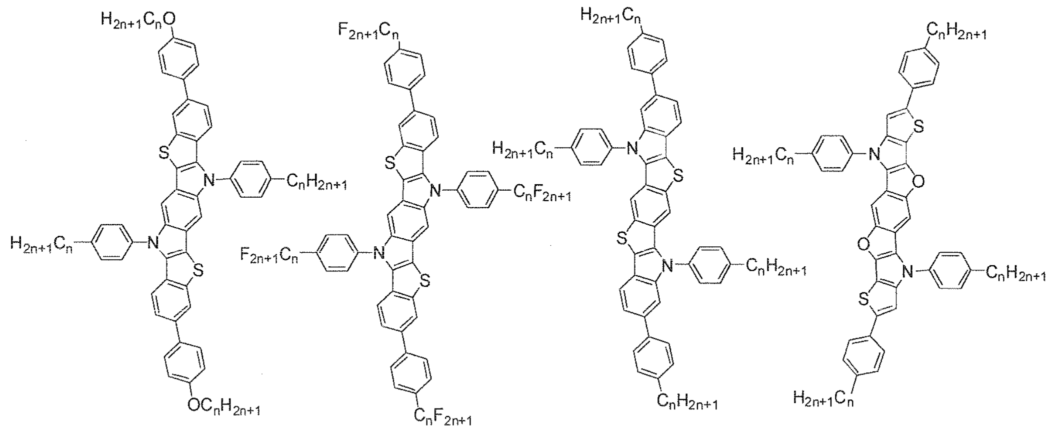

(1-376) (1-377) (1-378) (1-379)

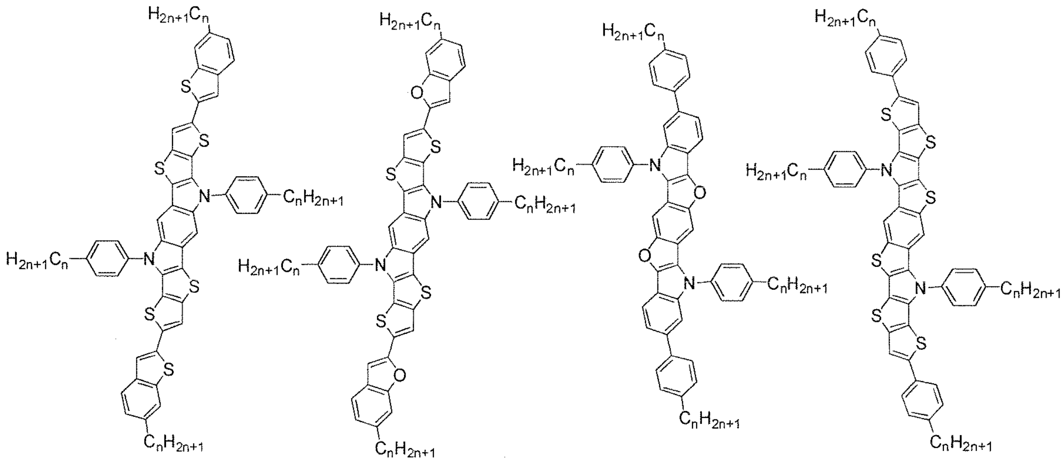

(1-380) (1-381) (1-382) (1-383)

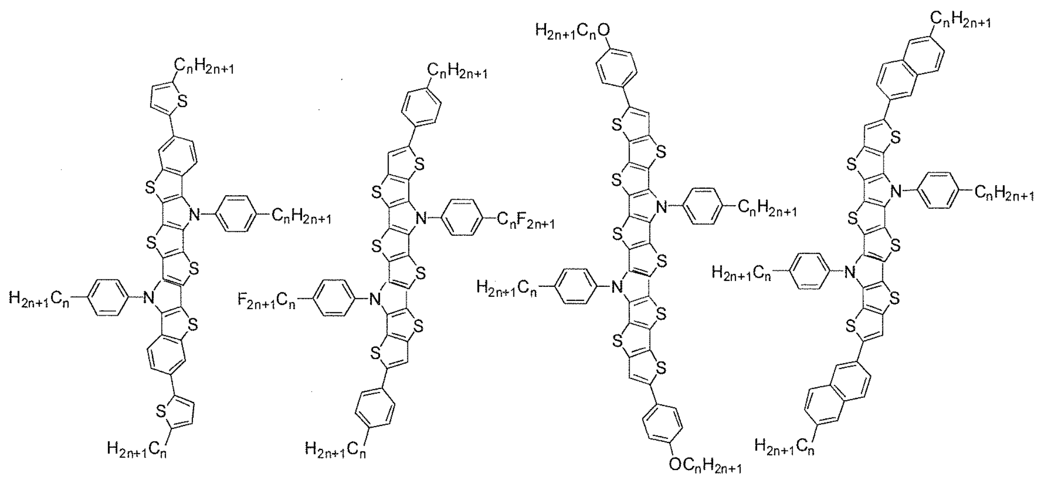

(1-384) (1-385) (1-386) (1-387)

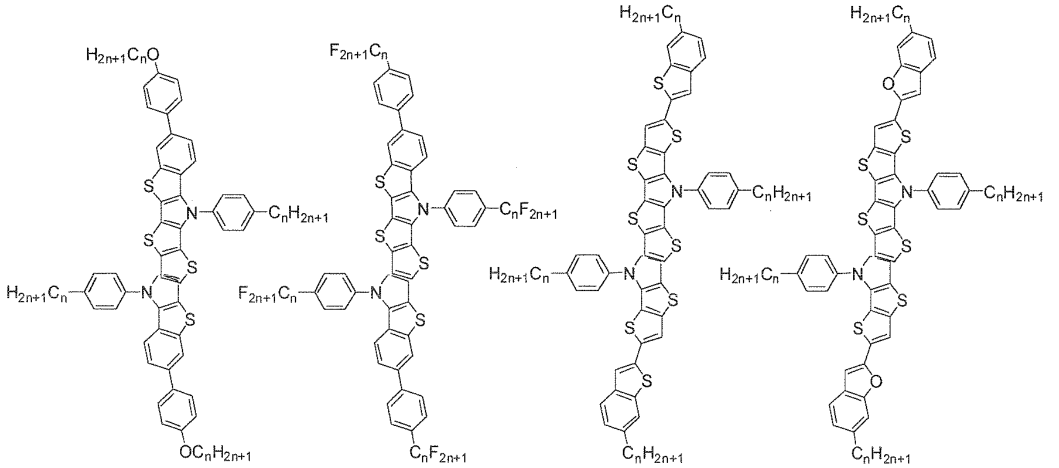

(1-388) (1-389) (1-390) (1-391)

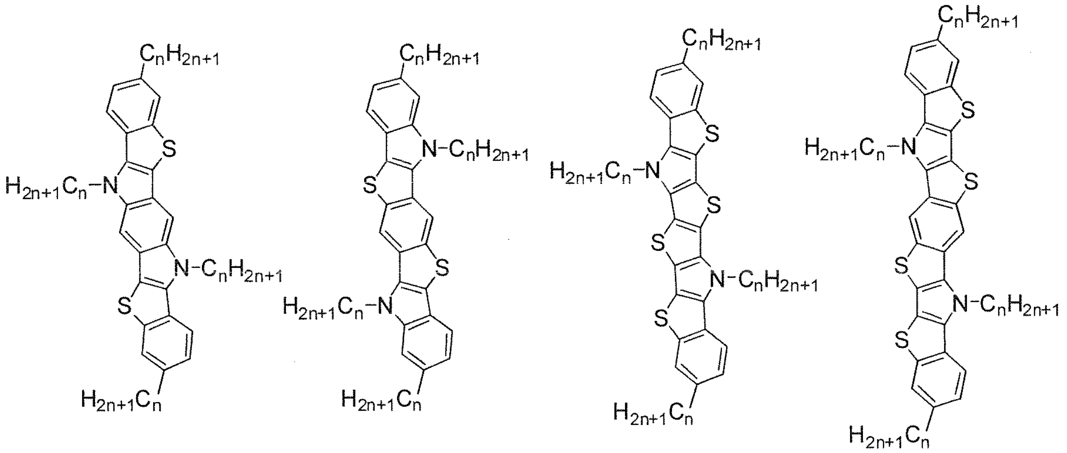

(1-392) (1-393) (1-394) (1-395)

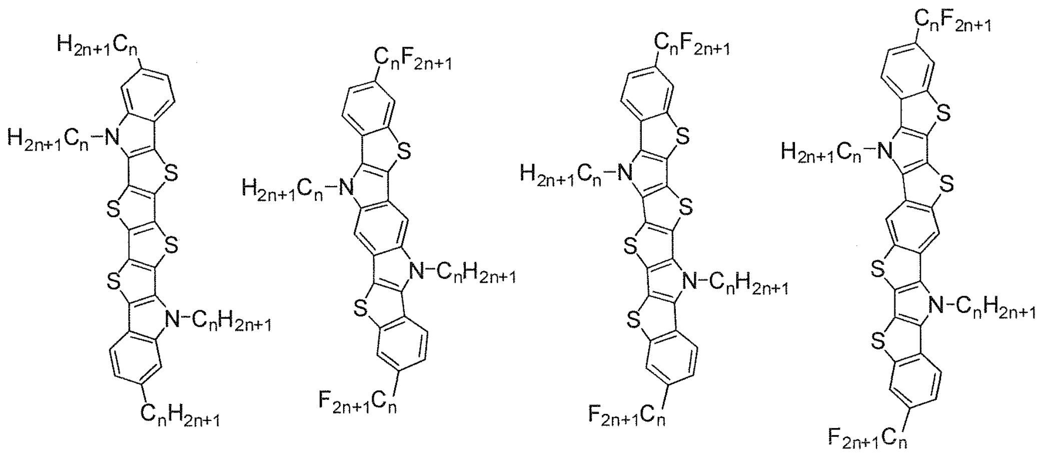

(1-396) (1-397) (1-398) (1-399)

Examples of the compound I of the present invention preferably include (1-1), (1-2), (1-3), (1-4), (1-6), (1-8), (1-13), (1-14), (1-15), (1-16), (1-21), (1-22), (1-23), (1-24), (1-29), (1-30), (1-31), (1-32), (1-34), (1-36), (1-41), (1-42), (1-43), (1-44), (1-49), (1-50), (1-51), (1-52), (1-65), (1-66), (1-67), (1-68), (1-69), (1-70), (1-72), (1-77), (1-78), (1-79), (1-80), (1-85), (1-86), (1-87), (1-88), (1-101), (1-102), (1-103), (1-104), (1-115), (1-116), (1-121), (1-122), (1-123), (1-124), (1-129), (1-130), (1-131), (1-132), (1-137), (1-138), (1-139), (1-140), (1-146), (1-153), (1-154), (1-155), (1-156), (1-157), (1-158), (1-159), (1-160), (1-161), (1-162), (1-163), (1-164), (1-173), (1-174), (1-175), (1-176), (1-177), (1-178), (1-179), (1-180), (1-181), (1-182), (1-185), (1-186), (1-193), (1-194), (1-195), (1-196), (1-201), (1-202), (1-205), (1-206), (1-215), (1-243), (1-244), (1-250), (1-251), (1-256), (1-257), (1-258), (1-259), (1-262), (1-264), (1-265), (1-268), (1-269), (1-270), (1-271), (1-274), (1-275), (1-276), (1-277), (1-278), (1-279), (1-280), (1-281), (1-282), (1-283), (1-284), (1-285), (1-286), (1-287), (1-288), (1-289), (1-290), (1-291), (1-292), (1-293), (1-294), (1-295), (1-296), (1-297), (1-298), (1-299), (1-305), (1-306), (1-307), (1-309), (1-310), (1-311), (1-313), (1-315), (1-316), (1-304), (1-321), (1-322), (1-323), (1-324), (1-327), (1-328), (1-329), (1-330), (1-331), 1-333), (1-334), (1-335), (1-336), (1-368), (1-369), (1-370), (1-371), (1-372), (1-373), (1-374), (1-375), (1-376), (1-377), (1-378), (1-379), (1-380), (1-381), (1-382), (1-383), (1-384), (1-385), (1-386), (1-387), (1-388), (1-389), (1-390), (1-391), (1-392), (1-394), (1-395), (1-397), and (1-398), and more preferably (1-1), (1-2), (1-3), (1-4), (1-21), (1-22), (1-23), (1-24), (1-29), (1-30), (1-31), (1-32), (1-49), (1-50), (1-51), (1-52), (1-65), (1-66), (1-67), (1-68), (1-85), (1-86), (1-87), (1-88), (1-101), (1-102), (1-103), (1-104), (1-121), (1-122), (1-123), (1-124), (1-129), (1-130), (1-131), (1-132), (1-137), (1-138), (1-139), (1-140), (1-153), (1-154), (1-155), (1-156), (1-161), (1-162), (1-164), (1-17),

(1-174), (1-175), (1-176), (1-178), (1-180), (1-181), (1-182), (1-193), (1-194), (1-195), (1-196), (1-201), (1-202), (1-205), (1-206), (1-250), (1-251), (1-256), (1-257), (1-258), (1-259), (1-264), (1-265), (1-268), (1-269), (1-270), (1-271), (1-274), (1-275), (1-278), (1-279), (1-280), (1-281), (1-282), (1-283), (1-284), (1-285), (1-286), (1-287), (1-288), (1-289), (1-292), (1-293), (1-296), (1-297), (1-305), (1-306), (1-307), (1-309), (1-310), (1-311), (1-313), (1-315), (1-316), (1-321), (1-322), (1-323), (1-324), (1-327), (1-328), (1-329), (1-330), (1-333), (1-334), (1-335), (1-336), (1-392), (1-394), and (1-395) .

The compound I of the present invention is obtained, for example, by a reaction of a tetrahalogen compound represented by the formula (2) or (3) (hereinafter, this compound is referred to as a "tetrahalogen compound A") with $R^5$-$NH_2$ ($R^5$ is as defined above). The method for producing the compound I including a step of carrying out the reaction is also one aspect of the present invention:

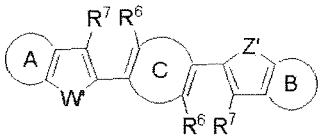 (2)

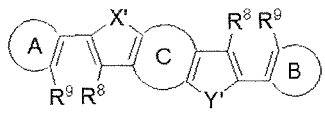 (3)

wherein a ring structure A, a ring structure B and a ring structure C are each as defined above, W' ,X' ,Y' and Z' independently represent a sulfur atom, an oxygen atom, a selenium atom, a tellurium atom, $SO_2$, or a group represented by ($R^1$) -C- ($R^2$) , ($R^3$) -Si- ($R^4$) , or N- ($R^5$) , $R^1$, $R^2$, $R^3$, $R^4$ and $R^5$ is as defined above, and $R^6$, $R^7$, $R^8$ and $R^9$ independently represent a halogen atom.

The reaction in the production method of the present invention is usually carried out in an organic solvent.

The organic solvent may be an organic solvent which is inert to the reaction and examples thereof include aromatic hydrocarbon solvents such as toluene and xylene; halogenated aromatic hydrocarbon solvents such as chlorobenzene and o-dichlorobenzene; aliphatic hydrocarbon solvents such as hexane, heptane, and dimethoxyethane; halogenated aliphatic hydrocarbon solvents such as chloroform and 1,2-dichloroethane; alcohols having 1 to 4 carbon atoms such as methanol, isopropanol, and t-butanol; and ether solvents such as tetrahydrofuran and dioxane; alone or a mixed solvent thereof, preferably aromatic hydrocarbon solvents and aliphatic hydrocarbon solvents, and more preferably toluene and xylene.

In the production method of the present invention, $R^5$ of $R^5$-$NH_2$ is preferably an alkyl group having 1 to 30 carbon atoms, a fluoroalkyl group having 1 to 30 carbon atoms, a phenyl group, a phenyl group substituted with an alkyl group having 1 to 30 carbon atoms, a phenyl group substituted with a fluoroalkyl group having 1 to 30 carbon atoms, or a phenyl group substituted with an alkoxy group having 1 to 30 carbon atoms, more preferably an alkyl group having 1 to 20 carbon atoms, a fluoroalkyl group having 1 to 20 carbon atoms, a phenyl group substituted with an alkyl group having 1 to 20 carbon atoms, a phenyl group substituted with a fluoroalkyl group having 1 to 20 carbon atoms, or a phenyl group substituted with an alkoxy group having 1 to 20 carbon atoms, and still more preferably an alkyl group having 1 to 16 carbon atoms, a phenyl group substituted with an alkyl group having 1 to 16 carbon atoms, or a phenyl group substituted with an alkoxy group having 1 to 16 carbon atoms.

The use amount of $R^5$-$NH_2$ is usually from 0.5 to 50 mol times, preferably from 1 to 20 mol times, and more preferably from 1 to 15 mol times, relative to the tetrahalogen compound A.

In the tetrahalogen compound A, it is preferred that $R^6$, $R^7$, $R^8$ and $R^9$ independently represent bromine or iodine. It is preferred that X', Y', W' and Z' independently represent a sulfur atom or an oxygen atom.

The concentration of the tetrahalogen compound A in a reaction solution is not particularly limited, and is usually within a range from 0.0001 mol to 20 mol, preferably from 0.001 mol to 10 mol, and more preferably from 0.01 mol to 5 mol, per liter of the solvent.

The reaction is commonly carried out in the presence of a palladium catalyst and a basic reagent.

In the reaction, the palladium catalyst can be usually used in the proportion within a range from 0.01 to 30 mol%, and preferably from 0.01 to 20 mol%, in terms of palladium relative to the tetrahalogen compound A.

The palladium catalyst may be prepared by contacting a ligand and a palladium compound in advance in an organic solvent, or may be prepared by contacting a ligand and a palladium compound in a reaction system.

The ligand may be a ligand which is coordinatable with palladium and is soluble in an organic solvent and

includes, for example, a monodentate phosphine ligand, a multidentate ligand, a carbene ligand, and the like, and is preferably a monodentate ligand, and more preferably a monodentate phosphine ligand.

Examples of the monodentate phosphine ligand include tri(n-butyl)phosphine, tri(t-butyl)phosphine, tricyclohexylphosphine, triphenylphosphine, tri(o-tolyl)phosphine, trinaphthylphosphine, diphenylnaphthylphosphine, dicyclohexylnaphthylphosphine, and the like, and preferably tri(t-butyl)phosphine.

Examples of the bidentate ligand include bidentate phosphine ligands having two phosphorus atoms, such as 2,2'-bis(diphenylphosphino)-1,1'-binaphthyl, 1,2-bis(diphenylphosphino)ethane, 1,3-bis(diphenylphosphino)propane, 1,4-bis(diphenylphosphino)butane, 1,1'-(diphenylphosphino)ferrocene, 4,5-bis(diphenylphosphino)-9,9-dimethylxanthene, 2,2'-bis(diphenylphosphino)diphenylether, and 5,5'-bis(diphenylphosphino)-4,4'-bi(1,3-benzodioxole); bidentate aminophosphine ligands having one nitrogen atom and one phosphorus atom, such as 2-(N,N-dimethylamino)-2'-(dicyclohexylamino)biphenyl; and the like.

It is possible to use, as the ligand, commercially available ligands, or ligands produced by a known method.

The use amount of the ligand is usually from 0.5 to 20 mol times relative to palladium of a palladium compound.

Examples of the palladium compound include divalent palladium compounds such as palladium acetate, palladium chloride, dichlorobis(acetonitrile)palladium, palladiumacetylacetonate, dichloro(cycloocta-1,5-diene)palladium, dibromobis(benzonitrile)palladium, di-μ-chlorobis(n-allyl)dipalladium, dichlorobis(pyridine)palladium, dichlorobis(triphenylphosphine)palladium, and a dichloro-[1,1'-bis(diphenylphosphino)ferrocene]palladium dichloromethane complex; 0-valet palladium compounds such as tris(dibenzylidineacetone)dipalladium, a tris(dibenzylidineacetone)dipalladium chloroform complex, and terakis(triphenylphosphine)palladium; and the like. Among these palladium compounds, tris(dibenzylidineacetone)dipalladium and a tris(dibenzylidineacetone)dipalladium chloroform complex are preferred. It is possible to use, as the palladium compound, commercially available palladium compounds, or palladium compounds produced by a known method.

Examples of the basic reagent include alkali earth metal hydroxides such as calcium hydroxide; alkali metalcarbonic acid salts such as potassium carbonate, sodium carbonate, and cesium carbonate; alkali earth metal carbonic acid salts such as magnesium carbonate, calcium carbonate, and barium carbonate; alkali metal phosphoric acid salts such as lithium phosphate, potassium phosphate, and sodium phosphate; and alkali metal alkoxides such as sodium methoxide, sodium ethoxide, sodium t-butoxide, potassium methoxide, potassiumethoxide, potassium t-butoxide, and lithium t-butoxide; preferably alkali metalcarbonic acid salts and alkali metal alkoxides, more preferably alkali metal alkoxides, and still more preferably alkali metal alkoxides having 1 to 6 carbon atoms. These basic reagents may be used alone, or two or more kinds thereof may be mixed.

The use amount of the basic reagent is usually from 0.1 mol times to 25 mol times, preferably from 1 mol time to 20 mol times, and more preferably from 2 mol times to 10 mol times, relative to the tetrahalogen compound A. When the use amount of the basic reagent is too small, the proportion of the unreacted primary amine may increase.

The reaction temperature is usually within a range from 0°C to a reflux temperature of a reaction solution, preferably 250°C, and more preferably from 40°C to 200°C. The reaction time is not particularly limited, and usually from 1 minute to 120 hours.

In the case where this reaction is stopped, water, dilute hydrochloric acid or the like is added to the reaction solution. A crude product of the compound I can be usually obtained by a post-treatment, for example, an operation such as extraction or washing after stopping of the reaction. The crude product may be purified by operations such as crystallization, sublimation and various chromatographies.

The compound represented by the formula (3) (hereinafter, this compound is referred to as a "tetrahalogen compound (3)") is also one aspect of the present invention.

Each of a tetrahalogen compound of the above formula (2) in which a ring structure C is a thieno[3,2-b]thiophene ring, W' and Z' independently represent a sulfur atom or an oxygen atom, and $R^6$, $R^7$, $R^8$ and $R^9$ independently represent a halogen atom (hereinafter, this compound is referred to as a "tetrahalogen compound (2)"), and tetrahalogen compound of the above formula (3) in which a ring structure C is a thieno[3,2-b]thiophene ring, a furo[3,2-b]furan ring, a benzo[1,2-b:4,5-b']dithiophene ring which may be substituted, or a benzo[1,2-b:4,5-b']difuran ring which may be substituted, and X' and Y' independently represent a sulfur atom or an oxygen atom (hereinafter, this compound is referred to as a "tetrahalogen compound (3)") is one aspect of the present invention.

Examples of the ring structure A and the ring structure B in the tetrahalogen compound (2) and the tetrahalogen compound (3) each include the same ring structures as those of the compound I of the present invention, and preferably benzene rings, thiophene rings, or furan rings.

Examples of the ring structure C in the tetrahalogen compound (3) preferably include a thieno[3,2-b]thiophene ring, a furo[3,2-b]furan ring, a benzo[1,2-b:4,5-b']dithiophene ring which may be substituted, and a benzo[1,2-b:4,5-b'] difuran ring which may be substituted, more preferably a thieno[3,2-b]thiophene ring and a benzo[1,2-b:4,5-b']dithiophene ring which may be substituted, and still more preferably a thieno[3,2-b]thiophene ring.

Examples of W' and Z' in the tetrahalogen compound (2) as well as X' and Y' in the tetrahalogen compound (3) include the same ones as those of the compound I of the present invention.

$R^6$, $R^7$, $R^8$ and $R^9$ in the tetrahalogen compound (2) and the tetrahalogen compound (3) each represent a halogen atom, and preferably include bromine and iodine atoms.

Examples of the tetrahalogen compound (2) of the present invention include, but are not limited to, compounds shown below. Provided that n each represents an integer of 0 to 30.

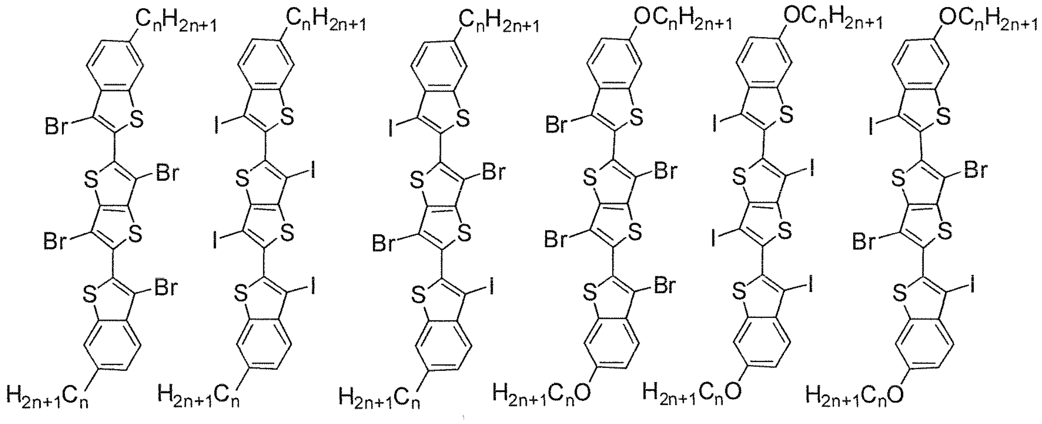

(2-1) (2-2) (2-3) (2-4) (2-5) (2-6)

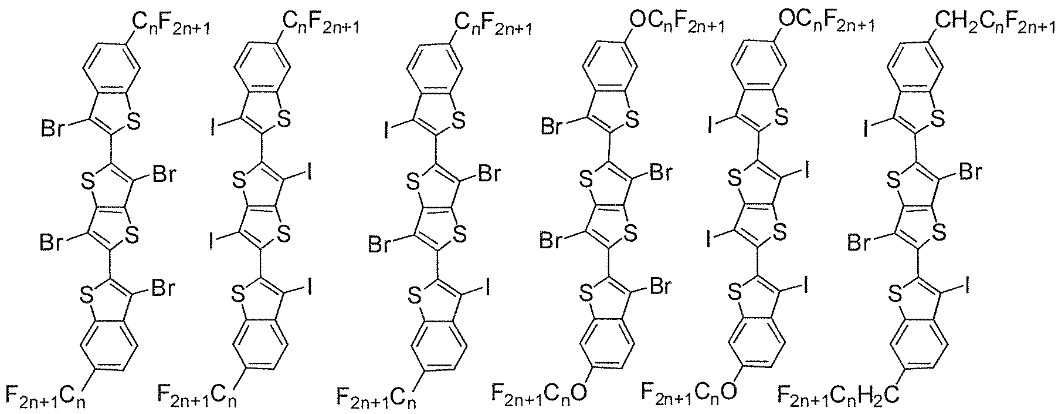

(2-7) (2-8) (2-9) (2-10) (2-11) (2-12)

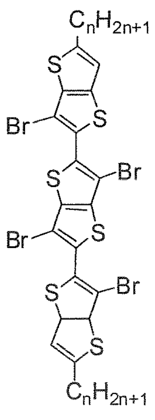
(2-13)

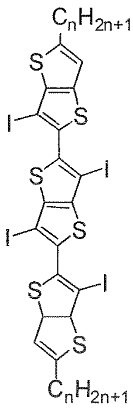
(2-14)

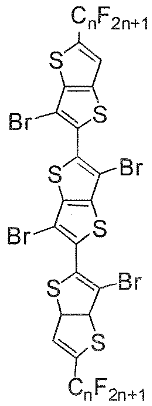
(2-15)

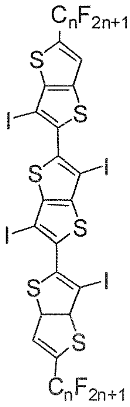
(2-16)

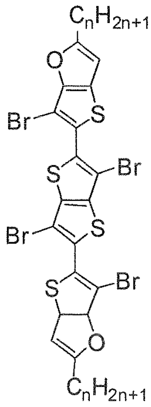
(2-17)

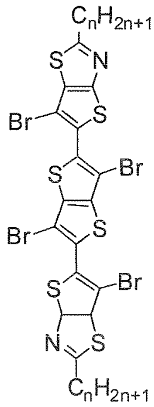
(2-18)

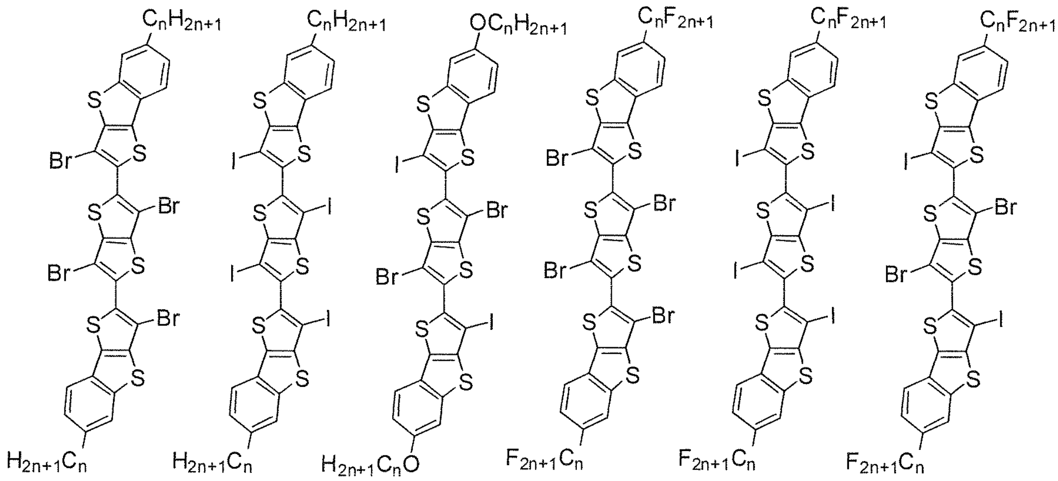
(2-19) (2-20) (2-21) (2-22) (2-23) (2-24)

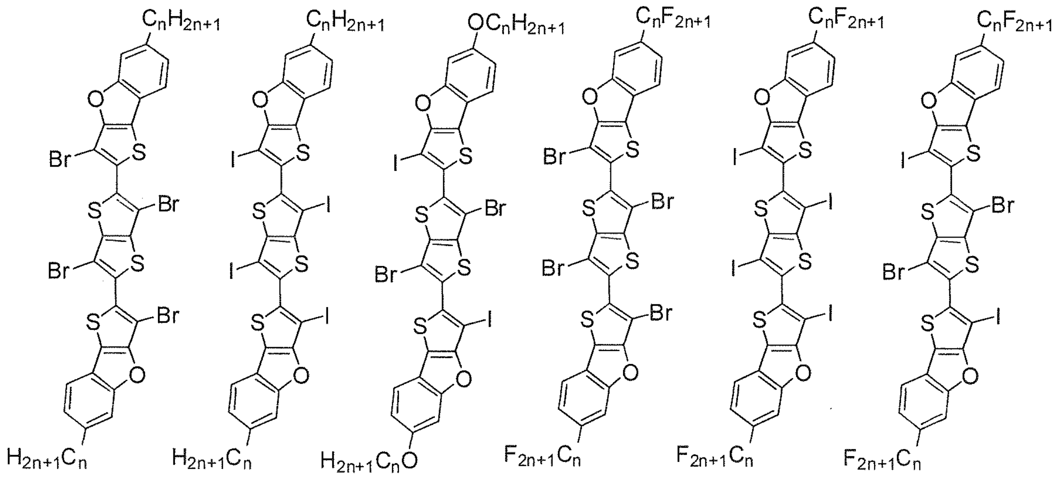

(2-25) (2-26) (2-27) (2-28) (2-29) (2-30)

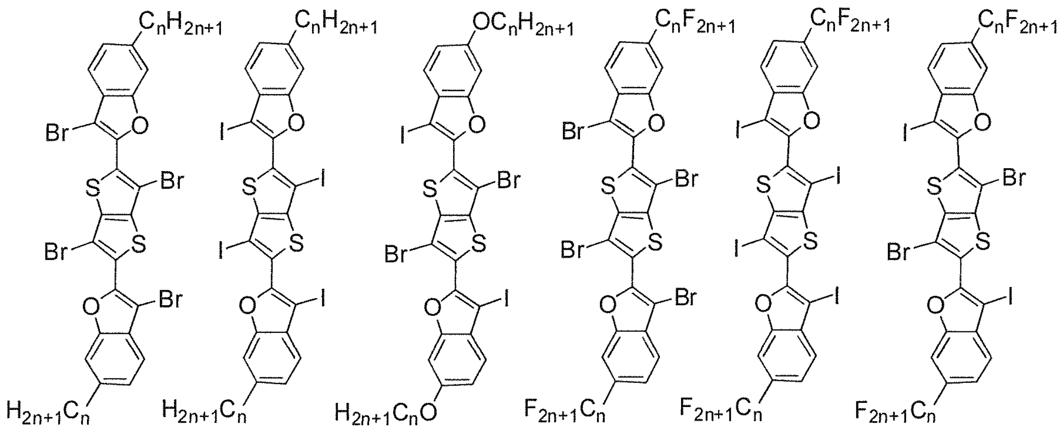

(2-31) (2-32) (2-33) (2-34) (2-35) (2-36)

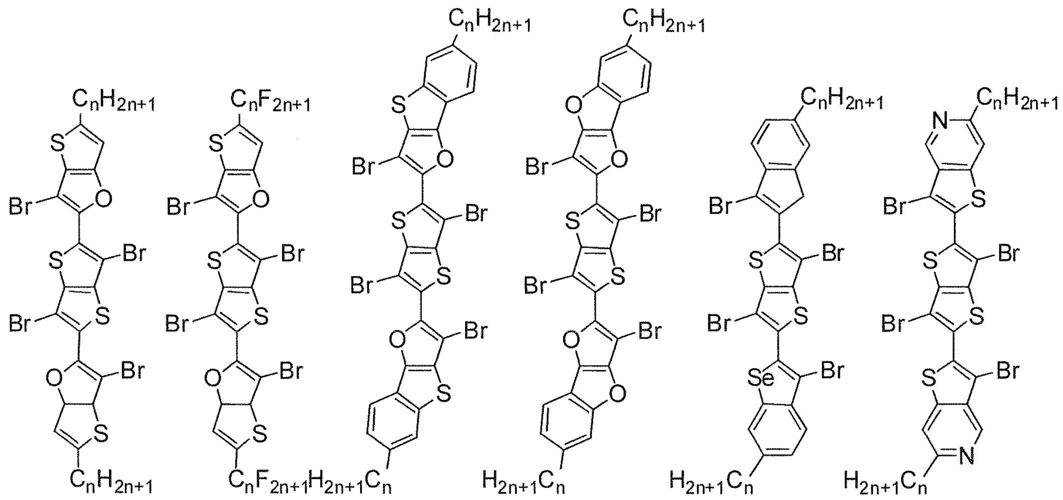

(2-37) (2-38) (2-39) (2-40) (2-41) (2-42)

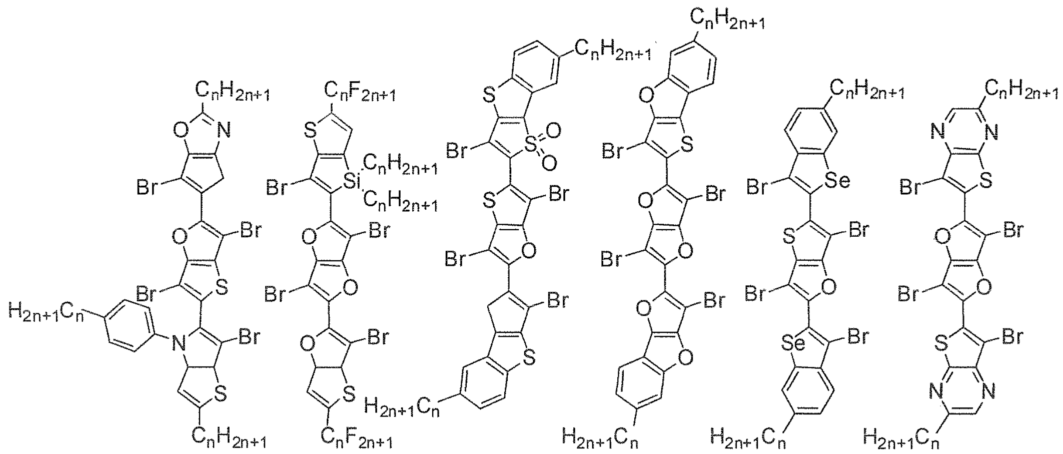

(2-43) (2-44) (2-45) (2-46) (2-47) (2-48)

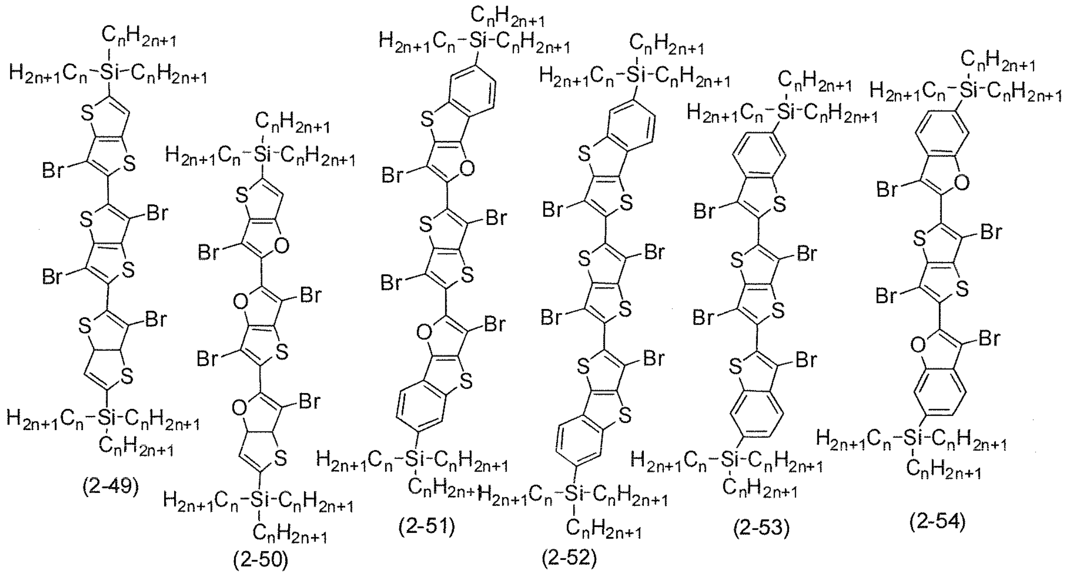

(2-49) (2-50) (2-51) (2-52) (2-53) (2-54)

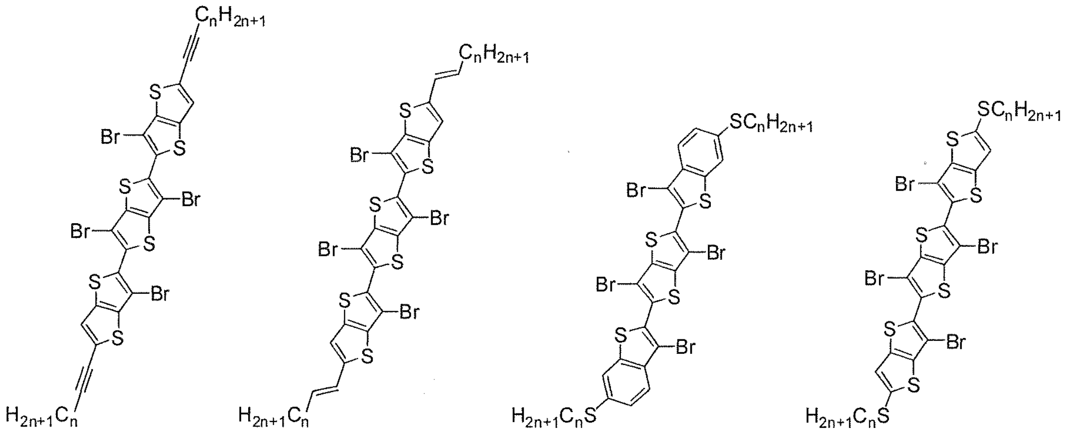

(2-55) (2-56) (2-57) (2-58)

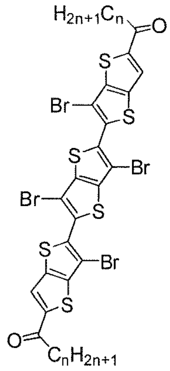
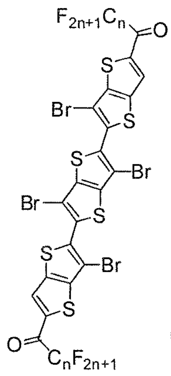
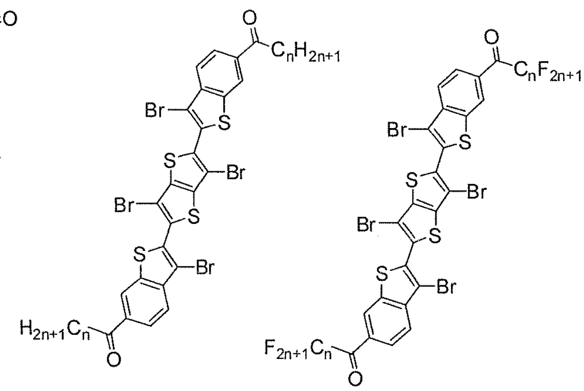

(2-59) (2-60) (2-61) (2-62)

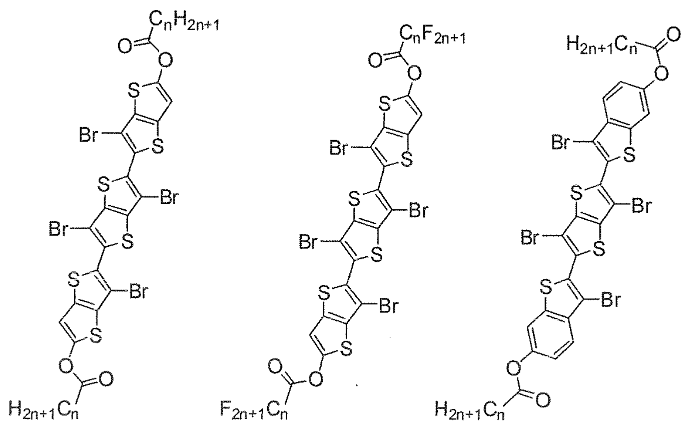
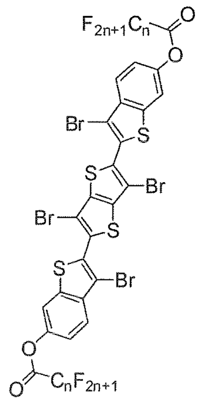

(2-63) (2-64) (2-65) (2-66)

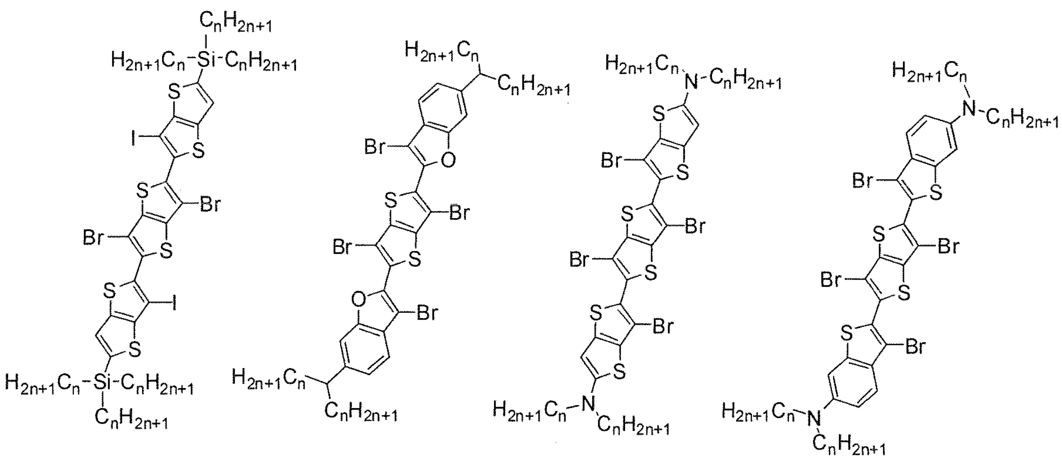

(2-67) (2-68) (2-69) (2-70)

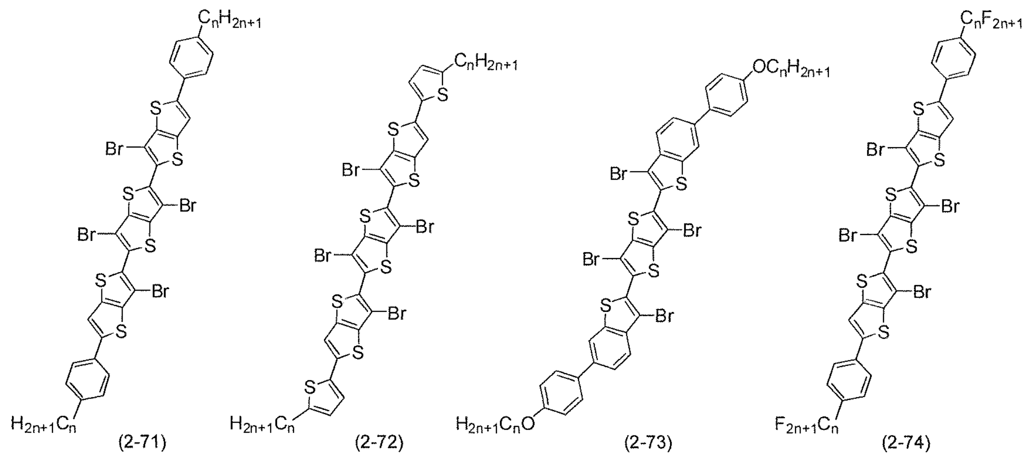

(2-71) (2-72) (2-73) (2-74)

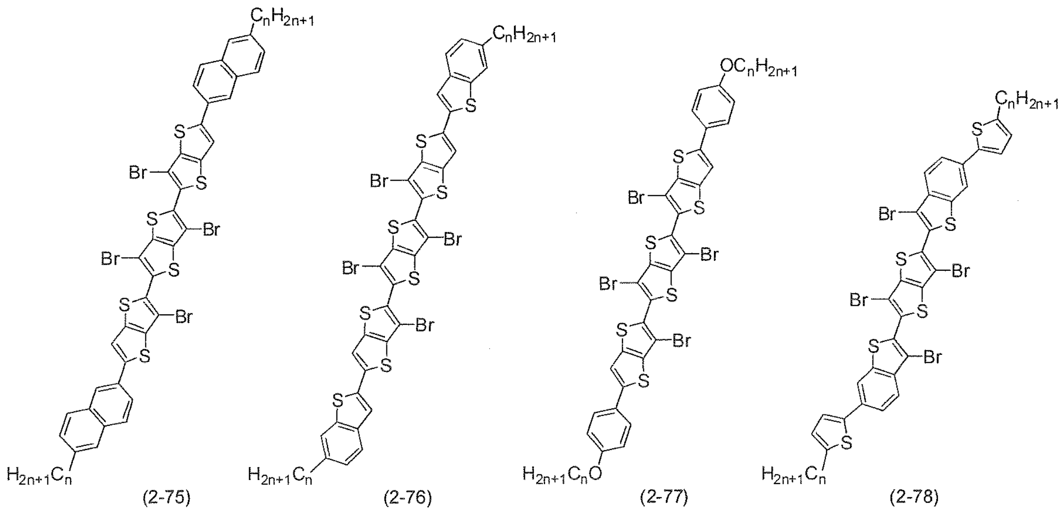

(2-75) (2-76) (2-77) (2-78)

Examples of the tetrahalogen compound preferably include (2-1), (2-4), (2-7), (2-10), (2-13), (2-15), (2-19), (2-22), (2-25), (2-28), (2-31), (2-34), (2-37), (2-38), (2-39), (2-40), (2-49), (2-51), (2-52), (2-53), and (2-54), and more preferably (2-1), (2-4), (2-7), (2-10), (2-13), (2-19), (2-22), (2-31), (2-34), (2-49), and (2-53).

Specific examples of the tetrahalogen compound (3) of the present invention include, but are not limited to, compounds shown below. Provided that n each represents an integer of 0 to 30.

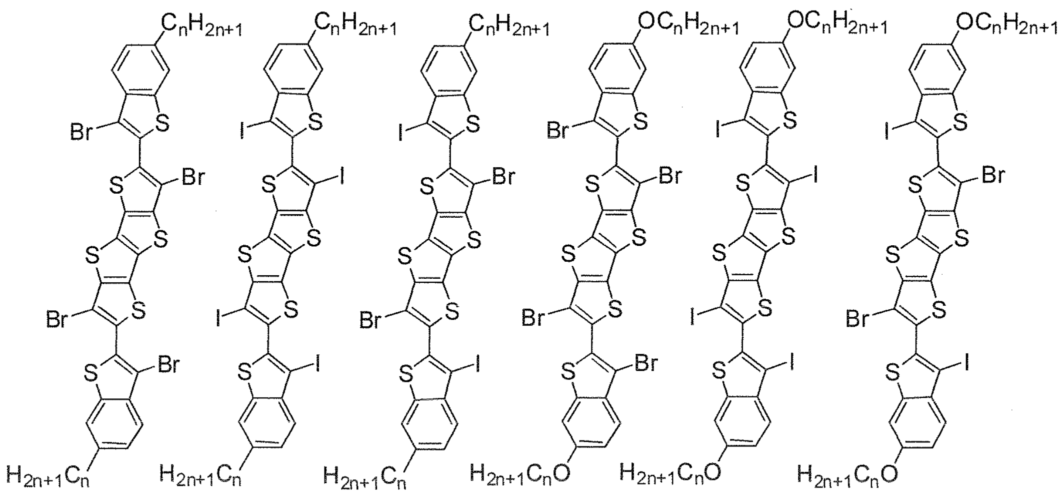

(3-1) (3-2) (3-3) (3-4) (3-5) (3-6)

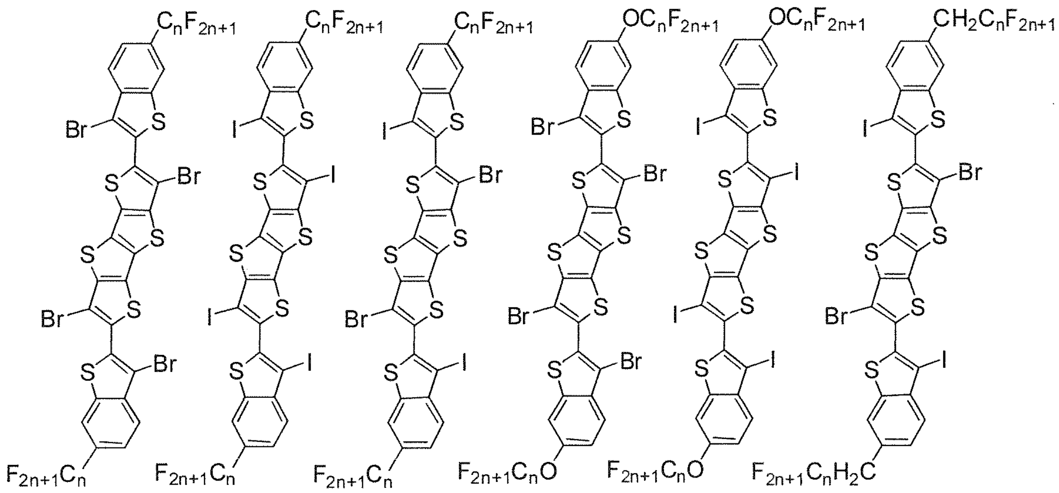

(3-7) (3-8) (3-9) (3-10) (3-11) (3-12)

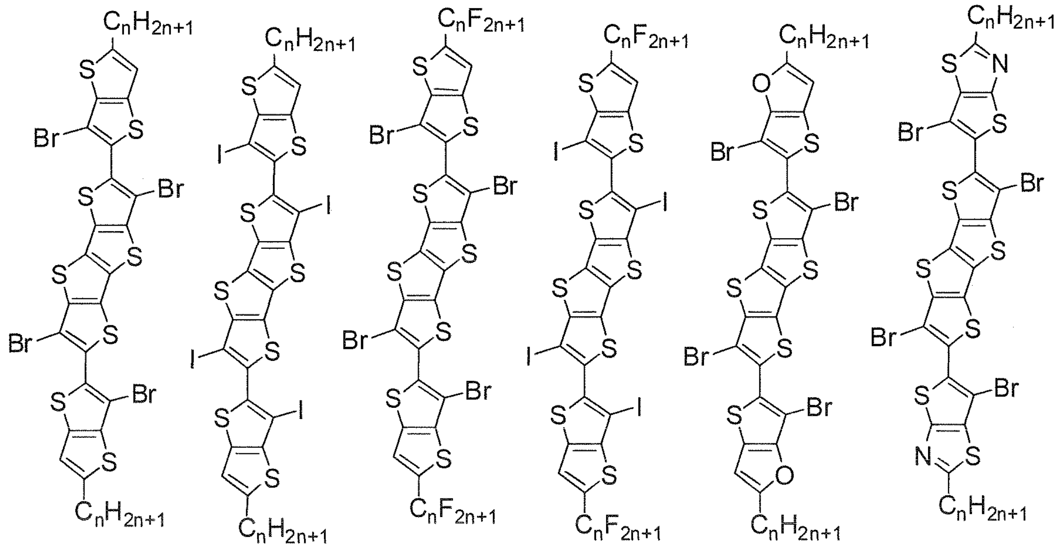

(3-13) (3-14) (3-15) (3-16) (3-17) (3-18)

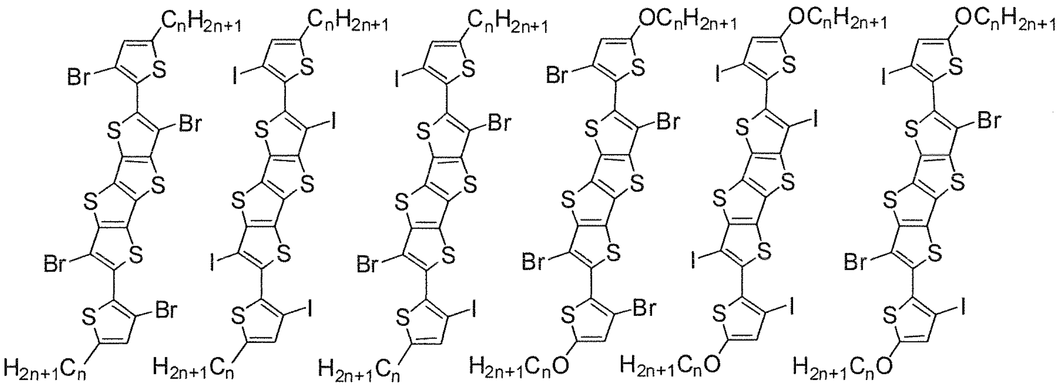

(3-19) (3-20) (3-21) (3-22) (3-23) (3-24)

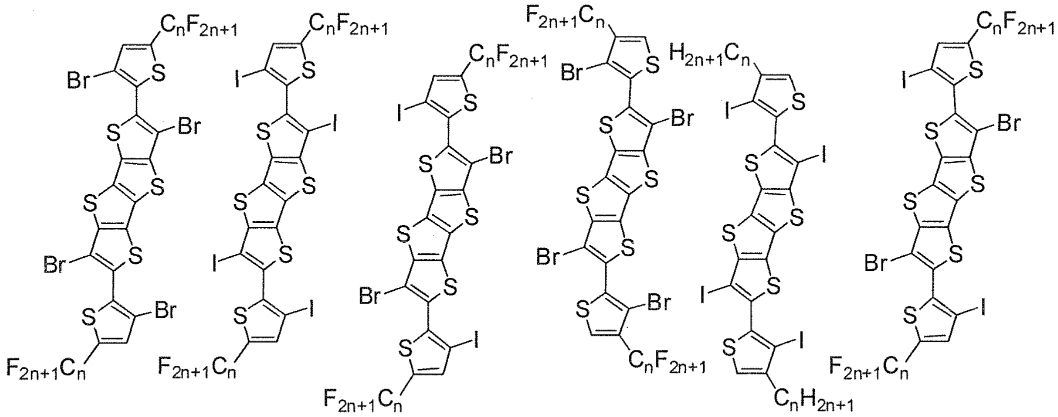

(3-25) (3-26) (3-27) (3-28) (3-29) (3-30)

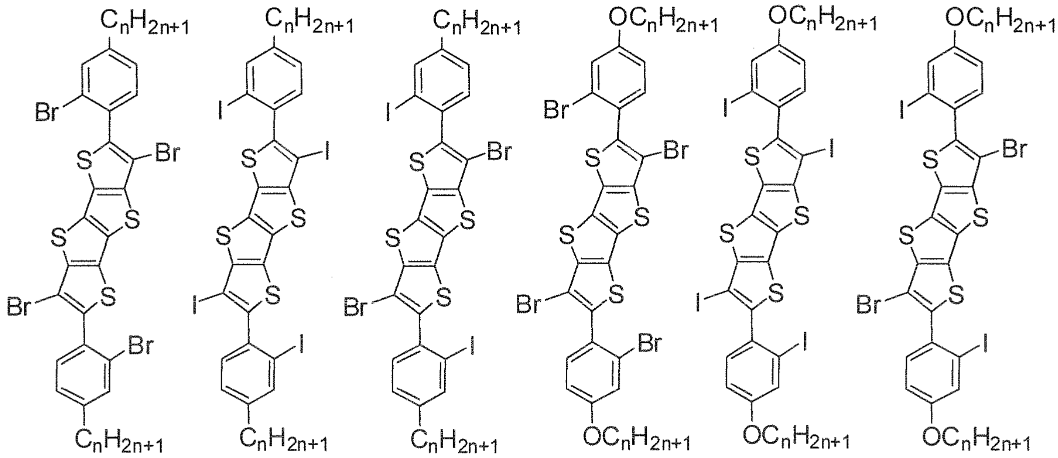

(3-31) (3-32) (3-33) (3-34) (3-35) (3-36)

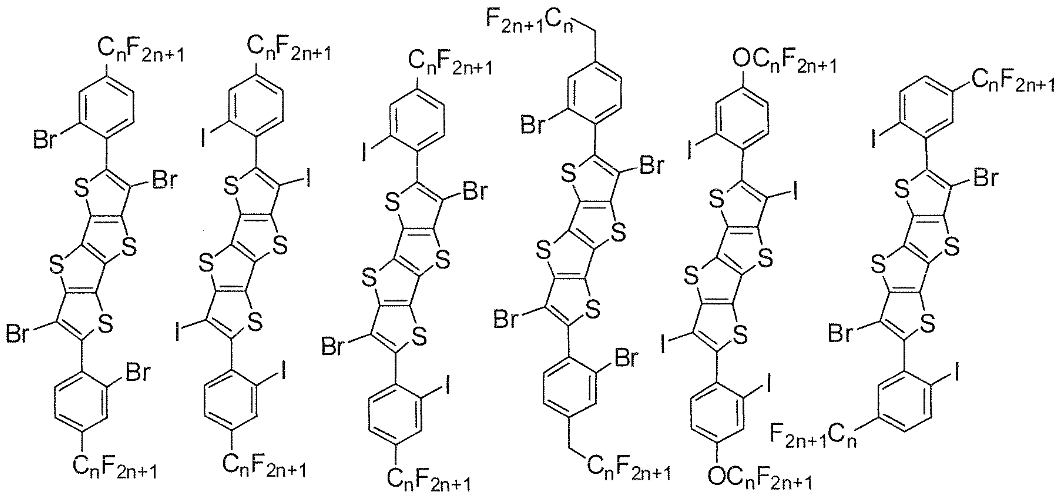

(3-37) (3-38) (3-39) (3-40) (3-41) (3-42)

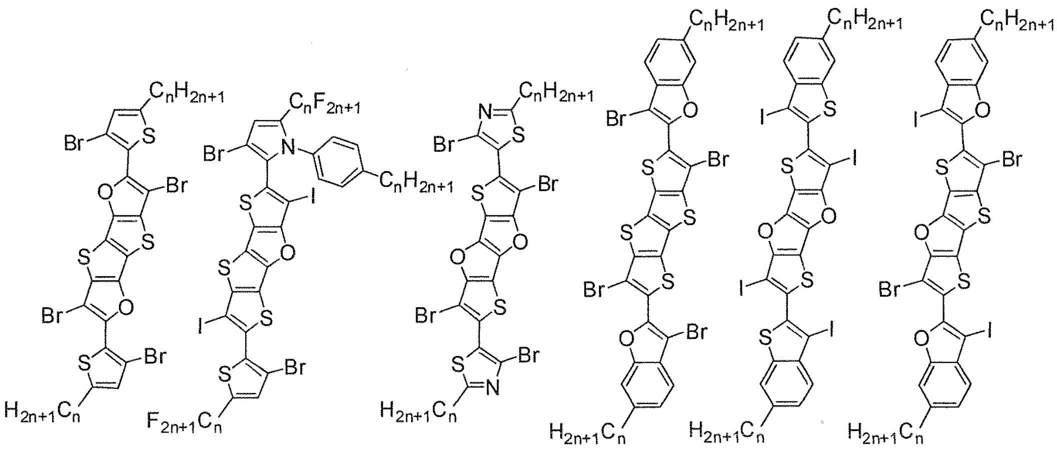

(3-43) (3-44) (3-45) (3-46) (3-47) (3-48)

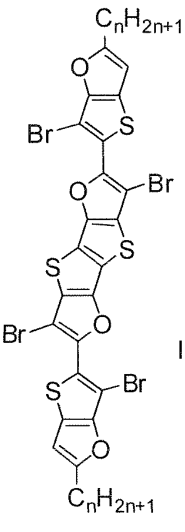
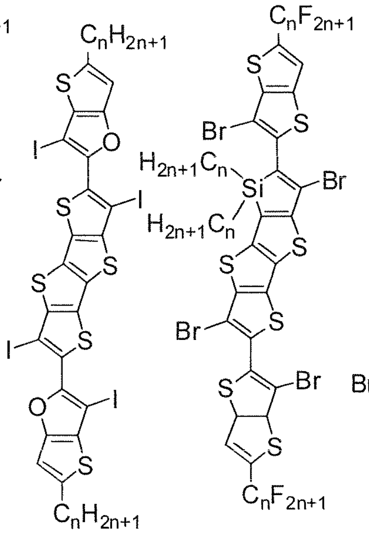
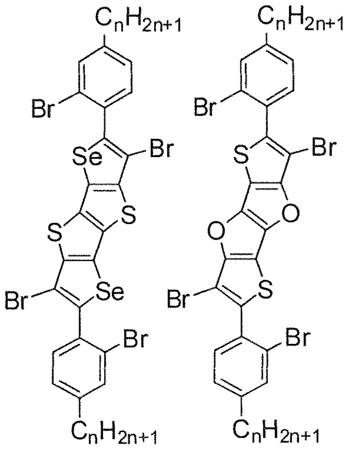
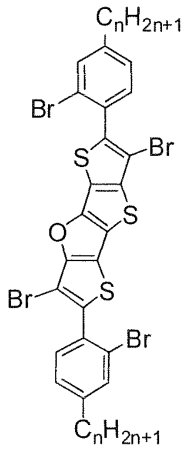

(3-49) (3-50) (3-51) (3-52) (3-53) (3-54)

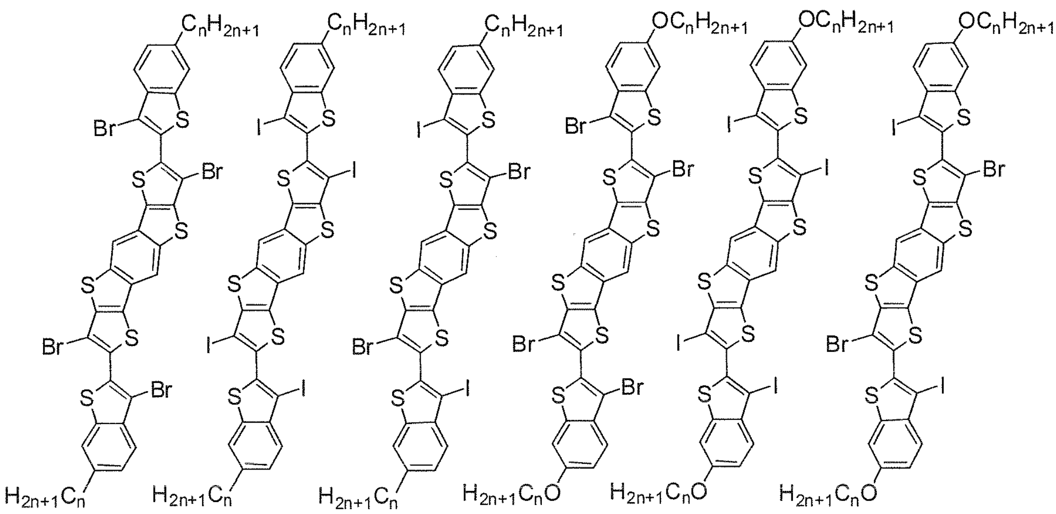

(3-55) (3-56) (3-57) (3-58) (3-59) (3-60)

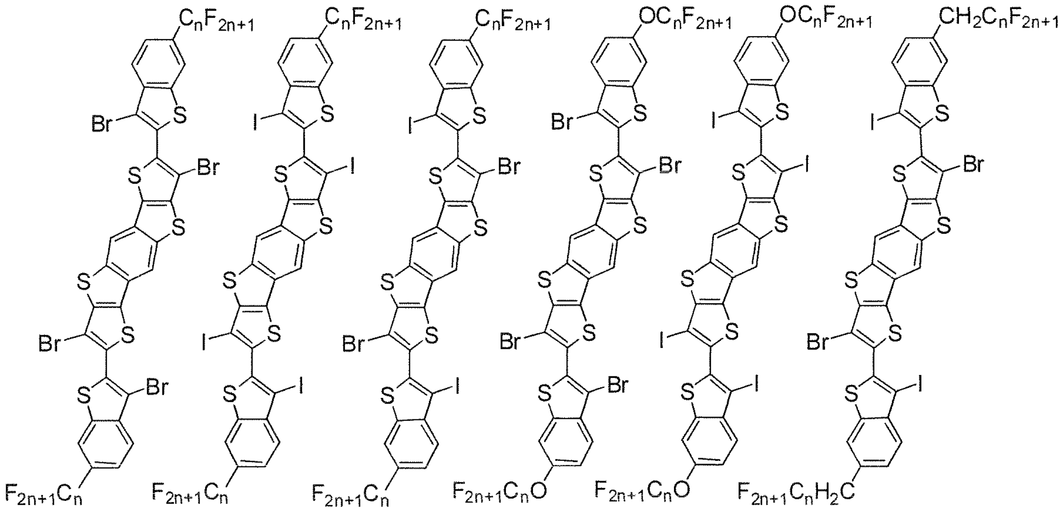

(3-61) (3-62) (3-63) (3-64) (3-65) (3-66)

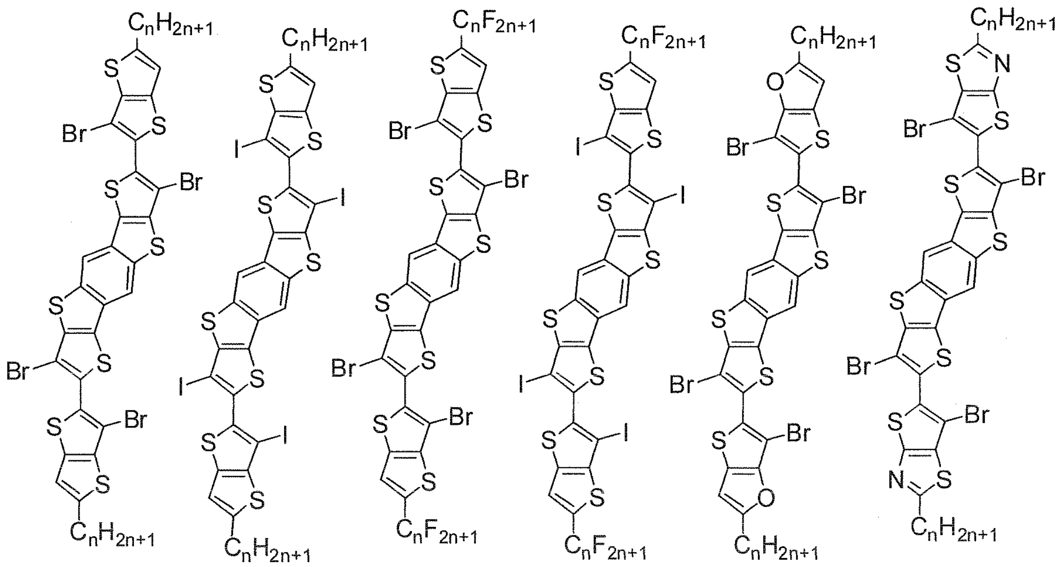

(3-67) (3-68) (3-69) (3-70) (3-71) (3-72)

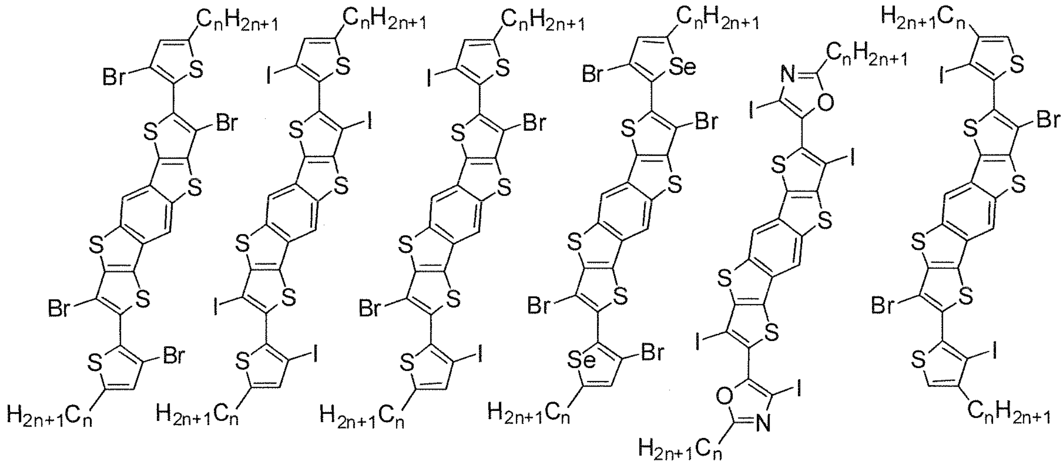

(3-73) (3-74) (3-75) (3-76) (3-77) (3-78)

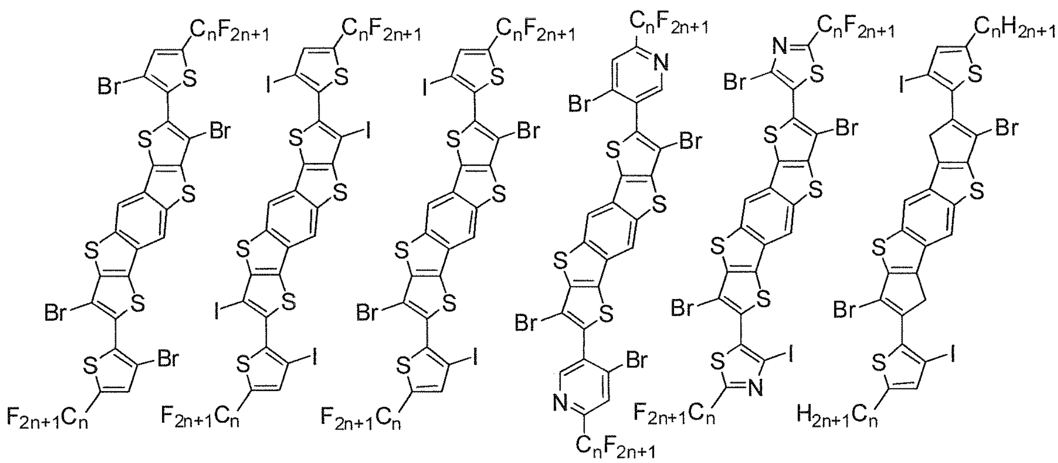

(3-79) (3-80) (3-81) (3-82) (3-83) (3-84)

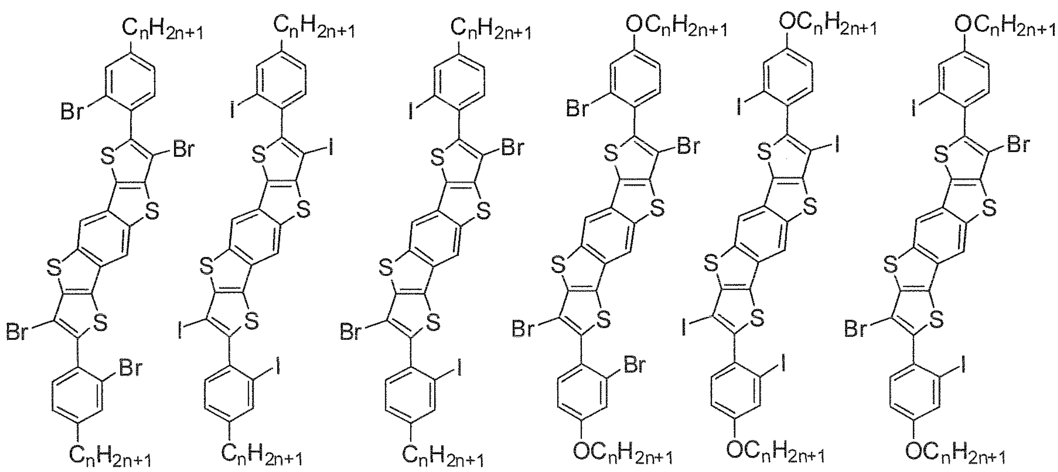

(3-85) (3-86) (3-87) (3-88) (3-89) (3-90)

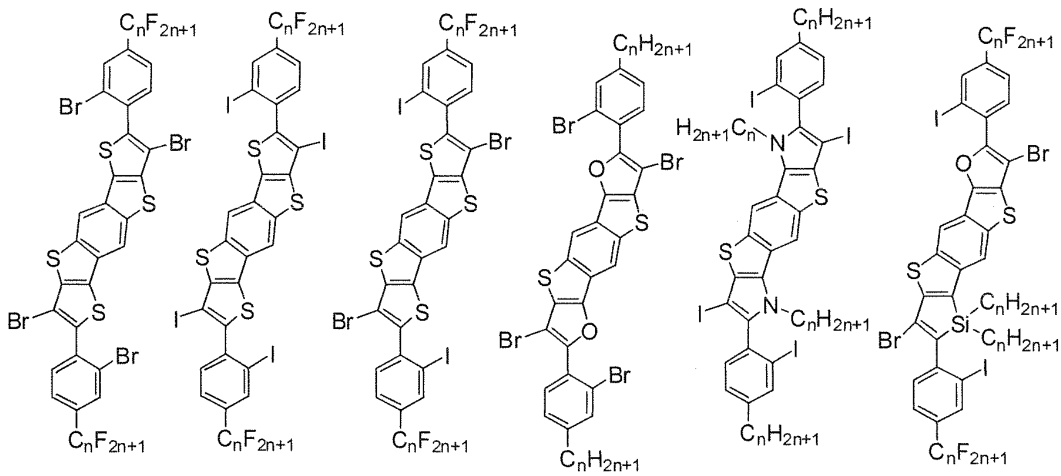

(3-91) (3-92) (3-93) (3-94) (3-95) (3-96)

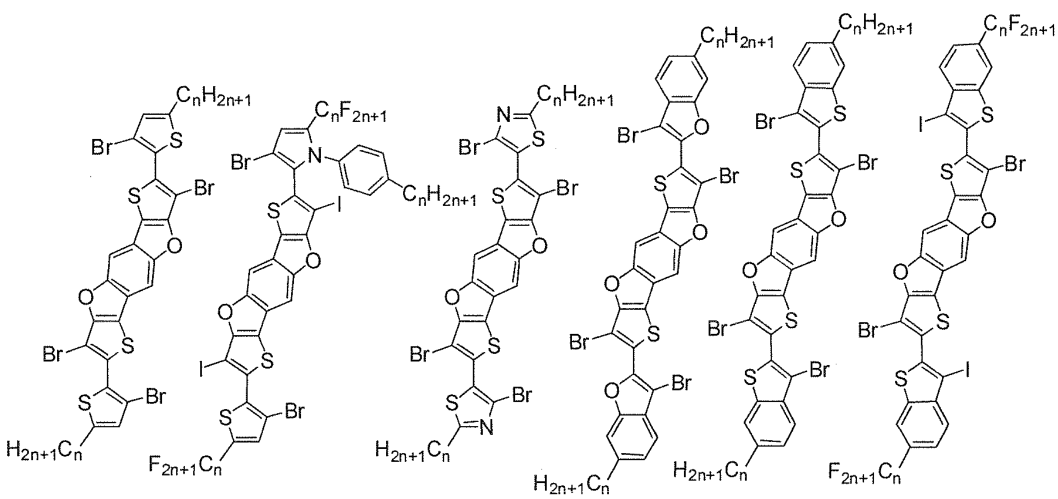

(3-97) (3-98) (3-99) (3-100) (3-101) (3-102)

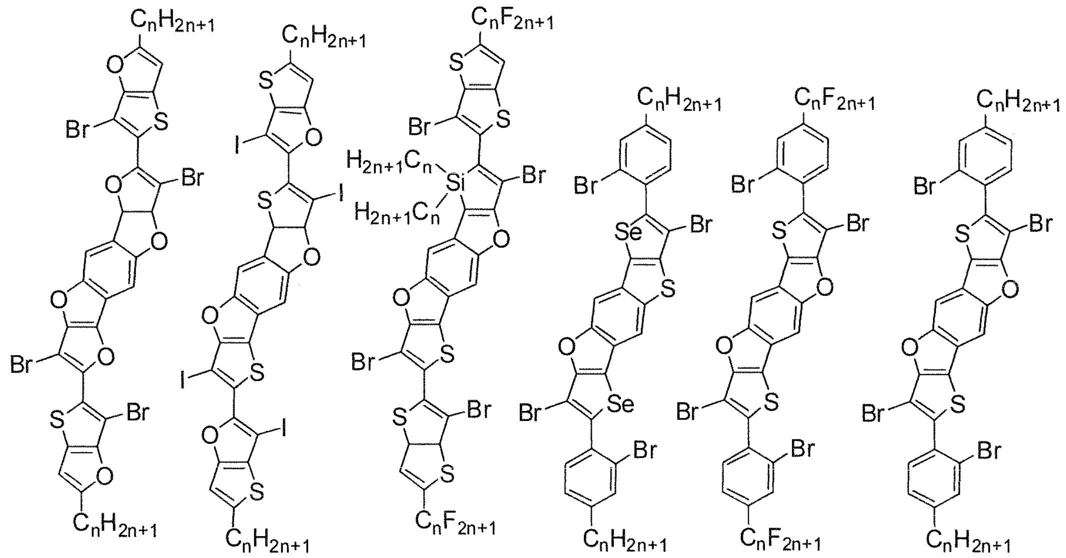

(3-103) (3-104) (3-105) (3-106) (3-107) (3-108)

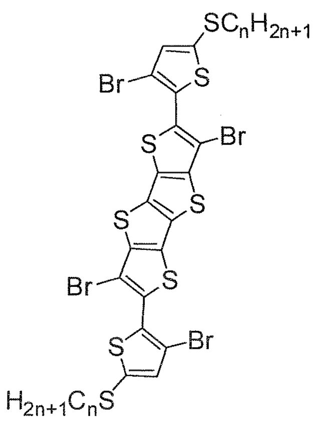

(3-109)

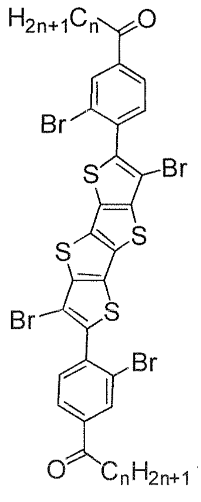

(3-110)

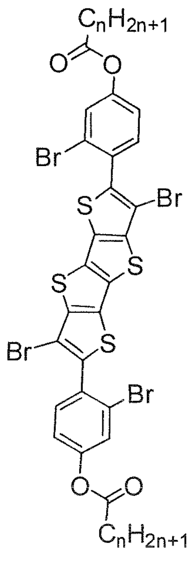

(3-111)

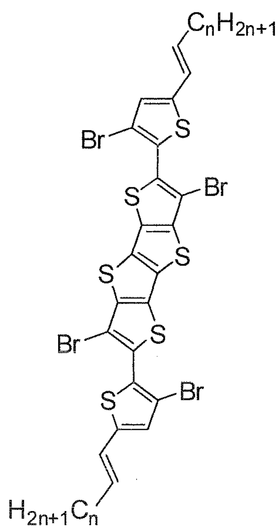

(3-112)

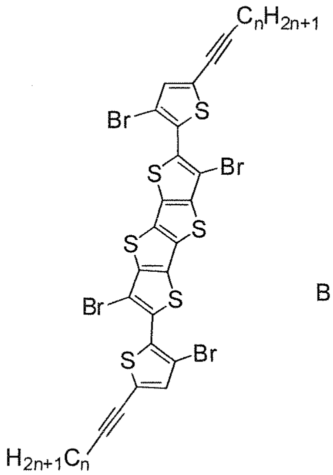

(3-113)

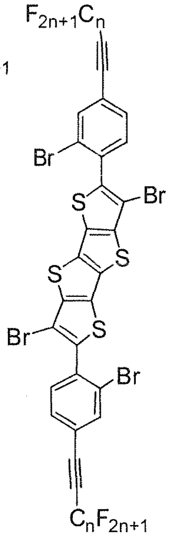

(3-114)

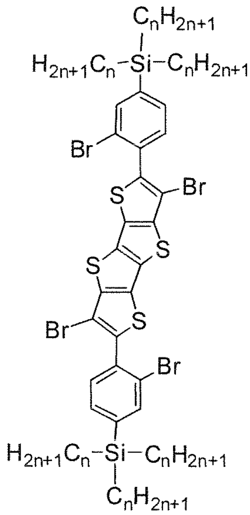

(3-115)

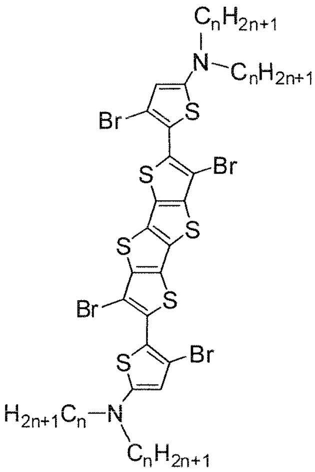

(3-116)

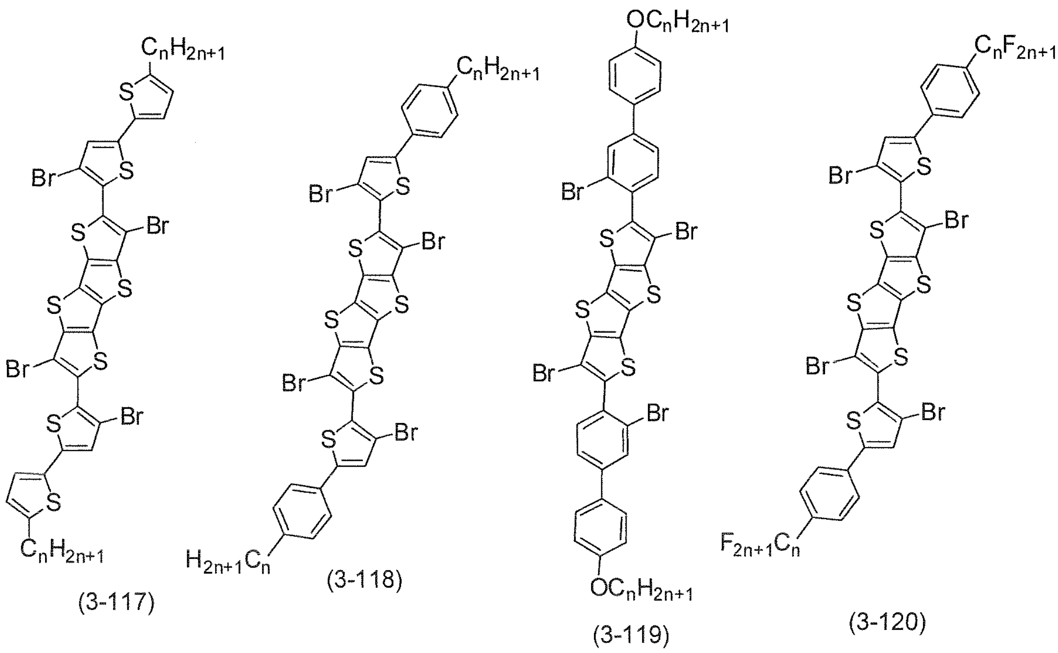

(3-117)

(3-118)

(3-119)

(3-120)

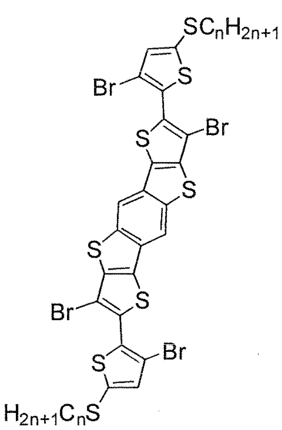

(3-121)

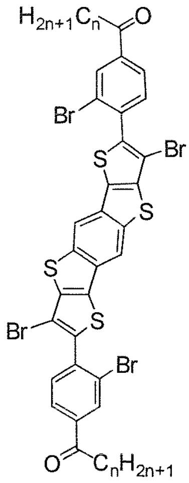

(3-122)

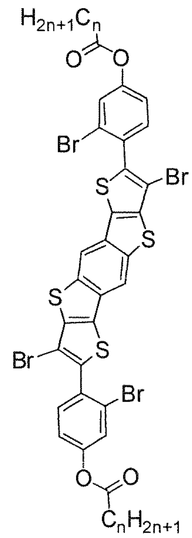

(3-123)

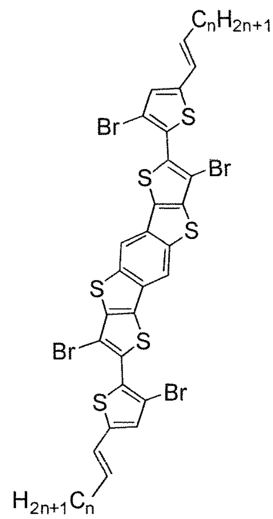

(3-124)

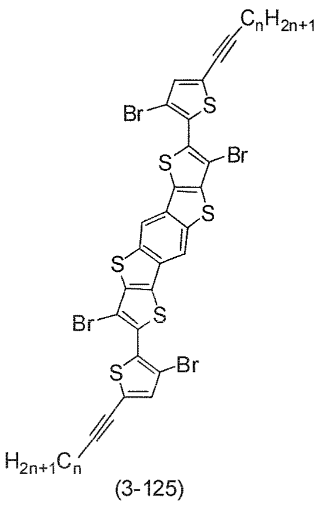

(3-125)

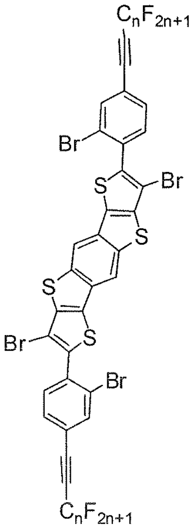

(3-126)

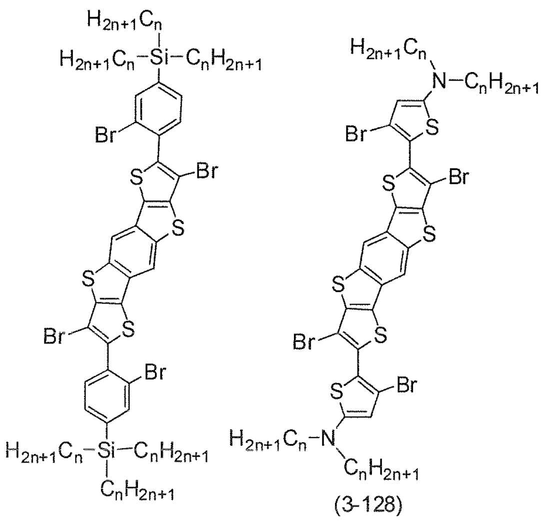

(3-127)

(3-128)

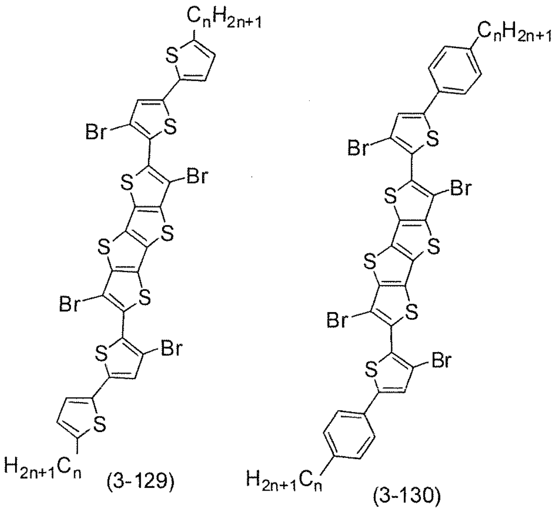

(3-129) (3-130)

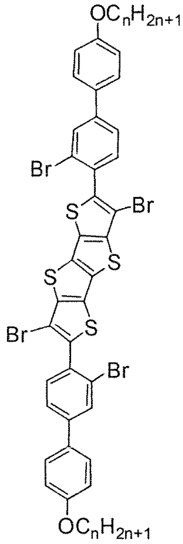

(3-131)

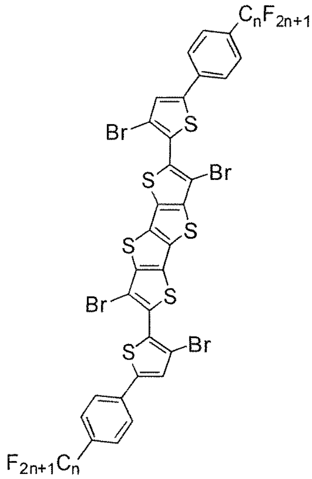

(3-132)

Examples of the tetrahalogen compound (3) preferably include (3-1), (3-4), (3-7), (3-10), (3-13), (3-15), (3-19), (3-22), (3-25), (3-31), (3-34), (3-37), (3-46), (3-55), (3-58), (3-61), (3-64), (3-67), (3-69), (3-73), (3-79), (3-85), (3-88), (3-91), (3-97), (3-100), (3-101), (3-107), and (3-108), and more preferably (3-1), (3-4), (3-7), (3-10), (3-13), (3-19), (3-31), (3-34), (3-37), (3-46), (3-55), (3-58), (3-61), (3-64), (3-67), (3-85), (3-88), (3-91), (3-100), (3-101), (3-107), and (3-108).

The tetrahalogen compound (2) can be prepared by a reaction of the compound represented by the formula (4) with a halogenating agent:

Formula (4)

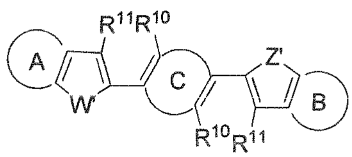

(4)

wherein a ring structure A, a ring structure B, a ring structure C, W' and Z' are as defined above, and $R^{10}$ and $R^{11}$ each represent a hydrogen atom or a halogen atom, provided that at least one of $R^{10}$ and $R^{11}$ represents a hydrogen atom.

The tetrahalogen compound (3) can be prepared by a reaction of the compound represented by the formula (5) with a halogenating agent:

Formula (5)

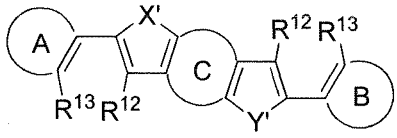

(5)

wherein a ring structure A, a ring structure B, a ring structure C, X' and Y' are as defined above, and $R^{12}$ and $R^{13}$ each represent a hydrogen atom or a halogen atom, provided that at least one of $R^{12}$ and $R^{13}$ represents a hydrogen atom.

Each of a method for producing a tetrahalogen compound (2), including a step of carrying out a reaction of the

compound represented by the formula (4) with a halogenating agent, and a method for producing a tetrahalogen compound (3), including a step of carrying out a reaction of the compound represented by the formula (5) with a halogenating agent is also one aspect of the present invention.

In the formula (4), it is preferred that $R^{10}$ and $R^{11}$ independently represent hydrogen, bromine or iodine atom, and at least one of $R^{10}$ and $R^{11}$ is a hydrogen atom.

In the formula (5), it is preferred that $R^{12}$ and $R^{13}$ independently represent hydrogen, bromine or iodine atom, and at least one of $R^{12}$ and $R^{13}$ is a hydrogen atom.

In the formulas (4) and (5), it is preferred that W', X', Y' and Z' independently represent a sulfur atom or an oxygen atom.

Examples of the compound represented by the formula (4) include 2,2'-(2,5-dibromobenzene-1,4-diyl)bis(1-benzothiophene), 2,2'-(2,5-diiodobenzene-1,4-diyl)bis(1-benzothiophene), 2,2'-(2,5-dichlorobenzene-1,4-diyl)bis(1-benzothiophene), 2,2'-(2,5-dibromobenzene-1,4-diyl)bis(1-benzofuran), 2,2'-(2,5-diiodobenzene-1,4-diyl)bis(1-benzofuran), 2,2'-(2,5-dichlorobenzene-1,4-diyl)bis(1-benzofuran), 2,5-bis(1-benzothiophen-2-y1)thieno[3,2-b]thiophene, 2,5-bis(1-benzofuran-2-yl)thieno[3,2-b]thiophene, 2,5-bis(1-benzothiophen-2-yl)furo[3,2-b]furan, 2,5-bis(1-benzofuran-2-yl) furo[3,2-b]furan, 2,5-bis(3-bromo-1-benzothiophen-2-yl)thieno[3,2-b]thiophene, 2,6-bis(1-benzothiophen-2-yl)benzo [1,2-b;4,5-b']dithiophene, 2,6-bis(1-benzofuran-2-yl)benzo[1,2-b;4,5-b']dithiophene, 2,6-bis(1-benzothiophen-2-yl)benzo[l,2-b;4,5-b']difuran, 2,6-bis(1-benzofuran-2-yl)benzo[1,2-b;4,5-b']difuran, and 2,6-bis(3-bromo-1-benzothiophen-2-yl)benzo[1,2-b;4,5-b']dithiophene.

Examples of the compound represented by the formula (5) include 2,6-diphenylbenzo[1,2-b;4,5-b']dithiophene, 2,6-bis(2-bromophenyl)benzo[1,2-b;4,5-b']dithiophene, 2,6-diphenyl-3,7-dibromobenzo[1,2-b;4,5-b']dithiophene, 2,6-diphenylbenzo[1,2-b;4,5-b']difuran, 2,6-bis(2-bromophenyl)benzo[1,2-b;4,5-b']difuran, 2,6-bis(1-benzothiophen-2-yl) benzo[1,2-b;4,5-b']dithiophene, 2,6-bis(3-bromo-1-benzothiophen-2-yl)benzo[1,2-b;4,5-b']dithiophene, 2,6-bis(1-benzofuran-2-yl)benzo[1,2-b;4,5-b']dithiophene, 2,6-bis(3-bromo-1-benzofuran-2-yl)benzo[1,2-b;4,5-b']dithiophene, 2,6-bis(1-benzothiophen-2-yl)benzo[1,2-b;4,5-b']difuran, and 2,6-bis(3-bromo-1-benzothiophen-2-yl)benzo[1,2-b;4,5-b']difuran.

The compound represented by the formula (4) and the compound represented by the formula (5) can be each synthesized in accordance with Synth. Met. 2002, 130, 139; J. Org. Chem. 2003, 68, 9813; J. Am. Chem. Soc. 2007, 129, 12386, and the like.

The reaction of the compound represented by the formula (4) with a halogenating agent, and the reaction of the compound represented by the formula (5) with a halogenating agent are each usually carried out in an organic solvent.

Examples of the organic solvent in the respective reactions include solvents exemplified above, and preferably toluene, chloroform, carbon tetrachloride, and N,N-dimethylformamide.

In the respective reactions, the use amount of the halogenating agent is usually from 0.1 mol time to 50 mol times, preferably from 0.5 mol time to 20 mol times, and more preferably from 1 mol time to 10 mol times, relative to the compound represented by the formula (4) or the compound represented by the formula (5).

Each concentration of the compound represented by the formula (4) or the compound represented by the formula (5) in a reaction solution is not particularly limited, and is usually within a range from 0.0001 mol to 20 mol, preferably from 0.001 mol to 10 mol, and more preferably from 0.01 mol to 5 mol, per liter of the solvent.

Examples of the halogenating agent include N-bromosuccinimide, 2-bromoacetoamide, bromine, iodine, iodine-periodic acid, iodine monochloride-peracetic acid, benzyltrimethylammonium dichloriodide-zinc(II) chloride, and the like, and preferably N-bromosuccinimide, bromine, and iodine-periodic acid.

In the case where the reaction does not easily proceed, additives such as benzoyl peroxide and azobisbutyronitrile may be added in an amount of a catalyst.

The reaction temperature is usually within a range from -78°C to a reflux temperature of a reaction solution, preferably 200°C, and more preferably from -20°C to 50°C. The reaction time is not limited, and is usually from 1 minute to 48 hours.

In the case where the present reaction is stopped, water or the like is added to the reaction solution. A crude product can be usually obtained by a post-treatment, for example, an operation such as extraction or washing after stopping of the reaction. The crude product may be purified by operations such as crystallization, sublimation and various chromatographies.

When the compound I of the present invention is formed into a film having a thickness of 1 nm to 10 μm, and preferably a thickness of 5 nm to 1 μm, as described above, the obtained film exhibits luminosity, or conductivity similar to that of a semiconductor. Therefore, the compound I of the present invention is excellent as an organic semiconductor material.

Each of an organic semiconductor device including the compound I of the present invention, an organic semiconductor thin film including the compound I, a conductivity thin film including the compound I, and a light emitting thin film including the compound I is also one aspect of the present invention.

In the present description, the light emitting thin film means a film having a thickness of 1 nm to 10 μm, which

emits light under the condition of light or electrical stimulation. The light emitting thin film is also useful as a material of the light emitting element. The light emitting element including the light emitting thin film is also one aspect of the present invention. The emitting element of the present invention is, for example, useful as a material such as an organic light emitting diode.

In the present description, the light emitting element means a device using a light emitting thin film.

In the present description, the conductivity thin film means a film having a thickness of 1 nm to 10 μm, which exhibits conductivity under the condition of light or electrical stimulation. The conductivity thin film is useful as a material such as an organic semiconductor device described hereinafter.

The organic semiconductor thin film, conductivity thin film and light emitting thin film of the present invention can be each produced in the same manner as in a conventionally known method, except that the compound I of the present invention is used as a material.

The organic semiconductor device will be described below.

The organic semiconductor device of the present invention contains the compound I of the present invention. The organic semiconductor device commonly includes an organic transistor, and the organic transistor includes an organic semiconductor thin film containing the compound I. The organic semiconductor thin film containing the compound I is also one aspect of the present invention.

The organic transistor contains the compound I of the present invention and therefore has high carrier mobility. The organic transistor can control the carrier mobility to $10^{-6}$ $cm^2/Vs$ or more.

In the present description, the carrier mobility can be measured by applying the formula shown below with respect to a drain current and a gate voltage measured using a parameter analyzer or the like:

$$Id = (W/2L)\mu Ci(Vg-Vt)2 \qquad (a)$$

wherein Id is a drain current in a saturation range of electrical characteristics, L is a channel length of an organic transistor, W is a channel width of an organic transistor, Ci is a capacity per unit area of a gate insulating film, Vg is a gate voltage, and Vt is a threshold voltage of a gate voltage.

Examples of the organic field effect transistor of the present invention include an organic field effect transistor.

Regarding the organic field effect transistor, a source electrode and a drain electrode are usually contacted with a semiconductor layer, and a gate electrode may be further provided interposing an insulating layer (dielectric layer) contacting with an active layer.

Examples of the element structure of the organic transistor include (1) a structure consisting of substrate/gate electrode/insulator layer/source electrode and drain electrode/semiconductor layer, (2) a structure consisting of substrate/semiconductor layer + source electrode and drain electrode/insulator layer/gate electrode, (3) a structure consisting of substrate/source electrode (or drain electrode)/semiconductor layer + insulator layer + gate electrode/drain electrode (or source electrode), and (4) a structure consisting of substrate/gate electrode/insulator layer/semiconductor layer/source electrode (or drain electrode).

In the respective structures, the semiconductor layer includes an organic semiconductor thin film of the present invention. In the respective structures, a plurality of the semiconductor layers exist, they may be provided in the same plane, or as a laminate. In the respective structures, a plurality of source electrodes, drain electrode and gate electrode may be provided.

Examples of the method for forming the organic semiconductor thin film into a semiconductor layer include formation methods by a vacuum process, such as a vacuum vapor deposition method, a sputtering method, a CVD method, and a molecular beam epitaxial growth method, and preferably a vacuum vapor deposition method.

The vacuum vapor deposition method is a method in which an organic semiconductor material such as the compound I is heated under vacuum in a crucible or a metal boat, and the evaporated organic semiconductor material is vapor deposited on a substrate or an insulator material.

The degree of vacuum at vapor deposition is usually $1 \times 10^{-1}$ Pa or less, and preferably $1 \times 10^{-3}$ Pa or less. The temperature of the substrate at vapor deposition is usually from 0°C to 300°C, and preferably from 20°C to 200°C. The rate of vapor deposition is usually from 0.001 nm/sec to 10 nm/sec, and preferably from 0.01 nm/sec to 1 nm/sec. The film thickness of the organic semiconductor thin film is usually from 1 nm to 10 μm, and preferably from 5 nm to 1 μm.

The method for forming an organic semiconductor thin film may use a solution process. The solution process is a method of coating a solution or dispersion, which is prepared by dissolving or dispersing an organic semiconductor material in a solvent, on a substrate or an insulator layer.

Examples of the coating methods include those such as a casting method, a dip coating method, a die coater method, a roll coater method, a bar coater method and a spin coat method, an ink jet method, a screen printing method, an offset printing method, a microcontact printing method, and the like. These methods may be used alone or in combination of two or more.

In the present invention, materials constituting a source electrode, a drain electrode and a gate electrode are not particularly limited as long as they are common conductive materials, and platinum, gold, silver, nickel, chromium, copper, iron, tin, antimonial lead, tantalum, indium, palladium, tellurium, rhenium, iridium, aluminum, ruthenium, germanium, molybdenum, tungsten, tin-antimony oxide, indium-tin oxide (ITO), fluorine-doped zinc oxide, zinc, carbon, graphite, glassy carbon, silver paste and carbon paste, lithium, beryllium, sodium, magnesium, potassium, calcium, scandium, titanium, manganese, zirconium, gallium, niobium, sodium, a sodium-potassium alloy, magnesium, lithium, aluminum, a magnesium/copper mixture, a magnesium/silver mixture, a magnesium/aluminum mixture, a magnesium/indium mixture, an aluminum/aluminum oxide mixture, a lithium/aluminum mixture, and the like, are used, and platinum, gold, silver, copper, aluminum, indium, ITO and carbon are particularly preferred. Alternatively, well-known conductive polymers improved in electric conductivity by doping or the like, for example, conductive polyaniline, conductive polypyrrole and conductive polythiophene, a complex of a polyethylene dioxythiophene and a polystyrene sulfonic acid, and the like, are suitably used. Particularly, materials having a small electric resistance at the contact surface with a semiconductor layer are preferable. These electrode materials may be used alone or as a mixture of two or more.

The film thickness of the electrode varied depending on the material, and is usually from 0.1 nm to 10 $\mu$m, preferably from 0.5 nm to 5 $\mu$m, and more preferably from 1 nm to 3 $\mu$m. In the case of serving both as a gate electrode and a substrate, the film thickness may be more than the above film thickness.

Examples of the method of forming an electrode film include known various methods. Specific examples thereof include a vacuum vapor deposition method, a sputtering method, a coating method, a thermal transfer method, a printing method, a sol-gel method, and the like. At the film formation, or after the film formation, patterning is preferably carried out according to needs. Methods of patterning to be used include various methods. Specific examples thereof include a photolithography in combination of patterning and etching of a photoresist. The methods further include printing methods such as an ink jet printing, a screen printing, an offset printing and a letterpress printing, and a method of a soft lithography such as a microcontact printing method.

Patterning may be carried out using these methods alone or by mixing two or more thereof.

An insulating layer to be used includes various insulating films such as inorganic oxide and organic compound films. Examples of the inorganic oxide include silicon oxide, aluminum oxide, tantalum oxide, titanium oxide, tin oxide, vanadium oxide, barium strontium titanate, barium zirconate titanate, lead zirconate titanate, lead lanthanum titanate, strontium titanate, barium titanate, barium magnesium fluoride, bismuth titanate, strontium bismuth titanate, strontium bismuth tantalate, bismuth niobate tantalite, yttrium trioxide, and the like, and preferable are silicon oxide, aluminum oxide, tantalum oxide, and titanium oxide. Inorganic nitrides such as silicon nitride and aluminum nitride are included.

Examples of the organic compound film include polyimide, polyamide, polyester, polyacrylate, photocuring resins based on photoradical polymerization or photocationic polymerization, copolymers containing an acrylonitrile component, polyvinylphenol, polyvinyl alcohol, novolac resins, cyanoethylpullulans, and the like, and preferably include polyimide, polyvinylphenol and polyvinyl alcohol.

These insulating layer materials may be used alone or in combination of two or more. The film thickness of an insulating layer varies depending on the material, and is usually from 0.1 nm to 100 $\mu$m, preferably from 0.5 nm to 50 $\mu$m, and more preferably from 5 nm to 10 $\mu$m.

Formation methods of an insulating layer to be used include various methods. Specific examples thereof include coating methods such as spin coating, spray coating, dip coating, casting, bar coating and blade coating, printing methods such as screen printing, offset printing and ink jet printing, and dry process methods such as a vacuum vapor deposition method, a molecular beam epitaxial growth method, an ion cluster beam method, an ion plating method, a sputtering method, an atmospheric pressure plasma method and a CVD method. The methods further include a sol-gel method, a method of forming an oxide film on a metal such as alumite on aluminum and a thermally oxidized film of silicon, and the like.

Examples of the material for a substrate include glass, paper, quartz, ceramics, flexible resin sheets, and the like. Specific examples of the resin film include films made of polyethylene terephthalate (PET), polyethylene naphthalate (PEN), polyethersulfone (PES), polyetherimide, polyether ether ketone, polyphenylene sulfide, polyallylate, polyimide, polycarbonate (PC), cellulose triacetate (TAC), cellulose acetatepropionate (CAP), and the like. The thickness of the substrate is usually from 1 $\mu$m to 10 mm, and preferably from 5 $\mu$m to 5 mm.

In portions of the insulator layer and the substrate contacting with the semiconductor layer, a surface treatment may be carried out on the insulator layer and the substrate. The surface treatment on the insulator layer on which the semiconductor layer is to be laminated allows improvement in transistor characteristics of an element. Specific examples of the surface treatment include a hydrophobizing treatment with hexamethyldisilazane, octadecyltrichlorosilane, octyltrichlorosilane or the like, an acid treatment with hydrochloric acid, sulfuric acid, a hydrogen peroxide solution or the like, an ammonia treatment with sodium hydroxide, potassium hydroxide, calcium hydroxide, ammonia or the like, an ozone treatment, a fluorinating treatment, a plasma treatment with oxygen, argon or the like, a treatment to form a Langmuir-Blodgett film, a treatment to form a thin film of another insulator or another semiconductor, a mechanical treatment, an

electric treatment with corona discharge or the like, and a rubbing treatment utilizing fiber or the like.

Examples of the method of the surface treatment include a vacuum vapor deposition method, a sputtering method, a coating method, a printing method, a sol-gel method, and the like.

A protective film made of a resin or an inorganic compound may be provided on a semiconductor layer. The formation of the protective film can suppress an influence of outside air, thus stabilizing the driving of a transistor.

EXAMPLES

The present invention will be described in more detail by way of Examples.

In the respective Examples, the measurement of compounds was carried out by the following method.

1. $^1$H-NMR: measured using EX270 (manufactured by JEOL Ltd.).
2. HRMS: measured using JMS-T100GC (manufactured by JEOL Ltd.).

[Example 1]

Production of 2,2'-(2,5-dibromobenzene-1,4-diyl)bis(1-benzothiophene)

A mixed liquid of 1,4-dibromo-2,5-diiodobenzene (1.38 g, 2.83 mmol), benzo[b]thiophen-2-ylboronic acid (1.5 g, 8.43 mmol), a dichloro-[1,1'-bis(diphenylphosphino)ferrocene]palladium dichloromethane complex (0.26 g, 0.23 mmol), tetrahydrofuran (16 mL) and an aqueous sodium carbonate solution (2M, 8 mL) was refluxed under a nitrogen atmosphere at about 65°C for 12 hours. After the reaction mixed liquid was allowed to cool to room temperature, water was added to the reaction mixed liquid and the reaction mixed liquid was extracted with chloroform. The obtained organic layer was dried over sodium sulfate and filtered, and then the solvent was distilled off under reduced pressure. The obtained mixture was separated and purified by silica gel chromatography to obtain 2,2'-(2,5-dibromobenzene-1,4-diyl) bis(1-benzothiophene) (1.2 g, 2.4 mmol) in a yield of 85%.

Physical properties of 2,2'-(2,5-dibromobenzene-1,4-diyl)bis(1-benzothiophene) were as follows.
$^1$H-NMR($CDCl_3$, δppm):7.91(s, 2H), 7.84-7.90(m, 4H), 7.59(s, 2H), 7.35-7.45(m, 4H)

[Example 2]

Production of 2,2'-(2,5-dibromobenzene-1,4-diyl)bis(3-bromo-1-benzothiophene)

A mixed liquid of 2,2'-(2,5-dibromobenzene-1,4-diyl)bis(1-benzothiophene) (0.47 g, 0.94 mmol), N-bromosuccinimide (0.37 g, 2.07 mmol), benzoyl peroxide (an amount of a catalyst) and chloroform (71 mL) was stirred at room temperature (20 to 25°C) for 8 hours. After the solvent was distilled off, the obtained solid was washed with ethanol and dried under reduced pressure to obtain 2,2'-(2,5-dibromobenzene-1,4-diyl)bis(3-bromo-1-benzothiophene) in a yield of 73%.

Physical properties of 2,2'-(2,5-dibromobenzene-1,4-diyl)bis(3-bromo-1-benzothiophene) were as follows. $^1$H-NMR($CDCl_3$, δppm) : 7.86-7.93(m, 4H), 7.81(s, 2H), 7.41-7.57(m, 4H)

[Example 3]

Production of Compound 1

A mixed liquid of 2,2'-(2,5-dibromobenzene-1,4-diyl)bis(3-bromo-1-benzothiophene) (0.1 g, 0.15 mmol), 4-hexylaniline (0.059 g, 0.33 mmol), tris(dibenzylidineacetone)dipalladium (0.028 g, 0.03 mmol), tri-tert-butylphosphine (0.025 g, 0.12 mmol), sodium tert-butoxide (0.38 g, 4 mmol) and toluene (5 mL) was refluxed under a nitrogen atmosphere at about 110°C for 48 hours. After the reaction mixed liquid was allowed to cool to room temperature and water was added to the reaction mixture liquid, and then the reaction mixture liquid was extracted with chloroform. The obtained organic layer was dried over sodium sulfate and filtered, and then the solvent was distilled off under reduced pressure. The obtained mixture was separated and purified by silica gel chromatography to obtain the compound 1 (0.63 g, 0.9 mmol) represented by the following Chemical Formula 1 in a yield of 60%.

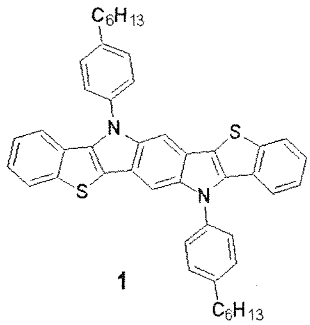

1

Physical properties of the compound 1 were as follows.
$^1$H-NMR ($CDCl_3$, δppm) : 7.86(d, 2H), 7.68(s, 2H), 7.57(d, 4H), 7.47(d, 4H), 7.17-7.33(m, 6H), 2.81(t, 4H), 1.74-1.85(m, 4H), 1.38-1.53(m, 12H), 0.95(t, 6H)
HRMS(MLDI-TOF):calcd for $C_{46}H_{44}N_2S_2$, 688.2946; found 688.2913

[Example 4]

Production of Compound 2

A mixed liquid of 2,2'-(2,5-dibromobenzene-1,4-diyl)bis(3-bromo-1-benzothiophene) (8.8 g, 13.4 mmol), 4-n-dodecylaniline (38.5 g, 147.1 mmol), tris(dibenzylidineacetone) dipalladium (2.45 g, 2.7 mmol), sodium tert-butoxide (11.31 g, 117.7 mmol), tri-tert-butylphosphine (2.16 g, 10.7 mmol) and toluene (430 mL) was refluxed under a nitrogen atmosphere at about 110°C for 15 hours. After the reaction mixed liquid was allowed to cool to room temperature, water was added to the reaction mixed liquid and then the reaction mixed liquid was extracted with chloroform. The obtained organic layer was dried over sodium sulfate and filtered, and then the solvent was distilled off under reduced pressure. The obtained mixture was subjected to silica gel chromatography followed by recrystallization from chloroform to obtain the compound 2 (5.11 g, 5.96 mmol) represented by the following formula 2 in a yield of 45%.

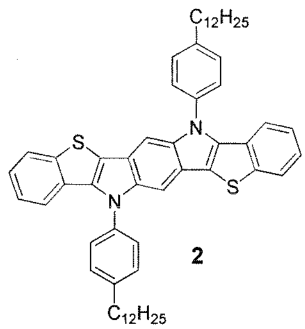

2

Physical properties of the compound 2 were as follows. $^1$H-NMR ($CDCl_3$ , δppm):7.87(d, 2H), 7.69(s, 2H), 7.58(d, 4H), 7.49(d, 4H), 7.18-7.33(m, 6H), 2.82(t, 4H), 1.76-1.82(m, 4H), 1.29-1.55(m, 36H), 0.91(t, 6H)
HRMS (MLDI-TOF) : calcd for $C_{58}H_{68}N_2S_2$, 856.4824; found 856.4783

[Example 5]

Production of Compound 3

A mixed liquid of 2,2'-(2,5-dibromobenzene-1,4-diyl)bis(3-bromo-1-benzothiophene) (8.8 g, 13.4 mmol), 4-n-octylaniline (30.21 g, 147.1 mmol), tris(dibenzylidineacetone) dipalladium (2.45 g, 2.7 mmol), sodium tert-butoxide (11.31 g, 117.7 mmol), tri-tert-butylphosphine (2.16 g, 10.7 mmol) and toluene (430 mL) was refluxed under a nitrogen atmosphere at about 110°C for 18 hours. After the reaction mixed liquid was allowed to cool to room temperature and water was added to the reaction mixture liquid, and then the reaction mixture liquid was extracted with chloroform. The obtained

organic layer was dried over sodium sulfate and filtered, and then the solvent was distilled off under reduced pressure. The obtained mixture was separated and purified by silica gel chromatography to obtain compound 3 (4.34 g, 5.82 mmol) represented by the following formula 3 in a yield of 44%.

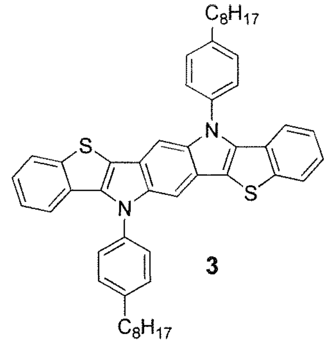

3

Physical properties of the compound 3 were as follows. $^1$H-NMR($CDCl_3$, δppm) : 7.87(d, 2H), 7.69(s, 2H), 7.58 (d, 4H), 7.49(d, 4H), 7.18-7.33(m, 6H), 2.82(t, 4H), 1.78-1.84(m, 4H), 1.34-1.55(m, 20H), 0.92(t, 6H) HRMS (MLDI-TOF) : calcd for $C_{50}H_{52}N_2S_2$, 744.3572; found 744.3555

[Example 6]

Production of Compound 4

A mixed liquid of 2,2'-(2,5-dibromobenzene-1,4-diyl)bis(3-bromo-1-benzothiophene) (8.8 g, 13.4 mmol), 4-n-octyloxyaniline (32.56 g, 147.1 mol), tris(dibenzylidineacetone) dipalladium (2.45 g, 2.7 mmol), sodium tert-butoxide (11.31 g, 117.7 mmol), tri-tert-butylphosphine (2.16 g, 10.7 mmol) and toluene (430 mL) was refluxed under a nitrogen atmosphere at about 110°C for 8 hours. After the reaction mixed liquid was allowed to cool to room temperature and water was added to the reaction mixture liquid, and then the reaction mixture liquid was extracted with chloroform. The obtained organic layer was dried over sodium sulfate and filtered, and then the solvent was distilled off under reduced pressure. The obtained mixture was subjected to silica gel chromatography followed by recrystallization from chloroform to obtain the compound 4 (4.56 g, 5.87 mmol) represented by the following formula 4 in a yield of 43%.

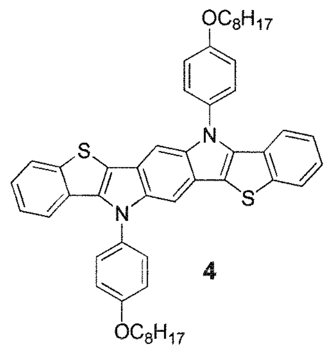

4

Physical properties of the compound 4 were as follows. $^1$H-NMR($CDCl_3$, δppm) : 7.87(d, 2H), 7.62(s, 2H), 7.58 (d, 4H), 7.16-7.56(m, 10H), 4.13(t, 4H), 1.86-1.94(m, 4H), 1.34-1.59(m, 20H), 0.93(t, 6H)
HRMS (MLDI-TOF) : calcd for $C_{50}H_{52}N_2O_2S_2$, 776.3470; found 776.3450

[Example 7]

Production of Compound 5

A mixed liquid of 2,2'-(2,5-dibromobenzene-1,4-diyl)bis(3-bromo-1-benzothiophene) (0.3 g, 0.46 mmol), 4-n-hexyloxyaniline (0.97 g, 5.01 mmol), tris(dibenzylidineacetone)dipalladium (0.08 g, 0.09 mmol), sodium tert-butoxide

(0.39 g, 4.01 mmol), tri-tert-butylphosphine (0.07 g, 0.36 mmol) and toluene (15 mL) was refluxed under a nitrogen atmosphere at about 110°C for 25.5 hours. After the reaction mixed liquid was allowed to cool to room temperature and water was added to the reaction mixture liquid, and then the reaction mixture liquid was extracted with chloroform. The obtained organic layer was dried over sodium sulfate and filtered, and then the solvent was distilled off under reduced pressure. The obtained mixture was subjected to silica gel chromatography followed by recrystallization from chloroform to obtain the compound 5 (0.14 g, 0.2 mmol) represented by the following formula 5 in a yield of 44%.

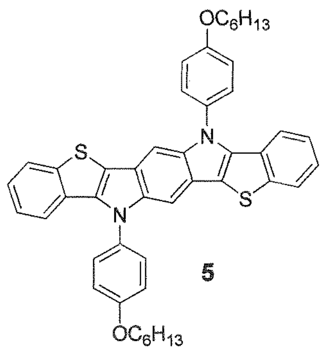

5

Physical properties of the compound 5 were as follows. $^1$H-NMR($CDCl_3$, δppm) : 7.87(d, 2H), 7.62(s, 2H), 7.56 (d, 4H), 7.16-7.31(m, 10H), 4.14(t, 4H), 1.86-1.97(m, 4H), 1.39-1.60(m, 12H), 0.98(t, 6H)
HRMS(MLDI-TOF):calcd for $C_{46}H_{44}N_2O_2S_2$, 720.2844;found 720.2833

[Example 8]

Production of Compound 6

A mixed liquid of 2,2'-(2,5-dibromobenzene-1,4-diyl)bis(3-bromo-1-benzothiophene) (10.0 g, 15.2 mmol), n-dodecylamine (31.0 g, 167.2 mmol), tris(dibenzylidineacetone)dipalladium (2.8 g, 3.04 mmol), sodium tert-butoxide (12.9 g, 133.7 mmol), (±)-2,2'-Bis(diphenylphosphino)-1,1'-binaphthyl (7.6 g, 12.2 mmol) and toluene (500 mL) was refluxed under a nitrogen atmosphere at about 110°C for 21 hours. After the reaction mixed liquid was allowed to cool to room temperature and water was added to the reaction mixture liquid, and then the reaction mixture liquid was extracted with chloroform. The obtained organic layer was dried over sodium sulfate and filtered, and then the solvent was distilled off under reduced pressure. The obtained mixture was subjected to silica gel chromatography followed by recrystallization from chloroform to obtain the compound 6 (4.91 g, 7.0 mmol) represented by the following Chemical Formula 6 in a yield of 46%.

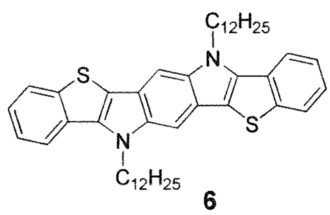

6

Physical properties of the compound 6 were as follows. $^1$H-NMR($CDCl_3$, δppm) : 8.03(d, 2H), 7.94(d, 2H), 7.33 (s, 2H), 7.46(dd, 2H), 7.33(dd, 2H), 4.65(t, 4H), 1.97-2.05(m, 4H)1.20-1.50(m, 36H), 0.86(t, 6H)
HRMS(MLDI-TOF):calcd for $C_{46}H_{60}N_2O_2S_2$, 704.4198; found 704.4194

[Example 9]

Production of 2,5-bis(1-benzothiophen-2-yl)thieno[3,2-b]thiophene

2,5-bis(1-benzothiophen-2-yl)thieno[3,2-b]thiophene was obtained by using 2,5-dibromothieno[3,2-b]thiophene in place of 1,4-dibromo-2,5-diiodobenzene in Example 1.

[Example 10]

Production of 2,5-bis(3-bromo-1-benzothiophen-2-yl)-3,6-dibromothieno[3,2-b]thiophene

2,5-bis(3-bromo-1-benzothiophen-2-yl)-3,6-dibromothieno[3,2-b]thiophene was obtained by using 2,5-bis(l-benzothiophen-2-yl)thieno[3,2-b]thiophene in place of 2,2'-(2,5-dibromobenzene-1,4-diyl)bis(1-benzothiophene) and using N-bromosuccinimide in the amount of 4 mol times relative to 2,5-bis(,1-benzothiophen-2-yl)thiena[3,2-b]thiophene in Example 2.

[Example 11]

Production of Compound 7

The compound 7 represented by the following formula 7 was obtained by using 2,5-bis(3-bromo-1-benzothiophen-2-yl)-3,6-dibromothieno[3,2-b]thiophene in place of 2,2'-(2,5-dibromobenzene-1,4-diyl)bis(3-bromo-1-benzothiophene) in Example 3.

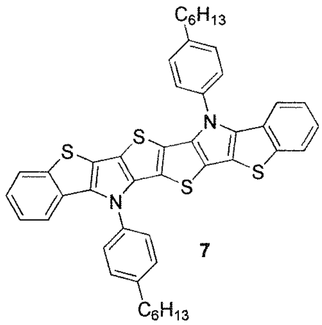

7

[Example 12]

Production of 2,6-bis(1-benzothiophen-2-yl)benzo[1,2-b;4,5-b']dithiophene

2,6-bis(1-benzothiophen-2-yl)benzo[1,2-b;4,5-b']dithiophene was obtained by using 2,6-dibromobenzo[1,2-b;4,5-b']dithiophene in place of 1,4-dibromo-2,5-diiodobenzene in Example 1.

[Example 13]

Production of 2,6-bis(3-bromo-1-benzothiophen-2-yl)-3,7-dibromobenzo[1,2-b;4,5-b']dithiophene

2,6-bis(3-bromo-1-benzothiophen-2-yl)-3,7-dibromobenzo[1,2-b;4,5-b']dithiophene was obtained by using 2,6-bis(1-benzothiophen-2-yl)benzo[1,2-b;4,5-b']dithiophene in place of 2,2'-(2,5-dibromobenzene-1,4-diyl)bis(1-benzothiophene) and using N-bromosuccinimide in the amount of 4 mol times relative to 2,6-bis(1-benzothiophen-2-yl)benzo [1,2-b;4,5-b']dithiophene in Example 2.

[Example 14]

Production of Compound 8

The compound 8 represented by the following formula 8 was obtained by using 2,6-bis(3-bromo-1-benzothzophen-2-yl)-3,7-dibromobenzo[1,2-b;4,5-b']dithiophene in place of 2,2'-(2,5-dibromobenzene-1,4-diyl)bis(3-bromo-1-benzothiophene) in Example 3.

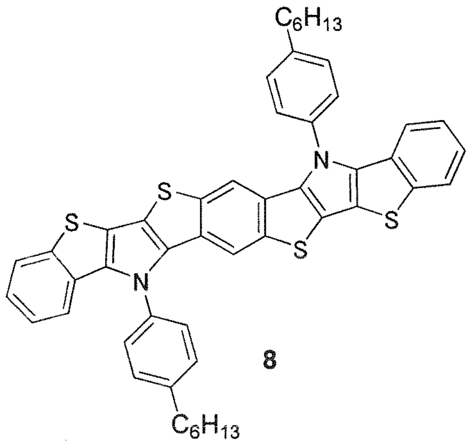

8

[Example 15]

Production of Organic Transistor I

A highly doped n-type silicon substrate with a 300 nm thick thermally oxidized film was subjected to ultrasonic cleaning in acetone for 10 minutes and then irradiated with ozone UV for 20 minutes. Thereafter, a surface of the thermally oxidized film was subjected to a silane treatment by spin coating of β-phenethyltrichlorosilane diluted with toluene.

Next, the compound 2 produced in Example 4 was dissolved in chloroform to prepare a solution having a concentration of the compound 2 of 0.8% by weight and the solution was filtered through a membrane filter to prepare a coating solution. The coating solution was applied to the above substrate subjected to the surface treatment by a spin coating method to form a coating film (thickness: about 60 nm) of the compound 2. Furthermore, the coating film was subjected to a heat treatment in a nitrogen atmosphere at 120°C for 30 minutes to form an organic semiconductor thin film of the compound 2.

Furthermore, by a vacuum vapor deposition method using a metal mask, a source electrode and a drain electrode (size of each electrode: channel length of 20 μm, channel width of 2 mm) having a laminated structure of molybdenum trioxide and gold were formed on an organic semiconductor thin film from the side of the organic semiconductor thin film, and thus an organic transistor I having a structure of Fig. 2 could be produced.

[Example 16]

Electrical Characteristics of Organic Transistor I

Electrical characteristics organic transistor I were measured using a semiconductor parameter 4200 (manufactured by Keithley Instruments Inc.).

As a result, variation curves of drain currents (Id) versus drain voltages (Vd) at certain gate voltages (Vg) were good, and exhibited saturated regions at high drain voltages. When a negative gate voltage applied to the gate electrode was increased, a negative drain current increases, and thus it could be confirmed that an organic transistor I is a p-type organic transistor.

The saturated field-effect mobility μ of a carrier in the organic transistor I was calculated using the following equation representing the drain current Id in the saturated region of electric characteristics of the organic transistor.

$$Id = (W/2L)\mu Ci(Vg - Vt)2 \qquad (a)$$

wherein L is a channel length of an organic transistor, W is a channel width of an organic transistor, Ci is a capacity per unit area of a gate insulating film, Vg is a gate voltage, and Vt is a threshold voltage of a gate voltage.

The field effect mobility (carrier mobility) of the carrier was $1 \times 10^{-3}$ $cm^2/Vs$, and the on/off current ratio was $10^5$.

[Example 17]

Production of Organic Transistor II

A highly-doped n-type silicon substrate (a thickness of a thermally oxidized film: 300 nm) with a $SiO_2$ thermally oxidized film, including a gold electrode having a channel width of 2 mm and a channel length of 20 μm was subjected to ultrasonic cleaning in acetone for 10 minutes and then irradiated with ozone UV for 20 minutes. Thereafter, a surface of the thermally oxidized film was subjected to a silane treatment by spin coating of β-phenethyltrichlorosilane diluted with toluene. Furthermore, a surface of the gold electrode was subjected to a thiol treatment by spin coating of pentafluorobenzenethiol diluted with isopropyl alcohol.

Next, the compound 2 synthesized in Example 4 was charged in a quartz crucible and set in a vacuum deposition device, together with the substrate subjected to the surface treatment. Under the conditions of a vacuum degree of $1 \times 10^{-4}$ Pa or less in a device chamber and a substrate temperature of 60°C, a crucible was heated thereby volatilizing the compound 2 to precipitate it on the substrate , and thus an organic semiconductor thin film made of the compound 2 was formed. An organic transistor II having a structure of Fig. 1 could be produced by the following process. The film thickness of the organic semiconductor thin film was about 100 nm.

[Example 18]

Electrical Characteristics of Organic Transistor II

As a result of the measurement of electrical characteristics of the organic transistor II in the same manner as in Example 16, the field effect mobility of the carrier was $1.4 \times 10^{-3}$ $cm^2/Vs$, and the on/off current ratio was $10^5$.

[Example 19]

Production of Organic Transistor III

A highly-doped n-type silicon substrate (a thickness of a thermally oxidized film: 300 nm) with a $SiO_2$ thermally oxidized film, including a gold electrode having a channel width of 2 mm and a channel length of 20 μm was subjected to ultrasonic cleaning in acetone for 10 minutes and then irradiated with ozone UV for 20 minutes. Thereafter, a surface of the thermally oxidized film was subjected to a silane treatment by spin coating of β-phenethyltrichlorosilane diluted with toluene. Furthermore, a surface of the gold electrode was subjected to a thiol treatment by spin coating of pentafluorobenzenethiol diluted with isopropyl alcohol.

Next, the compound 3 produced in Example 5 was dissolved in xylene to prepare a solution having a concentration of the compound 3 of 1.0% by weight and the solution was filtered through a membrane filter to prepare a coating solution.

This coating solution was applied to the above substrate by a spin coating method to obtain a coating film (thickness: about 60 nm). Furthermore, the coating film was subjected to a heat treatment in a nitrogen atmosphere at 120°C for 30 minutes to form an organic semiconductor thin film of the compound 3. An organic transistor III having a structure of Fig. 1 could be produced by the above process.

[Example 20]

Electrical Characteristics of Organic Transistor III

As a result of the measurement of electrical characteristics of the organic transistor III in the same manner as in Example 16, the field effect mobility of the carrier was $5 \times 10^{-5}$ $cm^2/Vs$, and the on/off current ratio was $10^5$.

[Example 21]

Production of Organic Transistor IV

An organic transistor IV having a structure of Fig. 1 could be produced by operating in the same manner as in Example 17, except that the compound 3 was used in place of the compound 2.

[Example 22]

Electrical Characteristics of Organic Transistor IV

As a result of the measurement of electrical characteristics of the organic transistor IV in the same manner as in Example 16, the field effect mobility of the carrier was $3.3 \times 10^{-3}$ $cm^2/Vs$, and the on/off current ratio was $10^6$.

[Example 23]

Production of Organic Transistor V

A highly-doped n-type silicon substrate (a thickness of a thermally oxidized film: 300 nm) with a $SiO_2$ thermally oxidized film, including a gold electrode having a channel width of 2 mm and a channel length of 20 μm was subjected to ultrasonic cleaning in acetone for 10 minutes and then irradiated with ozone UV for 20 minutes.
Next, the compound 4 produced in Example 6 was dissolved in xylene to prepare a solution having a concentration of the compound 4 of 0.5% by weight and the solution was filtered through a membrane filter to prepare a coating solution.
This coating solution was applied to the above substrate by a casting method to form a thin film having a film thickness of about 90 nm made of the compound 4. Furthermore, the thin film was subjected to a heat treatment in a nitrogen atmosphere at 60°C for 30 minute, and thus an organic transistor V having a structure of Fig. 1 could be produced.

[Example 24]

Electrical Characteristics of Organic Transistor V

As a result of the measurement of electrical characteristics of the organic transistor V in the same manner as in Example 16, the field effect mobility of the carrier was $1.1 \times 10^{-4}$ $cm^2/Vs$, and the on/off current ratio was $10^3$.

[Example 25]

Production of Organic Transistor VI

An organic transistor VI having a structure of Fig. 1 could be produced by operating in the same manner as in Example 17, except that the compound 4 was used in place of the compound 2, and also subjecting an organic semiconductor thin film to a heat treatment in a nitrogen atmosphere at 110°C for 30 minutes.

[Example 26]

Electrical Characteristics of Organic Transistor VI

As a result of the measurement of electrical characteristics of the organic transistor VI in the same manner as in Example 16, the field effect mobility of the carrier was $5.6 \times 10^{-4}$ $cm^2/Vs$, and the on/off current ratio was $10^4$.

INDUSTRIAL APPLICABILITY

The compound of the present invention can be applied to organic semiconductor materials such as organic transistors. The production method of the present invention is useful as a method of producing the above compound simply and easily.

**Claims**

1. A compound represented by the formula (1):

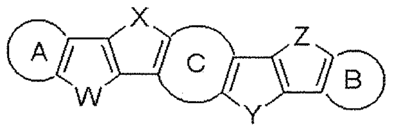

(1)

wherein a ring structure A and a ring structure B independently represent an aromatic ring which may be substituted or a heterocyclic ring which may be substituted;
a ring structure C represents a benzene ring which may be substituted, a hetero[3,2-b]heterole ring, or a benzo[1,2-b:4,5-b']diheterole ring which may be substituted;
W, X, Y and Z independently represent a sulfur atom, an oxygen atom, a selenium atom, a tellurium atom, $SO_2$, $(R^1)$-C-$(R^2)$, $(R^3)$-Si-$(R^4)$, or N-$(R^5)$, and at least one of W, X, Y and Z is N-$(R^5)$; and
$R^1$, $R^2$, $R^3$, $R^4$ and $R^5$ independently represent a hydrogen atom, a substituent i, a substituent ii, an aryl group which may be substituted with halogen, or a heteroaryl group which may be substituted with halogen, the substituent i is a group selected from Group P shown below, the substituent ii is a group selected from Group P shown below which group is substituted, and Group P consists of an alkyl group, an alkoxy group, an alkyl-substituted aryl group, an alkoxy-substituted aryl group, and an alkyl-substituted heteroaryl group.

2. The compound according to claim 1, wherein the ring structure A and the ring structure B independently represent an aromatic ring, a heterocyclic ring, an aromatic ring substituted with a substituent iii, an aromatic ring substituted with a substituent iv, a heterocyclic ring substituted with a substituent iii, a heterocyclic ring substituted with a substituent iv, a halogen-substituted aromatic ring, or a halogen-substituted heterocyclic ring,
the substituent iii is a substituent selected from Group Q shown below,
the substituent iv is a substituent selected from Group Q shown below, which is substituted with a fluorine atom, and Group Q consists of an alkyl group, an alkoxy group, an alkyl-substituted aryl group, an alkoxy-substituted aryl group, an alkoxy-substituted heteroaryl group, an alkyl-substituted heteroaryl group, an alkenyl group, an alkynyl group, an alkylthio group, an alkylcarbonyl group, an alkoxycarbonyl group, a (trialkyl)silyl group, and a (dialkyl) amino group.

3. The compound according to claim 2, wherein the ring structure A and the ring structure B independently represent a benzene ring, a benzene ring substituted with the substituent iii, a benzene ring substituted with the substituent iv, a halogen-substituted benzene ring, a naphthalene ring, a naphthalene ring substituted with the substituent iii, a naphthalene ring substituted with the substituent iv, a halogen-substituted naphthalene ring, a benzo[b]thiophene ring, a benzo[b]thiophene ring substituted with the substituent iii, a benzo[b]thiophene ring substituted with the substituent iv, a halogen-substituted benzo[b]thiophene ring, a benzo[b]furan ring, a benzo[b]furan ring substituted with the substituent iii, a benzo[b]furan ring substituted with the substituent iv, or a halogen-substituted benzo[b] furan ring.

4. The compound according to claim 2, wherein the ring structure A and the ring structure B independently represent a benzene ring, a benzene ring substituted with the substituent iii, a benzene ring substituted with the substituent iv, or a halogen-substituted benzene ring.

5. The compound according to claim 1, wherein the ring structure C is a benzene ring, a benzene ring substituted with a substituent iii, a benzene ring substituted with a substituent iv, a halogen-substituted benzene ring, a thieno[3,2-b]thiophene ring, a benzo[1,2-b:4,5-b']dithiophene ring, a benzo[1,2-b:4,5-b']dithiophene ring substituted with a substituent iii, a benzo[1,2-b:4,5-b']dithiophene ring substituted with a substituent iv, a halogen-substituted benzo [1,2-b:4,5-b']dithiophene ring, a benzo[1,2-b:4,5-b']difuran ring, a benzo[1,2-b:4,5-b']difuran ring substituted with a substituent iii, a benzo[1,2-b:4,5-b']difuran ring substituted with the substituent iv, or a halogen-substituted benzo [1,2-b:4,5-b']difuran ring,
the substituent iii is a substituent selected from Group Q shown below,
the substituent iv is a substituent selected from Group Q shown below, which is substituted with a fluorine atom, and Group Q consists of an alkyl group, an alkoxy group, an alkyl-substituted aryl group, an alkoxy-substituted aryl group, an alkoxy-substituted heteroaryl group, an alkyl-substituted heteroaryl group, an alkenyl group, an alkynyl group, an alkylthio group, an alkylcarbonyl group, an alkoxycarbonyl group, a (trialkyl)silyl group, and a (dialkyl) amino group.

6. The compound according to claim 5, wherein the ring structure C is a benzene ring, a benzene ring substituted with the substituent iii, a benzene ring substituted with the substituent iv, a halogen-substituted benzene ring, or a thieno[3,2-b]thiophene ring.

**7.** The compound according to claim 1, wherein any one of W and X is N-($R^5$), the other one is sulfur, oxygen, ($R^1$)-C-($R^2$) or ($R^3$)-Si-($R^4$), Z and W are the same, and Y and X are the same.

**8.** The compound according to claim 1, wherein any one of W and X is N-($R^5$), the other one is a sulfur atom or an oxygen atom, Z and W are the same, and Y and X are the same.

**9.** The compound according to claim 1, wherein $R^1$, $R^2$, $R^3$, $R^4$ and $R^5$ independently represent a hydrogen atom, an alkyl group having 1 to 30 carbon atoms, a fluoroalkyl group having 1 to 30 carbon atoms, a phenyl group, a phenyl group substituted with an alkyl group having 1 to 30 carbon atoms, a phenyl group substituted with a fluoroalkyl group having 1 to 30 carbon atoms, a phenyl group substituted with an alkoxy group having 1 to 30 carbon atoms, a phenyl group substituted with a fluoroalkoxy group having 1 to 30 carbon atoms, a thienyl group, a thienyl group substituted with an alkyl group having 1 to 30 carbon atoms, or a thienyl group substituted with a fluoroalkyl group having 1 to 30 carbon atoms.

**10.** The compound according to claim 1, wherein $R^5$ is an alkyl group having 1 to 20 carbon atoms, a fluoroalkyl group having 1 to 20 carbon atoms, a phenyl group, a phenyl group substituted with an alkyl group having 1 to 20 carbon atoms, a phenyl group substituted with a fluoroalkyl group having 1 to 20 carbon atoms, or a phenyl group substituted with an alkoxy group having 1 to 20 carbon atoms.

**11.** The compound according to claim 1, wherein the ring structure A, the ring structure B and the ring structure C independently represent a benzene ring, a benzene ring substituted with a substituent iii, or a halogen-substituted benzene ring, X and Y are the same, and N-($R^5$), W and Z are the same and sulfur atoms or oxygen atoms, the substituent iii is a substituent selected from Group Q shown below, and Group Q consists of an alkyl group, an alkoxy group, an alkyl-substituted aryl group, an alkoxy-substituted aryl group, an alkoxy-substituted heteroaryl group, an alkyl-substituted heteroaryl group, an alkenyl group, an alkynyl group, an alkylthio group, an alkylcarbonyl group, an alkoxycarbonyl group, a (trialkyl)silyl group, and a (dialkyl) amino group.

**12.** The compound according to claim 1, wherein the ring structure A, the ring structure B and the ring structure C independently represent a benzene ring, a benzene ring substituted with a substituent iii, or a halogen-substituted benzene ring, X and Y are the same and are sulfur atoms or oxygen atoms, W and Z are the same and are N-($R^5$), the substituent iii is a substituent selected from Group Q shown below, and Group Q consists of an alkyl group, an alkoxy group, an alkyl-substituted aryl group, an alkoxy-substituted aryl group, an alkoxy-substituted heteroaryl group, an alkyl-substituted heteroaryl group, an alkenyl group, an alkynyl group, an alkylthio group, an alkylcarbonyl group, an alkoxycarbonyl group, a (trialkyl)silyl group, and a (dialkyl) amino group.

**13.** The compound according to claim 2, wherein the ring structure A and the ring structure B independently represent a benzene ring, a benzene ring substituted with the substituent iii, or a halogen-substituted benzene ring, the ring structure C is a thieno[3,2-b]thiophene ring, X and Y are the same and are N-($R^5$), and W and Z are the same and are sulfur atoms or oxygen atoms.

**14.** The compound according to claim 2, wherein the ring structure A and the ring structure B independently represent a benzene ring, a benzene ring substituted with the substituent iii, or a halogen-substituted benzene ring, the ring structure C is a thieno[3,2-b]thiophene ring, X and Y are the same and are sulfur atoms or oxygen atoms, and W and Z are the same and are N-($R^5$).

**15.** The compound according to claim 1, wherein the ring structure A and the ring structure B independently represent a benzene ring, a benzene ring substituted with a substituent iii, or a halogen-substituted benzene ring, the ring structure C is a benzo[1,2-b:4,5-b']dithiophene ring, a benzo[1,2-b:4,5-b']dithiophene ring substituted with a substituent iii, a halogen-substituted benzo[1,2-b:4,5-b']dithiophene ring, a benzo[1,2-b:4,5-b']difuran ring, a benzo[1,2-b:4,5-b']difuran ring substituted with a substituent iii, or a halogen-substituted benzo[1,2-b:4,5-b']difuran ring, X and Y are the same and are N-($R^5$), W and Z are the same and are sulfur atoms or oxygen atoms, the substituent iii is a substituent selected from Group Q shown below, Group Q consists of an alkyl group, an alkoxy group, an alkyl-substituted aryl group, an alkoxy-substituted aryl group, an alkoxy-substituted heteroaryl group, an alkyl-substituted

heteroaryl group, an alkenyl group, an alkynyl group, an alkylthio group, an alkylcarbonyl group, an alkoxycarbonyl group, a (trialkyl)silyl group, and a (dialkyl)amino group.

**16.** The compound according to claim 1, wherein the ring structure A and the ring structure B independently represent a benzene ring, a benzene ring substituted with a substituent iii, or a halogen-substituted benzene ring, the ring structure C is a benzo[1,2-b:4,5-b']dithiophene ring, a benzo[1,2-b:4,5-b']dithiophene ring substituted with a substituent iii, a halogen-substituted benzo[1,2-b:4,5-b']dithiophene ring, a benzo[1,2-b:4,5-b']difuran ring, a benzo[1,2-b:4,5-b']difuran ring substituted with a substituent iii, or a halogen-substituted benzo[1,2-b:4,5-b']difuran ring, X and Y are the same and are sulfur atoms or oxygen atoms, W and Z are the same and are N-($R^5$), the substituent iii is a substituent selected from Group Q shown below,
Group Q consists of an alkyl group, an alkoxy group, an alkyl-substituted aryl group, an alkoxy-substituted aryl group, an alkoxy-substituted heteroaryl group, an alkyl-substituted heteroaryl group, an alkenyl group, an alkynyl group, an alkylthio group, an alkylcarbonyl group, an alkoxycarbonyl group, a (trialkyl)silyl group, and a (dialkyl) amino group.

**17.** A method for producing the compound according to claim 1, comprising a step of reacting a tetrahalogen compound represented by the formula (2) or (3):

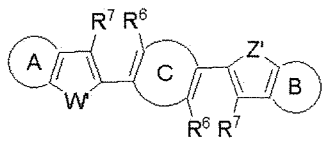

(2)

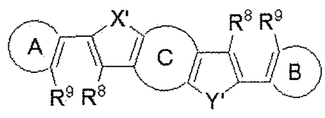

(3)

wherein a ring structure A, a ring structure B and a ring structure C are each as defined in claim 1, W', X', Y' and Z' independently represent a sulfur atom, an oxygen atom, a selenium atom, a tellurium atom, $SO_2$, ($R^1$)-C-($R^2$), ($R^3$)-Si-($R^4$), or N-($R^5$), $R^1$, $R^2$, $R^3$, $R^4$ and $R^5$ are as defined in claim 1, and $R^6$, $R^7$, $R^8$ and $R^9$ independently represent a halogen atom, with $R^5$-$NH_2$, wherein $R^5$ is as defined in claim 1.

**18.** The method according to claim 17, wherein $R^6$, $R^7$, $R^8$ and $R^9$ independently represent bromine or iodine.

**19.** The method according to claim 17 or 18, wherein X' ,Y' ,W' and Z' independently represent a sulfur atom or an oxygen atom.

**20.** The method according to any one of claims 17 to 19, wherein $R^5$ is an alkyl group having 1 to 20 carbon atoms, a fluoroalkyl group having 1 to 20 carbon atoms, a phenyl group, a phenyl group substituted with an alkyl group having 1 to 20 carbon atoms, a phenyl group substituted with a fluoroalkyl group having 1 to 20 carbon atoms, or a phenyl group substituted with an alkoxy group having 1 to 20 carbon atoms.

**21.** A tetrahalogen compound represented by the formula (2):

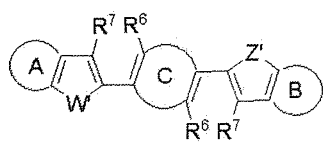

(2)

wherein a ring structure A and a ring structure B are each as defined in claim 1, a ring structure C is a thieno[3,2-b]thiophene ring, W' and Z' independently represent a sulfur atom or an oxygen atom, and $R^6$, $R^7$, $R^8$ and $R^9$

independently represent a halogen atom.

**22.** The tetrahalogen compound according to claim 21, wherein the ring structure A and the ring structure B independently represent a benzene ring, a naphthalene ring, a thiophene ring, a benzothiophene ring, a furan ring, or a benzofuran ring.

**23.** A tetrahalogen compound represented by the formula (3):

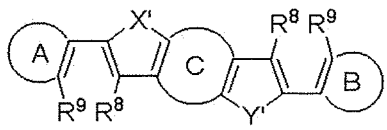

(3)

wherein a ring structure A and a ring structure B are each as defined in claim 1, a ring structure C is a thieno[3,2-b]thiophene ring, a furo[3,2-b]furan ring, a benzo[1,2-b:4,5-b']dithiophene ring which may be substituted, or a benzo [1,2-b:4,5-b']difuran ring which may be substituted, and X' and Y' independently represent a sulfur atom or an oxygen atom.

**24.** The tetrahalogen compound according to claim 23, wherein the ring structure A and the ring structure B independently represent a benzene ring, a naphthalene ring, a thiophene ring, a benzothiophene ring, a furan ring, or a benzofuran ring.

**25.** A method for producing a tetrahalogen compound represented by the formula (2):

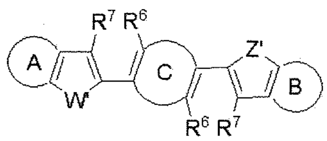

(2)

wherein W' , Z' , $R^6$ and $R^7$ are each as defined in claim 17, the method comprising a step of reacting a compound represented by the formula (4):

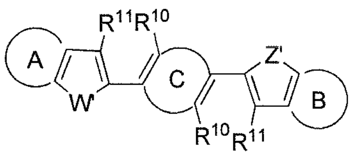

(4)

wherein a ring structure A, a ring structure B, a ring structure C, W' and Z' are as defined in claim 17, $R^{10}$ and $R^{11}$ independently represent a hydrogen atom or a halogen atom, at least one of $R^{10}$ and $R^{11}$ is a hydrogen atom, with a halogenating agent.

**26.** The method according to claim 25, wherein $R^{10}$ and $R^{11}$ independently represent a hydrogen, bromine or iodine atom, and at least one of $R^{10}$ and $R^{11}$ is a hydrogen atom.

**27.** The method according to claim 25, wherein W' and Z' independently represent a sulfur atom or an oxygen atom.

**28.** A method for producing a tetrahalogen compound represented by the formula (3):

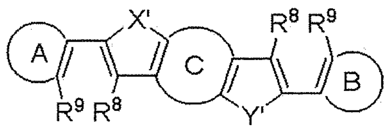

(3)

wherein X' , Y' , $R^8$ and $R^9$ are each as defined in claim 17, the method comprising a step of reacting a compound represented by the formula (5):

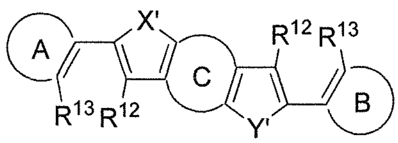

(5)

wherein a ring structure A, a ring structure B, a ring structure C, X' and Y' are as defined in claim 17, $R^{12}$ and $R^{13}$ independently represent a hydrogen atom or a halogen atom, and at least one of $R^{12}$ and $R^{13}$ is a hydrogen atom, with a halogenating agent.

**29.** The method according to claim 28, wherein $R^{12}$ and $R^{13}$ independently represent a hydrogen atom, a bromine atom or an iodine atom, and at least one of $R^{12}$ and $R^{13}$ is a hydrogen atom.

**30.** The method according to claim 28 or 29, wherein X' and Y' independently represent a sulfur atom or an oxygen atom.

**31.** An organic semiconductor device comprising the compound according to claim 1.

**32.** A conductive thin film comprising the compound according to claim 1.

**33.** A light emitting thin film comprising the compound according to claim 1.

**34.** An organic semiconductor thin film comprising the compound according to claim 1.

**35.** The organic semiconductor thin film according to claim 34, wherein a carrier mobility is $10^{-6}$ $cm^2/Vs$ or more.

**36.** An organic transistor comprising the organic semiconductor thin film according to claim 34 or 35.

**37.** A light emitting element comprising the light emitting thin film according to claim 33.

Fig.1

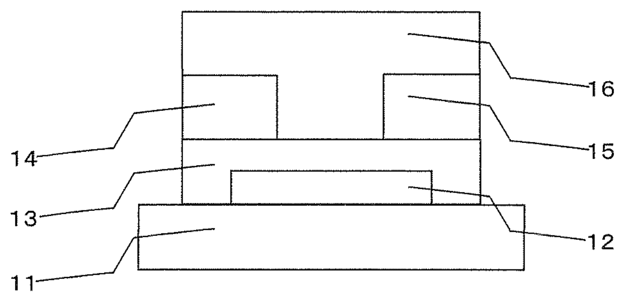


Fig.2

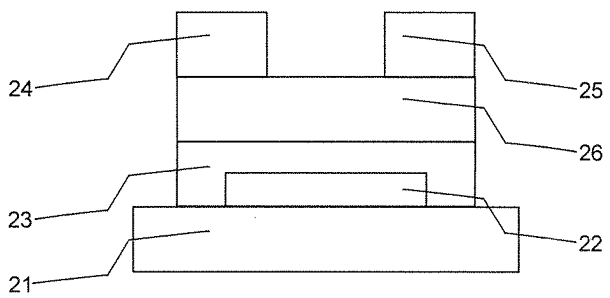

**INTERNATIONAL SEARCH REPORT**

International application No.
PCT/JP2009/065969

A. CLASSIFICATION OF SUBJECT MATTER
*C07D495/22*(2006.01)i, *C07D495/04*(2006.01)i, *H01L29/786*(2006.01)i, *H01L51/05*(2006.01)i, *H01L51/30*(2006.01)i, *H01L51/50*(2006.01)i

According to International Patent Classification (IPC) or to both national classification and IPC

B. FIELDS SEARCHED

Minimum documentation searched (classification system followed by classification symbols)
C07D495/22, C07D495/04, H01L29/786, H01L51/05, H01L51/30, H01L51/50

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | WO 2008/026602 A1 (Tosoh Corp.), 06 March 2008 (06.03.2008), particularly, paragraphs [0035] to [0038]; example 2 & JP 2008-81494 A & EP 2067782 A | 1-37 |
| Y | JP 2008-10541 A (Konica Minolta Holdings, Inc.), 17 January 2008 (17.01.2008), particularly, paragraphs [0044] to [0047] (Family: none) | 1-37 |
| Y | JP 2007-19294 A (Konica Minolta Holdings, Inc.), 25 January 2007 (25.01.2007), particularly, formula I (Family: none) | 1-37 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 November, 2009 (05.11.09) | 17 November, 2009 (17.11.09) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2009/065969

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2007-269775 A (Osaka University), 18 October 2007 (18.10.2007), particularly, formula I & US 2009/0065770 A & EP 2006291 A1 & WO 2007/105386 A1 | 1-37 |
| P,X | JP 2009-99658 A (Mitsui Chemicals, Inc.), 07 May 2009 (07.05.2009), entire text (Family: none) | 1-37 |
| P,A | JP 2009-190999 A (Osaka University), 27 August 2009 (27.08.2009), entire text (Family: none) | 1-37 |
| P,A | WO 2009/63780 A1 (Mitsui Chemicals, Inc.), 22 May 2009 (22.05.2009), entire text (Family: none) | 1-37 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *J. Appl. Phys.,* 2002, vol. 92, 5259
- *J. Am. Chem. Soc.,* 2004, vol. 126, 13859
- *Science,* 1998, vol. 280, 1741
- *J. Am. Chem. Soc.,* 2007, vol. 129, 2224
- *J. Am. Chem. Soc.,* 2005, vol. 127, 614
- *J. Am. Chem. Soc.,* 2007, vol. 129, 9125
- *Synth. Met.,* 2002, vol. 130, 139
- *J. Org. Chem.,* 2003, vol. 68, 9813
- *J. Am. Chem. Soc.,* 2007, vol. 129, 12386